# EUROPEAN PATENT APPLICATION

(11) **EP 2 272 977 A1**
(43) Date of publication of application: **12.01.2011**
(21) Application number: 09164947.5
(22) Date of filing: 08.07.2009
(51) Int. Cl.: C12Q 1/68

(54) **Diagnostic method and kit for the detection of a lymphocytic variant of hypereosinophilic syndrome**

(71) Applicant: UNIVERSITE LIBRE DE BRUXELLES, B-1050 Bruxelles (BE); UNIVERSITE CATHOLIQUE DE LOUVAIN, 1348 Louvain-la-Neuve (BE)
(72) Inventor: Willard-Gallo, Karen, 1300 Wavre (BE); Ravoet, Marie, 1050 Brussels (BE); Roufosse, Florence, 7000 Mons (BE); Sibille, Catherine, 1970 Wezembeek-Oppem (BE)
(74) Representative: pronovem

(57) **Abstract**

The present invention is related to a gene set, a kit and a method for diagnosis of lymphocytic variant hypereosinophilic syndrome.

## Description

### Field of the invention

The present invention is in the field of diagnosis of a new disease and is related to diagnostic methods and tools of the lymphocytic variant of hypereosinophilic syndrome.

### Background of the invention and state of the art

Hypereosinophilic syndromes (HES) are a group of rare diseases which share the following characteristics: long-term persistence of marked hypereosinophilia which is not due to an underlying disease known to cause eosinophil expansion (such as parasitic disease or drug allergy), complicated by eosinophil-mediated tissue and/or organ damage. Although linked by this common presentation, HESs are clinically very heterogeneous in terms of the nature and severity of complications (potentially life-threatening in some patients, and relatively benign in others), natural disease progression (possible development of acute myeloid/eosinophilic leukemia in some patients, of T cell lymphoma in others), prognosis, and response to therapy. In the past decade, several advances have allowed a better understanding of this heterogeneity, with the description of pathogenically distinct disease variants.
In lymphocytic variant hypereosinophilic syndrome (L-HES), hypereosinophilia develops as a result of IL-5 overproduction by a population of phenotypically aberrant T-cells, which are generally monoclonal. Although the most frequently observed phenotype is CD3⁻CD4⁺, other T cell subsets (namely CD3⁺CD4⁺CD7⁻, CD3⁺CD8⁺ or CD3⁺CD4⁻CD8⁻) have been shown to produce IL-5 in this setting, and are likely involved in HES pathogenesis as well. In addition to IL-5, the aberrant T cells may produce the other Th2 cytokines, IL-4 and/or IL-13, and thus be responsible for associated increases in serum IgE levels. Importantly, L-HES can progress to malignant T-lymphoma after a prolonged period of chronic disease. Indeed, the clonal CD3⁻CD4⁺ T-cell population characterizing L-HES persists *in vivo* for many years, sometimes at reduced levels in response to corticosteroids, and a subgroup of patients may eventually become refractory to treatment in parallel with malignant progression to T-lymphoma. In some cases, an abnormal karyotype can be detected at pre-neoplastic disease stages. The fact that this T-cell lymphoproliferative disorder involves activated Th2 cells leads to early patient follow-up by physicians, because of the very symptomatic complications of hypereosiniphilia per se. Thus diagnosis can generally/theoretically be made several years before malignant progression, allowing for appropriate management, namely choice of therapy which targets T cell functions as well as eosinophils, and close follow-up for early detection of transformation.

Despite these advances, the molecular changes associated with persistence and expansion of the pre-malignant T cell clone during the chronic phase disease, and the emergence of malignant subclones, are unknown, precluding the development of targeted and potentially curative therapy for this HES variant. Furthermore, diagnosis remains challenging, requiring T cell phenotyping by flow cytometry (the interpretation of which is best handled by expert investigators), investigation of T cell receptor (TCR) gene rearrangement patterns by PCR, and ideally determination of the cytokine profile of cultured T cells (which is not routinely available or standardised).

This contrasts with the discovery of the disease-inducing FIP1L1/PDGFRA fusion gene in another group of HES patients with features of myeloproliferative disease, which can be detected by well-described FISH (fluorescence in situ hybridization) and PCR tests, and which is remarkably well-suppressed by the tyrosine kinase inhibitor imatinib mesylate.

### Aims of the invention

The present invention aims to provide new tools and methods, especially a gene or protein set and diagnostic kit for improving the detection of a clonal Th2-mediated immune disorder, especially the detection of lymphocytic variant hypereosinophilic syndrome.

The present invention aims to provide new tools and methods for L-HES diagnosis, beyond the CD3⁻CD4⁺ T cell associated form, and not depending on expertise of investigators.

### Summary of the invention

The present invention is related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 89, 90, 95 possibly 100, 110, 120, 150 or 200 genes and/or corresponding coded proteins and/or antibodies (or specific hypervariable portions thereof both) being directed against these proteins selected from Tables 4 to 9, or the entire set of Tables 4 to 9 of genes, corresponding proteins, and/or antibodies (or specific hypervariable portion thereof both) being directed against the proteins encoded by these genes.

More particularly, an aspect of the present invention is related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 89, 90, 95 possibly 100, 110, 120, 150 or 200 genes and/or corresponding coded proteins and/or antibodies (or specific hypervariable portions thereof both) being directed against these proteins encoded by these genes or the entire set selected from Table 4.

Another aspect of the present invention is related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 89, 90, 95 possibly 100, 110, 120, 150 or 200 genes and/or corresponding coded proteins and/or antibodies (or specific hypervariable portions thereof both) being directed against these proteins encoded by these genes or the entire set selected from Table 5.

Still, another aspect of the present invention is related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 89, 90, 95 possibly 100, 110, 120, 150 or 200 genes and/or corresponding coded proteins and/or antibodies (or specific hypervariable portions thereof both) being directed against these proteins encoded by these genes or the entire set selected from Table 6.

The present invention is further related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 89, 90, 95 possibly 100, 110, 120, 150 or 200 genes and/or corresponding coded proteins and/or antibodies (or specific hypervariable portions thereof both) being directed against these proteins encoded by these genes or the entire set selected from Table 7.

The present invention is also related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 89, 90 genes and/or corresponding coded proteins and/or antibodies (or specific hypervariable portions thereof both) being directed against these proteins encoded by these genes or the entire set selected from Table 8.

The present invention is related to a gene or protein set comprising or consisting of at least 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85 genes and/or corresponding coded proteins and/or antibodies (or specific hypervariable portions thereof both) being directed against these proteins encoded by these genes or the entire set selected from Table 9.

Advantageously, the set according to the present invention comprises (or consists of) 10, 11, 12, 13, 14, 15, 16, 17, 18, 19, 20, 21, 22, 23, 24, or all the 25 genes and/or corresponding coded proteins and/or antibodies (or specific hypervariable portions thereof both) being directed against these proteins encoded by these genes that are selected from the group consisting of BCAT1, CACNA1D, CCR3, CCR8, CD200R1, CDH1, CHN2, CLECL1, CLU, DNM3, FANK1, GPR44, GPR68, IL17RB, IL9R/LOC729486, MAP3K8, NINJ2, P2RY14, PRSS21, PTPRN2, RBBP8, RGS1, TNFRSF11A, TNFSF11, ZNF365

Advantageously, the set according to the present invention comprises (or consists of) 10, 11, 12, 13, 14, 15, 16, 17, 18, 19, 20, 21, 22, 23, 24, 25 or all the genes and/or corresponding coded proteins and/or antibodies (or specific hypervariable portions thereof both) being directed against these proteins encoded by these genes that are selected from the group consisting of ACVR1C, ADRB2, AIF1, ALOX5AP, AMICA1, ANKRD55, ANKRD57, APBA2, BACH2, BAG2, BATF, BCAT1, BCL2, BNIP3, BTBD11, BTLA, CACNA1D, CBLB, CCL5, CCR3, CCR6, CCR7, CCR8, CD200R1, CD247, CD27, CD3G, CD5, CD55, CD7, CDC42, CDCA7, CDH1, CEP55, CHN2, CHRM3, CLEC2B, CLECL1, CLU, COTL1, CPNE2, CR1, CR2, CRTAM, CST7, CTLA4, CXCR4, CXCR6, CYSLTR1, DDX17, DNM3, DSC1, DUSP1, DUSP2, DUSP4, EMR1, EPHB6, EPPK1, F8, FAIM3, FANK1, FAS, FGF9, FHIT, FOXP1, GATA3, GBP2, GBP5, GPR137B, GPR44, GPR68, GZMK, HPCAL4, IER3, IFI44, IFI6, IGF1R, IGSF4, IGSF9B, IL17RB, IL18R1, IL23A, IL4R, IL9R/LOC729486, ITGA4, ITGA6, JAKMIP1, KIAA1199, KIF11, KIT, KLF9, KLRB1, KLRK1, KRT1, LASS6, LGALS3, LM02, LM04, LM07, LOC401233, LRRN3, MAN1C1, MAP3K8, MCOLN2, MFHAS1, MSC, MYB, MYBL1, NDFIP2, NELL2, NFKBIZ, NINJ2, NKG7, NOG, NPCDR1, NRIP1, NT5E, P2RY14, PAM, PDE4DIP, PDE9A, PIP3-E, PITPNC1, PLCB1, PLCL1, PLEKHA1, PLEKHA5, PLSCR1, PRSS21, PTPN13, PTPN4, PTPRM, PTPRN2, RAB27B/GPR30, RAPGEF6, RASGRF2, RBBP8, RGS1, RGS10, RIN3, RIPK2, RNF130, RUNX2, SCML1, SEMA5A, SESN1, SLAMF1, SLAMF7, SLC1A4, SMAD5, SMAD7, SNED1, SOS1, SOX4, SPON1, STK17A, TBL1X, TBXAS1, TCEA3, TCEAL4, TGFBR2, TGFBR3, TNFRSF10D, TNFRSF11A, TNFSF10, TNFSF11, TNFSF13B, TNFSF14, TNIK, TRAV20, TRBV21-1/19/7-2/5-4/3-1/TRBC1, TRDV2, TRERF1, TSHZ2, TXK, USP7, VIPR1, WWTR1, YES1, ZNF365.

Preferably, the set of the invention comprises (or consists of) RBBP8, MAP3K8 and PTPRN2 genes and/or corresponding coded proteins and/or antibodies (or specific hypervariable portions thereof) both being directed against these proteins encoded by these genes and possibly 1, 2, 3, 4, 5, 6, 7, 8, 9, 10, 20, 30, 40 or all the (other) gene(s) or coded protein(s) of Tables 4 to 9 and/or corresponding antibodies (or specific hypervariable portions thereof both) being directed against these proteins encoded by these genes.

Alternatively, the set of the invention comprises RBBP8, MAP3K8 and PTPRN2 and 1, 2, 3, 4, 5 or all the (other) gene(s) and/or corresponding coded protein(s) and/or antibodies (or specific hypervariable portion thereof both) being directed against these proteins encoded by these genes of Table 4.

Alternatively, the set of the invention comprises RBBP8, MAP3K8 and PTPRN2 and 1, 2, 3, 4, 5 or all the (other) gene(s) and/or corresponding coded protein(s) and/or antibodies (or specific hypervariable portion thereof both) being directed against these proteins encoded by these genes of Table 5.

Alternatively, the set of the invention comprises RBBP8, MAP3K8 and PTPRN2 and 1, 2, 3, 4, 5 or all the (other) gene(s) and/or corresponding coded protein(s) and/or antibodies (or specific hypervariable portion thereof both) being directed against these proteins encoded by these genes of Table 6.

Alternatively, the set of the invention comprises RBBP8, MAP3K8 and PTPRN2 and 1, 2, 3, 4, 5 or all the (other) gene(s) and/or corresponding coded proteins and/or antibodies (or specific hypervariable portions thereof both) being directed against these proteins encoded by these genes of Table 7.

Alternatively, the set of the invention comprises RBBP8, MAP3K8 and PTPRN2 and 1, 2, 3, 4, 5 or all the (other) gene(s) and/or corresponding coded protein(s) and/or antibodies (or specific hypervariable portions thereof both) being directed against these proteins encoded by these genes of Table 8.

Alternatively, the set of the invention comprises RBBP8, MAP3K8 and PTPRN2 and 1, 2, 3, 4, 5 or all the (other) gene(s) and/or corresponding coded protein(s) and/or antibodies (or specific hypervariable portions thereof both) being directed against these proteins encoded by these genes of Table 9.

Preferably, the set of the invention (further) consists of or comprises 2, 3, 4, 5, 6, 7, 8, 9 or all the gene(s) and/or corresponding coded proteins and/or antibodies (or specific hypervariable portions thereof both) being directed against these proteins encoded by these genes that are selected from the group consisting of ACVR1C, ADRB2, AMICA1, BTLA, CCR3, CCR6, CCR7, CCR8, CD200R1, CD247, CD27, CD3G, CD5, CD55, CD7, CDH1, CHRM3, CLEC2B, CLECL1, CR1, CR2, CRTAM, CTLA4, CXCR4, CXCR6, CYSLTR1, EMR1, EPHB6, FAS, GPR137B, GPR44, GPR68, IGF1R, IGSF4, IGSF9B, IL17RB, IL18R1, IL4R, IL9R/LOC729486, ITGA4, ITGA6, KIT, KLRB1, KLRK1, NINJ2, NT5E, P2RY14, SLAMF1, SLAMF7, SPON1, TGFBR2, TGFBR3, TNFRSF10D, TNFRSF11A, TRAV20, TRBV21-1/19/7-2/5-4/3-1/TRBC1, TRDV2 and VIPR1.

Advantageously, the set of the invention (further) consists of or comprises 2, 3, 4, 5, 6, 7, 8, 9 or all the gene(s) and/or corresponding coded proteins and/or antibodies (or specific hypervariable portions thereof both) being directed against these proteins encoded by these genes that are selected from the group consisting of AIF1, CCL5, CST7, F8, GZMK, IL23A, LGALS3, NOG, TNFSF10, TNFSF11, TNFSF13B and TNFSF14.

Preferably, the set according to the invention (further) consists of or comprises 2, 3, 4, 5, 6, 7, 8, 9 or all the gene(s) and/or corresponding coded proteins and/or antibodies (or specific hypervariable portions thereof both) being directed against these proteins encoded by these genes that are selected from the group consisting of BACH2, BATF, FOXP1, GATA3, KLF9, LM02, LM04, LM07, MSC, MYB, MYBL1, NFKBIZ, RBBP8, RUNX2, SOX4, TCEA3, TCEAL4, TRERF1, TSHZ2 and WWTR1.

Advantageously, the set of the invention comprises 2, 3, 4, 5, 6, 7, 8, 9 or all the gene (s) and/or corresponding coded proteins and/or antibodies (or specific hypervariable portions thereof both) being directed against these proteins encoded by these genes that are selected from the group consisting of ACVR1C, ADRB2, AMICA1, BTLA, CCR3, CCR6, CCR7, CCR8, CD200R1, CD247, CD27, CD3G, CD5, CD55, CD7, CDH1, CHRM3, CLEC2B, CLECL1, CR1, CR2, CRTAM, CTLA4, CXCR4, CXCR6, CYSLTR1, EMR1, EPHB6, FAS, GPR137B, GPR44, GPR68, IGF1R, IGSF4, IGSF9B, IL17RB, IL18R1, IL4R, IL9R/LOC729486, ITGA4, ITGA6, KIT, KLRB1, KLRK1, NINJ2, NT5E, P2RY14, SLAMF1, SLAMF7, SPON1, TGFBR2, TGFBR3, TNFRSF10D, TNFRSF11A, TRAV20, TRBV21-1/19/7-2/5-4/3-1/TRBC1, TRDV2 and VIPR1
and further comprises 2, 3, 4, 5, 6, 7, 8, 9 or all the gene(s) and/or corresponding coded proteins and/or antibodies (or specific hypervariable portions thereof both) being directed against these proteins encoded by these genes that are selected from the group consisting of AIF1, CCL5, CST7, F8, GZMK, IL23A, LGALS3, NOG, TNFSF10, TNFSF11, TNFSF13B and TNFSF14
and further comprises 2, 3, 4, 5, 6, 7, 8, 9 or all the gene(s) and/or corresponding coded proteins and/or antibodies (or specific hypervariable portions thereof both) being directed against these proteins encoded by these genes that are selected from the group consisting of BACH2, BATF, FOXP1, GATA3, KLF9, LM02, LM04, LM07, MSC, MYB, MYBL1, NFKBIZ, RBBP8, RUNX2, SOX4, TCEA3, TCEAL4, TRERF1, TSHZ2 and WWTR1.

Preferably, the set of the invention (further) comprises RUNX2 and/or RBBP8 gene(s) and/or corresponding coded protein(s) and/or antibodies (or specific hypervariable portions thereof both) being directed against these proteins encoded by these genes.

Preferably, the set of the invention further comprises a set consisting of 1, 2, 3, 4, 5, 6, 7, 8, 9, 10, 11, 12, 13, 14, 15, 16, 17, 18, 19, 20, 21, 22 or the 23 micro RNA(s) of Table 10.

Advantageously, The micro RNA set of the invention comprises or consists of at least 2, 3, 4, 5, 6, 7, 8, 9, 10, 11, 12, 13, 14, 15, 16, 17, 18, 19, 20, 21, 22 or all the 23 micro RNA(s) selected from the group consisting of has-let-7b, has-miR-9, has-miR-10a, has-miR-26a, has-miR-31, has-miR-95, has-miR-99a, has-miR-100, has-miR-125a, has-miR-126, has-miR-130a, has-miR-135b, has-miR-135a, has-miR-151, has-miR-181a, has-miR-193a, has-miR-213, has-miR-215, has-miR-221, has-miR-222, has-miR-335 and has-miR-340.

Preferably, the micro RNA set of the invention comprises or consists of MiR-181a and/or MiR-181b micro-RNA(s) and of at least one micro-RNA selected from the group consisting of miR-31, miRNA125a, miR-126, miR-130a, miRNA135a/b and miR-335. Preferably, the micro RNA set of the invention comprises or consists of MiR-181a and/or MiR-181b and of miRNA125a, and possibly of miRNA135a/b.

Advantageously, the gene, protein or micro RNA(s) set according to the invention, is (are) capture nucleotide sequences or capture antibodies (or specific hypervariable portions thereof) that are possibly bound to a solid support (polymeric or glass) surface, such as (according to) an array.

Advantageously, the protein set of (or the kit of) the invention comprises 2, 3, 4, 5, 6, 7, 8, 9, 10, all the coded proteins or corresponding antibodies (or specific hypervariable portions thereof) both being directed against these proteins encoded by these genes selected from the list comprising BTLA, CCL4, CCL5, CCR3, CCR6, CCR8, CD27, CD47, CD55, CD59, CD71, CD82, CD95, CD99, CD200R1, CLEC2B, CLEC2D, CR1, CR2, CRTAM, CRTH2, CTLA4, CXCR4, CXCR6, CYSLTR1, DCAL1, , , ICAM3, IGF1R, IL-4R, IL6ST, IL7R, IL9R, IL-17RB, IL-18R, , IL27RA, integrin α6, integrin α4 (ITGA4), integrin β1 (ITGB1), , LFA3 (CD58)- , SLAMF1(CD150), SLAMF5 (CD84) and SLAMF7 (CD319) proteins.

Advantageously, this protein set comprises or consist of one or two protein(s) or corresponding antibodies (or specific hypervariable portion thereof) both being directed against these proteins encoded by these genes selected from the group consisting of CCR3, CCR8, CD5, CD71, CD82, CD95, CD99, CD200R1, CRTH2, DCAL1, , ICAM3, IL-4R, IL9R, IL-17RB, integrin β1 (ITGB1), LFA3, SLAMF5, being preferably IL-17RB and/or CRTH2 proteins and one or two protein(s) or antibodies (or specific hypervariable portions thereof) both being directed against these proteins encoded by these genes selected from the group consisting of BTLA, CCL4, CCL5, CCR6, CD27, CD47, CD55, CD59, CLEC2B, CLEC2D, CR1, CR2, CRTAM, CTLA4, CXCR4, CXCR6, CYSLTR1, , IGF1R, IL6ST, IL7R, IL-18R, , IL27RA, integrin α6, integrin α4 (ITGA4), , SLAMF1 and SLAMF7, being preferably CD27 and/or CYSLTR1 proteins.

Another aspect of the present invention is a diagnostic kit or device comprising the gene set or protein set and possibly the micro RNAs set according to the invention and possibly other means for real time PCR analysis or protein analysis.

In the diagnostic kit or device (comprising the gene and possibly the micro RNAs set according to the invention) the means for real time PCR are means for qRT-PCR.

Alternatively, the diagnostic kit or device of the invention (that comprises the protein set according to the invention) contains means for protein analysis are FACS analysis detection systems or antibodies arrays.

Preferably, the diagnostic kit or device of the invention, is a computerized system comprising (the following elements):
- a bio-assay module configured for detecting a gene or micro RNA expression or protein synthesis from a blood sample based upon the gene or protein or micro RNA set according to the invention and possibly the gene or protein sets as disclosed in the present invention and
- a processor module configured to calculate expression of these genes, micro RNA or protein synthesis and to generate a risk assessment for the blood sample.

Another aspect of the invention is a method for a prognosis (prognostic) of an immune disorder, preferably the lymphocytic variant of hypereosinophilic syndrome (HES) in a mammal subject, preferably in a human patient, possibly human patients suffering from this immune disorder, which comprises the step of
- obtaining a biological sample, preferably a blood sample, possibly enriched in CD3⁻CD4⁺ cells, from the mammal subject,
- measuring gene, protein or micro RNA expression of this biological sample by putting into contact nucleotide and/or amino acids sequences obtained from this biological sample with the set of the invention or with the elements of the kit of the invention and
- possibly generating a risk assessment for this mammal subject (human patient) of being affected by this syndrome variant (L- HES) and/or of the progression of lymphocytic variant HES syndrome to T-lymphoma.

A last aspect of the invention is an anti RANKL (TNSF11) antibody (such as Denosumab) for use in the treatment of an immune disorder, being possibly L-HES, preferably as determined by using the set and/or the diagnostic and/or the method according to the invention.

A related aspect of the invention is an anti CRTH2 antibody and/or a CRTH2 antagonist for use in the treatment of an immune disorder, being possibly L-HES, preferably as determined by using the set and/or the diagnostic and/or the method according to the invention

### Short description of the drawings

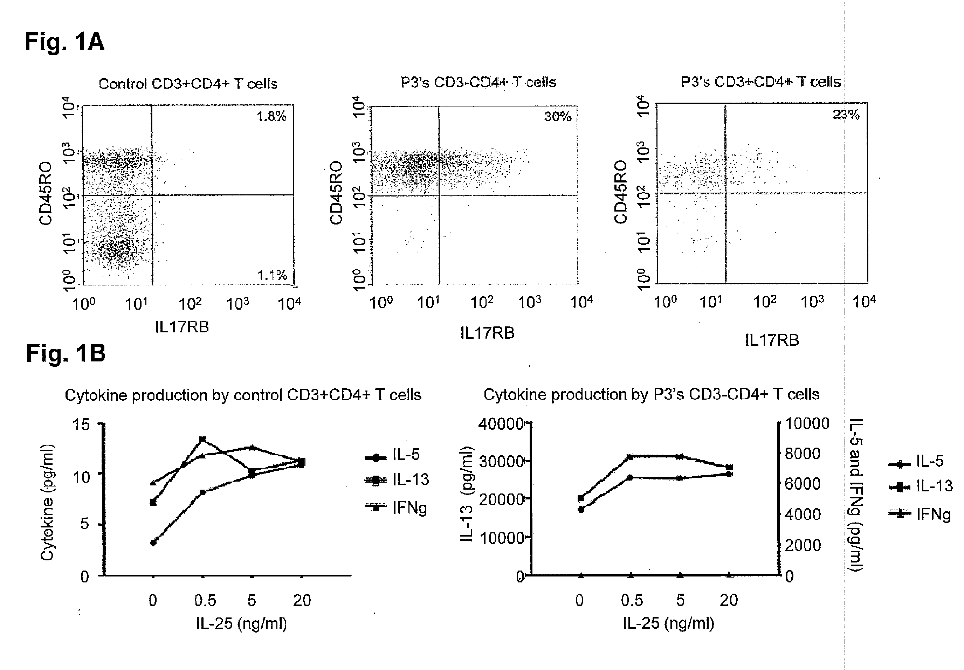

Fig. 1 represents IL-25RB (IL-25 receptor) and cytokine expression by L-HES CD3⁻CD4⁺ T-cells.
**A:** Four color immunoflourescent labelling of control and P3's PBMCs. The lymphocyte populations were gated on CD4 and CD3 positivity/negativity. **B:** Purified CD3⁻CD4⁺ T-cells from P3 were cultured for 48h with phorbol ester and anti-CD28 in the absence or presence of increasing concentrations of rhIL-25 and cytokine concentrations were determined using BD™ Cytometric Bead Array Flex Sets.

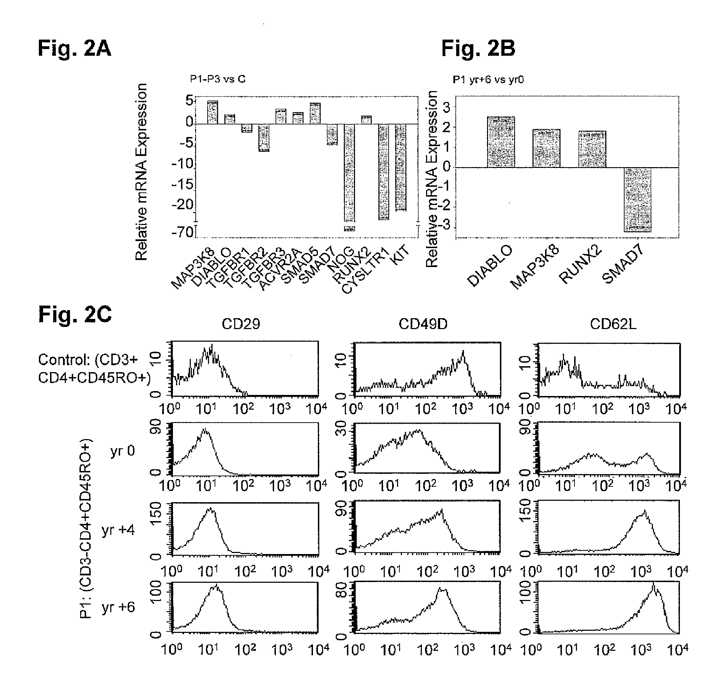

Fig. 2 represents validation of changes in gene expression using qRT-PCR and flow cytometry.
**A and B:** Fold change in the expression of selected genes measured by qRT-PCR for (A) patients (P1-P3) relative to controls (4) and (B) P1-yr+6 relative to P1-yr0. p values were calculated on three independent experiments using the student t-test and are indicated in the corresponding bar. **C:** Histograms showing the surface expression of CD29 (=ITGB1), CD49D (=ITGA4) and CD62L (=SELL) on control CD3⁺CD4⁺CD45RO⁺ T-cells and P1-yr0, P1-yr+4 and P1-yr+6 CD3⁻CD4⁺CD45RO⁺ T-cells. Isotype controls (not shown) for each sample were set between 10⁰ and 10¹.

### Detailed description of the invention

Patients: Patients with hypereosinophilic syndrome (hES) were selected for cohort inclusion based on the presence of a monoclonal CD3⁻CD4⁺ T-cell population in their peripheral blood. The clinical characteristics of all patients analyzed are summarized in Table 1. Informed consent was obtained from all patients and this study was approved by the local ethics committee.

One patient (P1) developed T-lymphoma after a 6-year follow-up, which was formally demonstrated by histopathological analysis of enlarged cervical lymph nodes; the infiltrating T-cells were shown to be CD3⁻CD4⁺. For this patient, blood was drawn at 3 time-points; yr0 and yr+4 (were during the chronic disease phase) while yr+6 was drawn at the time of lymphoma diagnosis.

**Table 1: Clinical characteristics of L-HES patients**

| **Patient** | **Sex** | **Diagnosis** | **Age at evaluation** | **Follow-up samples** | **Clinical Stage** | **Total WBC (#/µl)** | **Eosinophils (#/µl)** | **Lymphocytes (#/µl) (% CD3^{η}CD4⁺)** | **Refs** |
|---|---|---|---|---|---|---|---|---|---|
| P1 | F | L-HES | 16 | year 0 | chronic disease | 16.900 | 8.920 | 4630 (75%) | 1-3 |
| | | | | year +4 | chronic disease | 26.200 | 17.100 | 6320(76%) | |
| | | | | year +6 | T lymphoma a | 15.100 | 8.610 | 4000 (73%) | |
| P2 | F | L-HES | 21 | year 0 | chronic disease | 14.800 | 9.100 | 3420 (72%) | 1-3 |
| | | | | year +4 | chronic disease | 7.200 | 660 | 1420 (6%) | |
| P3 | M | L-HES | 39 | ^{a} | chronic disease | 7.740 | na^{b} | 4790 (70,9%) | |
| P4 | F | L-HES | 47 | ^{a} | chronic disease | 7.800 | 2.970 | 2239 (8%) | 1,3 |
| P5 | M | L-HES | 20 | na^{b} | chronic disease | na^{b} | 9.500 | 10,900 (96%) | 4 |
| P6 | M | L-HES | 44 | ^{c} | chronic disease | 12.700 | 1.067 | 3531 (25%) | |
| P7 | M | ATLL/eosinophilia | 48 | ^{d} | T lymphoma | 39.800 | 3.050 | 31700(53%) | |

| | | | | | | | | | |
|---|---|---|---|---|---|---|---|---|---|
| ^{a}: P1 was not receiving any treatment at the time points assessed on microarrays. She did received fludarabine for six months in yr+4, but this was started 6 months after our sampling. ^{b}: P2 was on low dose corticosteroids at yr+4 and this was alternate day medrol 8 mg/ 0 mg. ^{c}: P3 - persisting hypereosinophilia with a stable CD3-CD4+ T-cell population ^{d}: persisting hypereosinophilia with a stable CD3-CD4+ T-cell population ^{e}: not available ^{f}: currently in complete remission after successful interferon-alpha and corticosteriod therapy ^{g}: deceased shortly after this sampling from the T cell lymphoma | | | | | | | | | |

### Cell purification

Circulating leukocytes were obtained from the peripheral blood of patients and healthy donors either by venipuncture or cytapheresis. Peripheral blood mononuclear cells (PBMC) were isolated and frozen as previously described by Roufosse F et al. (1999). PBMC were thawed and resuspended in X-vivo-20 medium (Lonza). CD4⁺ T-cells were isolated from control and patient PBMCs by negative selection using the CD4+ T-cell Isolation Kit II (MACS - Miltenyi Biotech) (Ravoet M et al. Haematologica (2005)). The CD3⁺CD4⁺ T-cells from patients were depleted from the total CD4⁺ T-cell population using mouse anti-human CD3 (Pharmingen) and Dynabead sheep anti-mouse IgG (Dynal; following the manufacturers protocol) to produce the purified CD3⁻CD4⁺ T-cells. After purification, flow cytometry was used to determine the number of contaminating cells:
a) in the patient CD3⁻CD4⁺ population there were a small number of contaminating CD3⁺CD4⁺ T-cells (range 0.2% to 1.8%) and CD3⁻CD4⁻ cells (range 0.4% to 2.4%; likely B cells) and
b) in the control CD3⁺CD4⁺ T-cells there were some CD3⁺CD4⁻ T-cells (range 1.1% to 2.6%; likely CD8⁺ T-cells) and CD3⁻CD4⁻ (range 1.2% to 7.0%; likely B cells). Purified T-cells were incubated in X-vivo-20 at 37°C/5% CO₂ for 18 hours to eliminate dying cells prior to RNA extraction.

The isolated cell populations were checked for purity by flow cytometry and were consistently >95% pure for the CD3⁻CD4⁺ patient T-cells and >90% pure for the CD3⁺CD4⁺ control T-cells. Purified T-cells were incubated in X-vivo-20 at 37°C/5% CO₂ for 18 hours to eliminate dying cells prior to RNA extraction.

For the co-stimulation experiments, purified CD3-CD4+ T-cells were cultured for 18 hours in X-vivo-20 supplemented with rhIL-2 (100U/mL) and anti-CD28 (CLBCD28/1; 1 µg/mL) plus two anti-CD2 antibodies (CLB-T11.1/1 and CLB-T11.2/1; 5 µg/mL each) (Sanquin). In the IL-25 experiments purified CD3-CD4+ (patient) and CD3+CD4+ (patient and control) T-cells were stimulated for 48h with phorbol ester (10 ng/ml) and anti-CD28 in the absence or presence of increasing concentrations of rhIL-25 (R&D systems).

Flow cytometry : Flow cytometric analysis was performed by labelling 2-5x10⁵ cells with 5µl FITC-conjugated anti-CD45RO, 5µl PerCP-conjugated anti-CD3, 5µl APC-conjugated anti-CD4 and 5µl of the PE-conjugated test antibody (Becton-Dickenson; Table2) in 50µl X-vivo-20. 10,000 viable cells were acquired on a FACS Calibur. Measurement of cytokine concentrations in culture supernatants were performed using BD™ Cytometric Bead Array Flex Sets.

**Table 2 Flow cytometric analysis of surface marker expression on patients' vs controls**

| **Flow cytometry : Results on Lymphocytes CD4+ *⁽¹⁾: P3-Yr +3 vs Control K** | | | | | | |
|---|---|---|---|---|---|---|
| | **P3-Yr +3** | **Control K** | FACS | **P3-Yr +3** | **Control K** | **FACS** |
| **PE** | fresh whole blood % | fresh whole blood % | % *⁽²⁾ | | | **Fluo Mean ^{*(3)}** |
| **conjugated** | | | Fold | Fluo Mean Gate All ^{*(3)} | | Fold |
| **Ab** | **RO 4+3- *⁽⁴⁾** | **CD4+3+ *⁽⁵⁾** | Change | **RO 4+3-** | **CD4+3+** | Change |
| **BTLA** | 40,4 | 93,7 | **-2,32** | 32 | 222 | **-6,94** |
| **CCR2 (CD192)** | 19,3 | 6,7 | **2,88** | 17,2 | 5,5 | **3,13** |
| **CCR3 (CD193)** | 73,5 | 70,4 | **1,04** | 52 | 28,6 | **1,82** |
| **CCR5 (CD195)** | 4,7 | 18,2 | **-3,87** | 5,5 | 14,8 | **-2,69** |
| **CCR7 (CD197)** | 26,4 | 64,8 | **-2,45** | 12,6 | 49,8 | **-3,95** |
| **CCR8 (CDw198)** | 9,5 | 1,2 | **7,92** | 6,7 | 3,5 | **1,91** |
| **CXCR4 (CD184)** | 78,3 | 99,9 | **-1,28** | 50 | 696 | **-13,92** |
| **CXCR5 (CD185)** | 14,2 | 15,7 | **-1,11** | 11,8 | 25 | **-2,12** |
| **CD5** | 99,8 | 100 | **1** | 4227 | 2816 | **1,5** |
| **CD7** | 6,7 | 94,5 | **-14,1** | 28 | 1119 | **-39,96** |
| **CD27** | 0,7 | 95,7 | **-136,7** | 5 | 782 | **-156** |
| **CD29 (ITGB1)** | 93,5 | 1,1 | **85** | 66 | 4,8 | **13,75** |
| **CD49 D (ITGA4)** | 20,4 | 83,2 | **-4,08** | 17 | 289 | **-17** |
| **CD55R (DAF)** | 100 | 99,8 | **1** | 354 | 554 | **-1,56** |
| **CD59R** | 12,8 | 25,3 | **-1,98** | 11,7 | 26 | **-2,22** |
| **CD62L(SELL)** | 44,2 | 69,2 | **-1,57** | 63 | 85 | **-1,35** |
| **CD69** | 5,2 | 2,2 | **2,36** | 6,5 | 4,6 | **1,41** |
| **CD71 (TFRC)** | 0,6 | 0,14 | **4,29** | 4,4 | 3,6 | **1,22** |
| **CD73 (NTSE)** | 0,3 | 20,7 | **-69** | 4,2 | 25 | **-5,95** |
| **CD95 (FAS)** | 99,4 | 82,1 | **1,21** | 157 | 270 | **-1,72** |
| **CD127 (IL-7Ra)** | 99,1 | 91,1 | **1,09** | 269 | 97,5 | **2,76** |
| **CD130 (IL-6ST)** | 58,8 | 57,4 | **1,02** | 18,5 | 25,5 | **-1,38** |
| **CRTH2 (GPR44)** | 96,4 | 0,8 | **120** | 182 | 3,8 | **47,89** |
| **IL-17RB** | 36,7 | 2,7 | **13,59** | 33,4 | 6,1 | **5,48** |
| **FAS Ligand** | 10,2 | 1,9 | **5,37** | 7,5 | 5,4 | **1,39** |
| **BTLA** | 40,4 | 87,6 | **-2,17** | 32 | 228 | **-7,13** |
| **CCR2 (CD192)** | 19,3 | 12,6 | **1,53** | 17,2 | 6,6 | **2,61** |
| **CCR3 (CD193)** | 73,5 | 54,3 | **1,35** | 52 | 22 | **2,36** |
| **CCR5 (CD195)** | 4,7 | 37,7 | **-8,02** | 5,5 | 27 | **-4,91** |
| **CCR7 (CD197)** | 26,4 | 36 | **-1,36** | 12,6 | 28 | **-2,22** |
| **CCR8 (CDw198)** | 9,5 | 1,7 | **5,59** | 6,7 | 4 | **1,68** |
| **CXCR4 (CD184)** | 78,3 | 100 | **-1,28** | 50 | 803 | **-16,06** |
| **CXCR5 (CD185)** | 14,2 | 22,1 | **-1,56** | 11,8 | 37 | **-3,14** |
| **CD5** | 99,8 | 100 | **1** | 4227 | 3435 | **1,23** |
| **CD7** | 6,7 | 88,3 | **-13,18** | 28 | 670 | **-23,93** |
| **CD27** | 0,7 | 91,4 | **-130,6** | 5 | 651 | **-130** |
| **CD29 (ITGB1)** | 93,5 | 2 | **46,75** | 66 | 5,3 | **12,45** |
| **CD49 D (ITGA4)** | 20,4 | 71,6 | **-3,51** | 17 | 391 | **-23** |
| **CD55R (DAF)** | 100 | 99,7 | **1** | 354 | 441 | **-1,25** |
| **CD59R** | 12,8 | 24,5 | **-1,91** | 11,7 | 29 | **-2,48** |
| **CD62L (SELL)** | 44,2 | 38 | **1,16** | 63 | 48 | **1,31** |
| **CD69** | 5,2 | 3 | **1,73** | 6,5 | 5,3 | **1,23** |
| **CD71 (TFRC)** | 0,6 | 0,4 | **1,5** | 4,4 | 4 | **1,1** |
| **CD73 (NT5E)** | 0,3 | 12,9 | **-43** | 4,2 | 27 | **-6,43** |
| **CD95 (FAS)** | 99,4 | 98,1 | **1,01** | 157 | 543 | **-3,46** |
| **CD127 (IL-7Ra)** | 99,1 | 83,3 | **1,19** | 269 | 93 | **2,89** |
| **CD130 (IL-6ST)** | 58,8 | 19,4 | **3,03** | 18,5 | 9 | **2,06** |
| **CRTH2 (GPR44)** | 96,4 | 1,2 | **80,33** | 182 | 4,4 | **41,36** |
| **IL-17RB** | 36,7 | 3,7 | **9,92** | 33,4 | 6,8 | **4,91** |
| **FAS Ligand** | 10,2 | 3,2 | **3,19** | 7,5 | 4,7 | **1,6** |

| | | | | | | |
|---|---|---|---|---|---|---|
| (1) : Comparison between the patient CD4+CD3-CD45RO+hi T cells and the control CD4+CD3+ T cells (2) : Percentage of cells positive for PE conjugated Ab (3) : Mean fluorescence intensity of the whole population gated (4) : CD4+CD3-CD45RO+hi lymphocytes (5) : CD4+CD3+ lymphocytes (6) : CD4+CD3+CD45RO+hi lymphocytes (7) : Comparison between the patient CD4+CD3-CD45RO+hi cells and the control CD4+CD3+CD45RO+hi cells. | | | | | | |

### RNA extraction and gene expression microarray procedures:

RNA was extracted by the standard single-step method of isolation using Trizol (Invitrogen). RNA quantity and quality were assessed using a NanoDrop spectrophotometer (Thermo Scientific) and a Bioanalyzer (Agilent). 1.5µg of total RNA was labeled following the manufacturers protocols for probe preparation and hybridization (Affymetrix). Briefly, total RNA was reverse-transcribed using a T7 oligo dT(24) primer and Superscript II (Invitrogen). Second-strand cDNA synthesis was obtained with RNase H, E. coli DNA polymerase I and *E*. *coli* DNA ligase (Invitrogen). cDNA was blunt-ended with T4 DNA polymerase (Invitrogen) and purified using the Genechip sample module cleanup (Affymetrix). Labeling was performed using the GeneChip IVT Labeling kit (Affymetrix) according to the manufacturer's instructions. After purification, the labeled cRNA was quantified by OD and the quality was assessed on the Bioanalyzer. The probe preparation, hybridization, washing, staining and scanning of the array slides were performed according to standard protocols (Affymetrix). Hybridization (of 15µg of cRNA) was performed using Affymetrix U133 Plus 2.0 Genechips.

### Real-time reverse transcription polymerase chain reaction (qRT-PCR)

Total RNA (200ng-1µg) was reverse-transcribed with random hexanucleotides using the Superscript III First-Strand Synthesis System (Invitrogen) and standard protocols. The cDNA (12 ng/reaction) were subjected to a real-time PCR reaction using 2x SYBR Green PCR Master Mix (Applied Biosystems) and Qiagen QuantiTect Primer Assays.

The primers were specific for the MAP3K8, RUNX1, RUNX2, DIABLO, TGFBR1, TGFBR2, TGFBR3, KIT, SMAD5, SMAD7, NOG, ACVR2A, CYSLTR1, ABL gene 3 and CASC3 genes.

qRT-PCR was performed on a Roche LightCycler 480 (Roche Applied Science) and conducted under universal cycling conditions (40 cycles). LC Basic Software, v.1.2 was used for data analysis. Dissociation curves were verified to ensure the specificity of all amplification products.

All real-time PCR reactions were processed in duplicate and differences of more than 0.5 Ct were rejected. Biological replicates were done for P1-yr.0, P1-yr+4 and P1-yr+6. The relative expression was calculated according to the ΔΔCt method, using the Ct average of CASC3 and ABL endogenous controls for normalisation. P-values were obtained using the student t-test based on normalised ΔCt values.

Analyses (detailed in Table 3) were performed using LC Basic Software, v.1.2.

**Table 3: qRT-PCR confirmation of fold-changes in gene expression detected in the arrays**

| **GB Acc. No.** | **Symbol** | **Name** | **3P vs 4C^{a}** | | **6P vs 4C^{b}** | |
|---|---|---|---|---|---|---|
| | | | **Fold Change^{c}** | ***p*-value*^{d}*** | **Fold Change** | ***p*-value** |
| NM_005204 | MAP3K8 | mitogen-activated protein kinase kinase kinase 8 | 5,1 | 0,005 | 4,9 | 0,001 |
| NM_019887 | DIABLO | diablo homolog (Drosophila) | 2,1 | 0,003 | 2,3 | 0,006 |
| NM_004612 | TGFBR1 | transforming growth factor, beta receptor I (activin A receptor type II-like kinase, 53kDa) | -1,9 | 0,055 | -2,4 | 0,045 |
| NM_003242 | TGFBR2 | transforming growth factor, beta receptor II (70/80kDa) | -6,1 | 0,036 | -4,5 | 0,002 |
| NM_003243 | TGFBR3 | transforming growth factor, beta receptor III (betaglycan, 300kDa) | 3,3 | 0,002 | 2,9 | 0,002 |
| NM_001616 | ACVR2A | activin A receptor, type IIA | 2,6 | 0,002 | 1,6 | 0,244 |
| NM_005903 | SMAD5 | SMAD, mothers against DPP homolog 5 (Drosophila) | 4,6 | <0.001 | 1,8 | 0,383 |
| NM_005904 | SMAD7 | SMAD, mothers against DPP homolog 7 (Drosophila) | -4,6 | 0,017 | -5,3 | 0,001 |
| NM_005450 | NOG | noggin | -68,9 | 0,001 | -10,3 | 0,047 |
| NM_001024630 | RUNX2 | runt-related transcription factor 2 | 1,8 | 0,049 | -1,5 | 0,654 |
| NM_006639 | GYSLTR1 | cysteinyl leukotriene receptor 1 | -21,6 | 0,132 | -7,4 | 0,168 |
| NM_000222 | KIT | v-kit Hardy-Zuckerman 4 feline sarcoma viral oncogene homolog | -19,7 | 0,001 | -14,3 | 0,002 |

| | | | | | | |
|---|---|---|---|---|---|---|
| ^{a,b}: Expression fold change of MAP3K8, DIABLO, TGFBR1, TGFBR2, TGFBR3, ACVR2A, SMAD5, SMAD7, NOG, RUNX2, CYSLTR1 and KIT genes, measured by real-time RT-PCR in a3 patients (P1-yr0, P2 and P3) relative to four controls and in b6 patients (P1-yr0, P2, P3, P4, P5, P7) relative to four controls. The ABL and CASC3 genes were used as endogenous controls. Dissociation curves were verified to ensure the specificity of the PCR reactions. All real-time PCR reactions were processed in duplicate and differences of more than 0.5 Ct were rejected. ^{c}: Genes Ct values were normalised by the average of CASC3 and ABL Ct values and fold changes were calculated using the ΔΔCt method. ^{d}: p-values were obtained using student t-test based on normalised ΔCt values. | | | | | | |

### MicroRNA quantification

Quantification of mature microRNAs was achieved using stem-loop-mediated reverse transcription RT-PCR with the TaqMan microRNA assay-early access kit or with individual microRNA assay mixes (based on Sanger miRbase v.8.2 as purchased from Applied Biosystems), according to the manufacturer's protocols.

Briefly, 10ng total RNA was reverse-transcribed in a 15µl-volume with the TaqMan miRNA reverse transcription kit using specific primers for each miRNA. Next, 1.33µl of the reverse-transcription reaction was taken for quantification using Taqman 2x universal PCR master mix with the specific primers and probe for each miRNA.

For the screening experiments, qRT-PCR was performed with the TaqMan miRNA assay-early access kit (Applied Biosystems) using hsa-let-7a as an endogenous control. Up- and downregulated miRNAs in abnormal CD3-CD4+ T-cells from P1-yr.6 were determined on the basis of a >2-fold change in comparison with normal CD3+CD4+ T-cells from a healthy control based on the comparative CT method in two independent experiments.

For assessment of all the patients as a group, specific miRNA assay mixes were used and normalization was performed using the average of let-7a and RNU48.

All qRT-PCR reactions were performed in triplicate with Ct value standard deviations set maximal to 1. Experimental duplicates for the 4 controls, P2-yr.0, P3-yr.0, P4, P5 and P7 and triplicates for P1-yr.0, P1-yr.4 and P1-yr.6 were normalized and averaged. P-values based on normalized ΔCt values were calculated using the student t-test and corrected by FDR (False discovery rate) in the program R, version 2.3.0 (a language and environment for statistical computing and graphics, available from http://www.r-project.org/).

Relative expression was calculated by the ΔΔC_{T} method. For each miRNA (except miR-213) whose expression varied in patients relative to controls, a list of the target genes predicted by the MiRanda algorithm-associated MirBase software (http://microrna.sanger.ac.uk); that are also deregulated in the microarrays experiments were uploaded (release 10.0).

### Statistical analyses

In order to identify genes consistently deregulated in three patients compared with four healthy donors, the inventors selected genes with each patient's p-value (generated by S-score algorithm and mean S-score value's Z transformation inferior to 0.05 separately compared to all donors. A further filter was applied to probe sets with low significance. Similar analysis was performed to find deregulated genes for P1's evolution from chronic to malignant disease.

Raw data were analyzed using the *SScoreBatch* (Zhang L et al. (2002), (Kennedy RE et al. (2006)) function from the *SScore* package (v.1.5.1) in the R statistical environment (v.2.3.0, http://www.r-project.org/; http://www.bioconductor.org). In order to identify genes consistently deregulated in the three patients relative to the four controls, the inventors selected genes where each patient's p-value (generated by S-score algorithm and mean S-score value Z transformation) was inferior to 0.05 in comparison with individual controls (See Table 5, below). A further filter was applied to probe sets of low significance. Similar analyses were performed to determine the genes deregulated during P1's evolution from chronic disease to T-lymphoma (See Table 6, below).

### Results

### Comprehensive gene expression analysis of CD3⁻CD4⁺ T-cells from L-HES patients

The inventors have compared the gene expression profiles of clonal CD3⁻CD4⁺ T-cells isolated from L-HES patients (P1-P3; Table 1) during chronic disease with CD3⁺CD4⁺ T-cells from controls (healthy donors).

The inventors also evaluated changes in gene expression associated with anti-CD2/CD28 activation of their CD3⁻CD4⁺ T-cells *in vitro,* an antibody combination targeting co-stimulatory receptors previously shown to mediate their Th2 cytokine production and proliferation.

The changes in gene expression associated with P1's clinical progression (Ravoet M et al. (2005), Willard-Gallo KE et al (2005)) was further analyzed by assessing CD3⁻CD4⁺ T-cells at diagnosis (yr0), follow-up at yr+4, both of which are pre-malignant stages of chronic L-HES and follow-up at yr+6 concurrent with T-lymphoma diagnosis. Following comprehensive and stringent statistical analyses the inventors detected 850 genes (=1397 probe sets) that were differentially regulated in all three patients CD3⁻CD4⁺ T-cells compared with control CD3⁺CD4⁺ T-cells (Tables 4 and 5), 312 genes (=411 probe sets) that were altered in all three patients CD3⁻CD4⁺ T-cells after CD2/CD28 co-stimulation (Table 8) and 349 genes (=450 probe sets) whose expression was altered in concert with P1' s malignant evolution (Table 9). The original data for all 54,675 probes from each array are provided at http://www.ncbi.nlm.nih.gov/projects/geo/query/acc.cgi?acc= GSE12079.

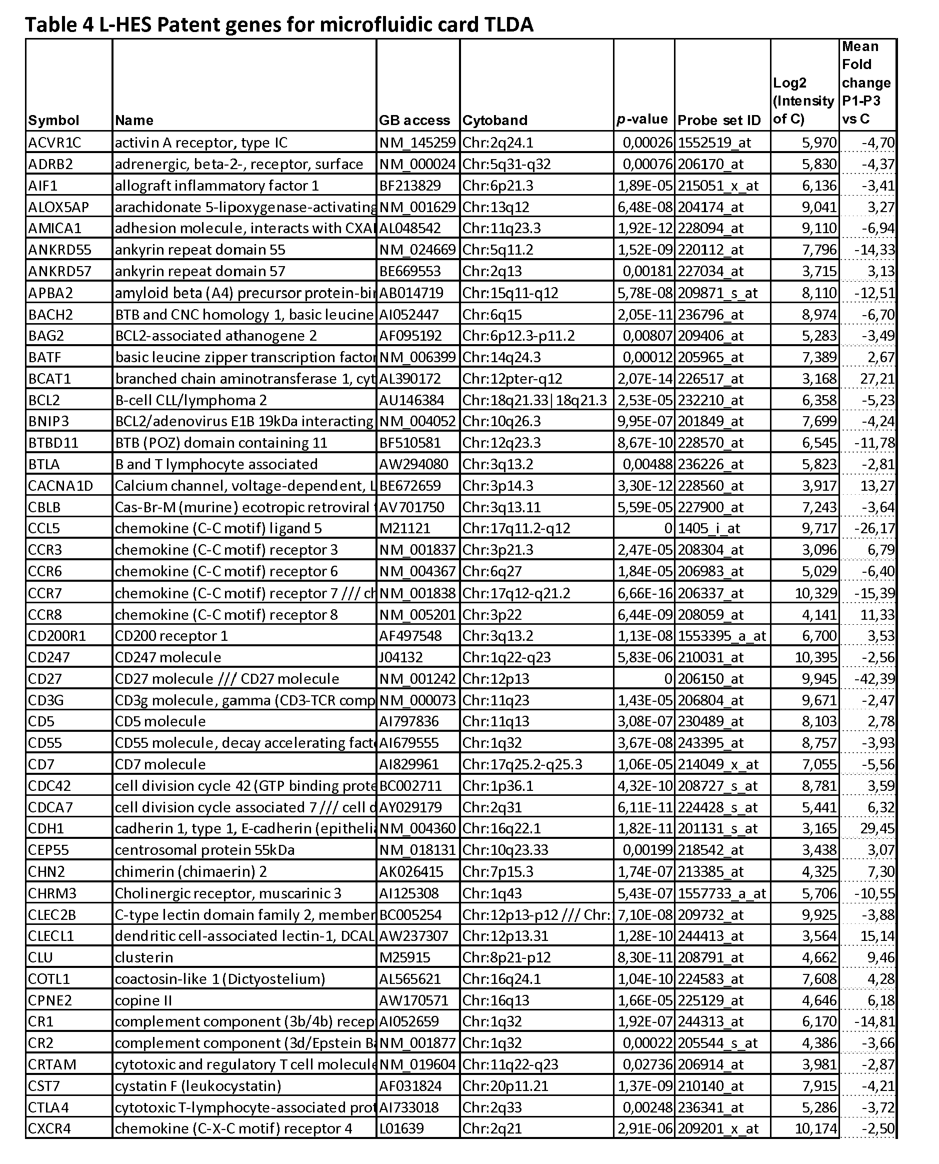

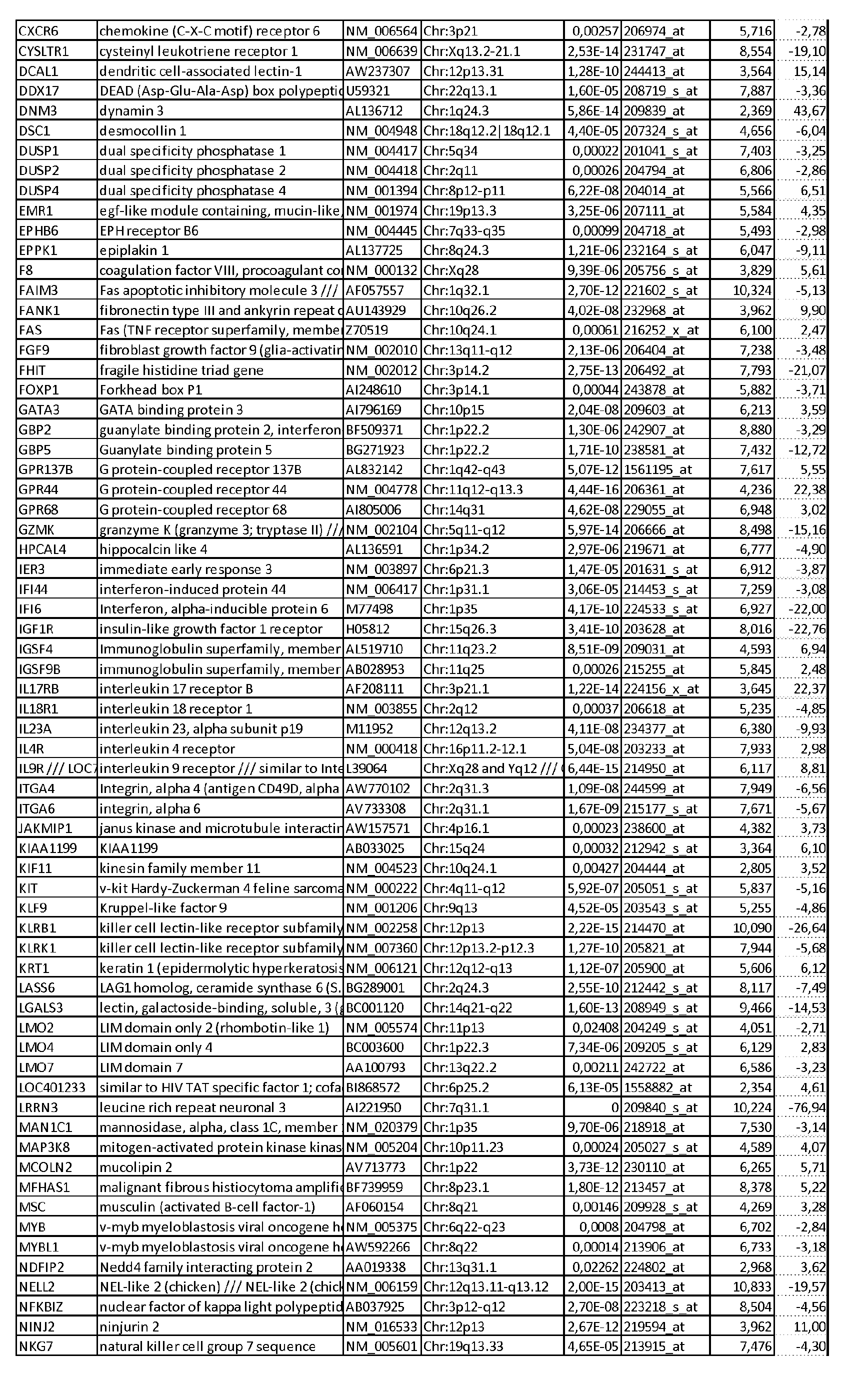

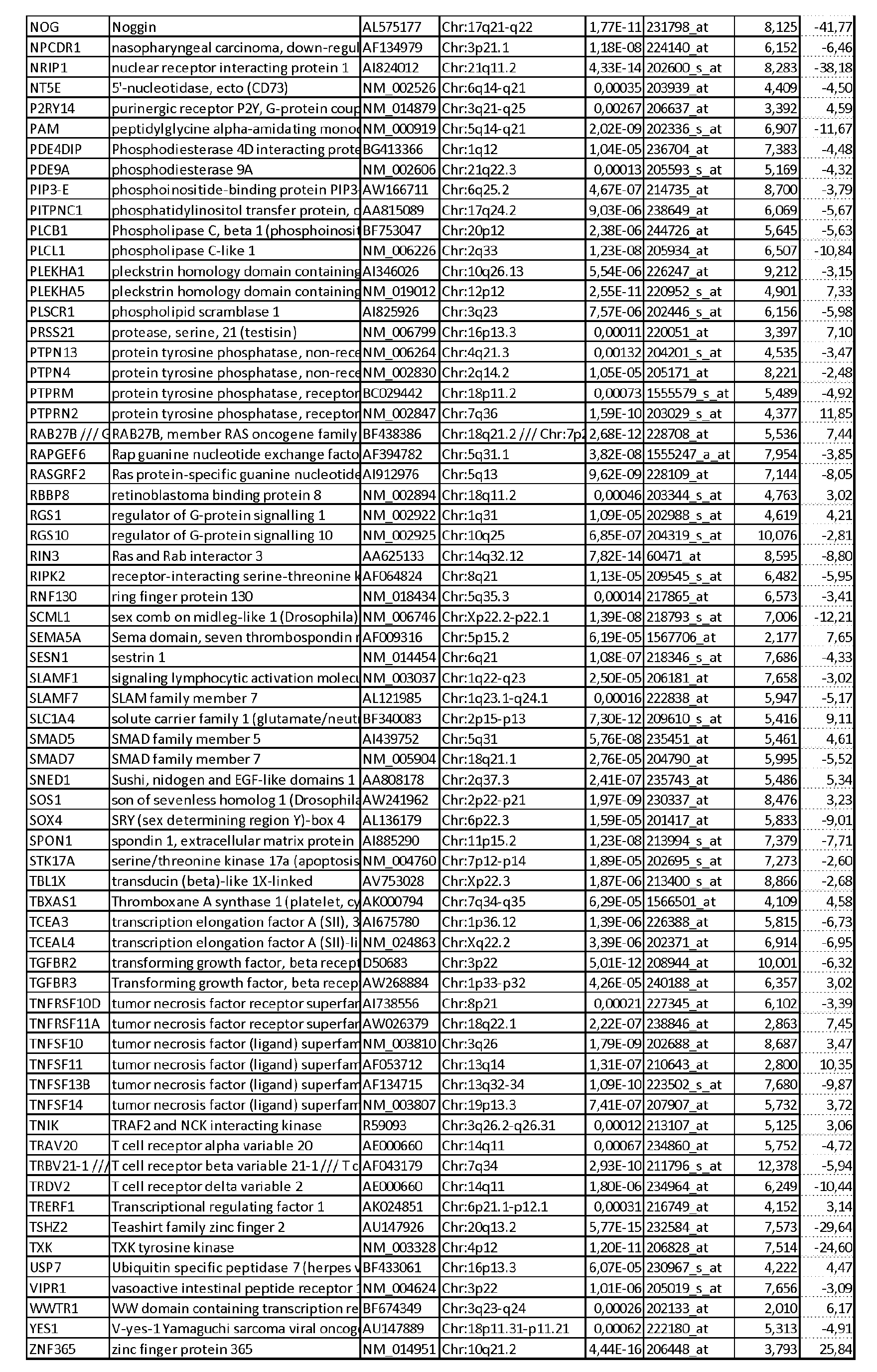

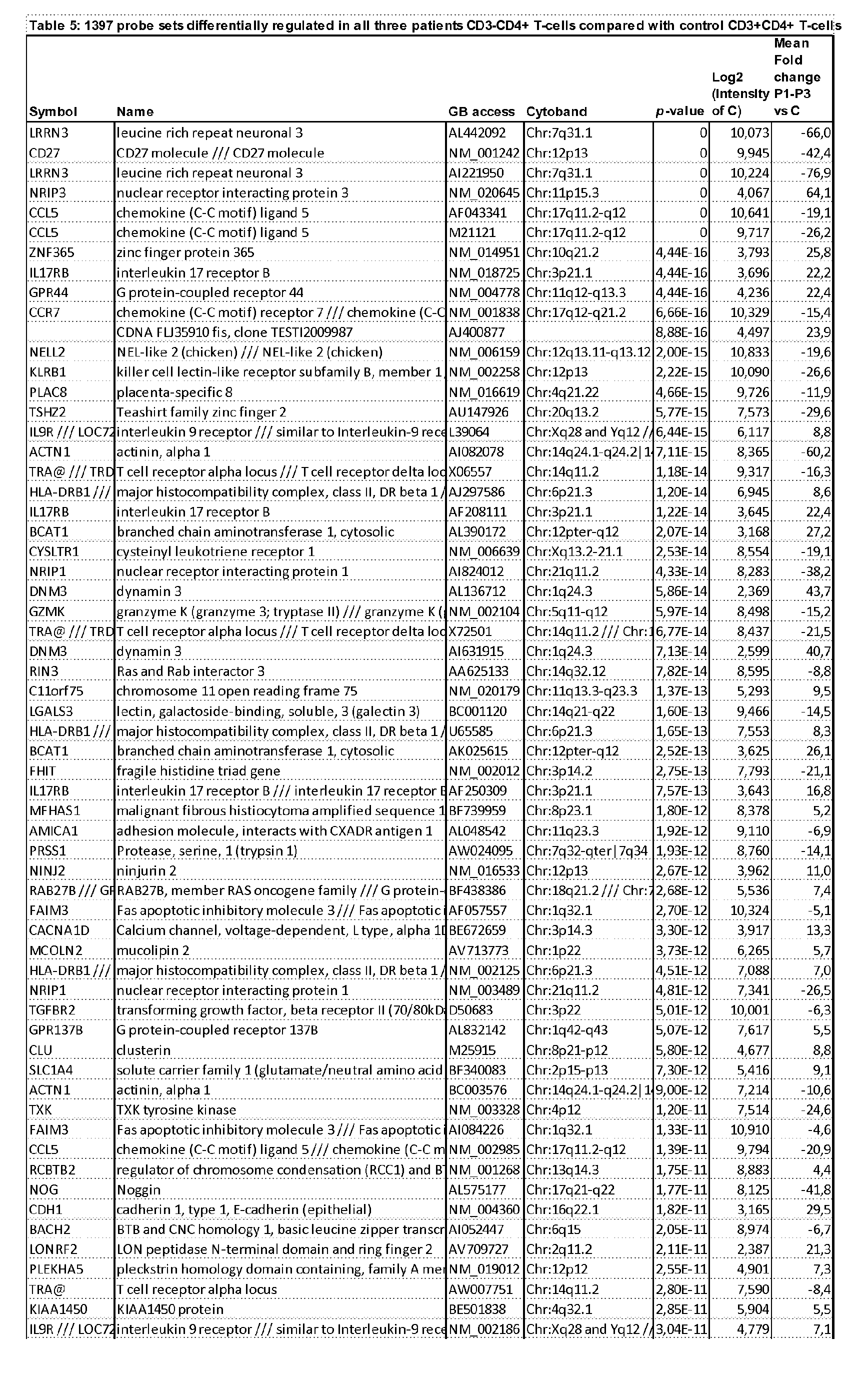

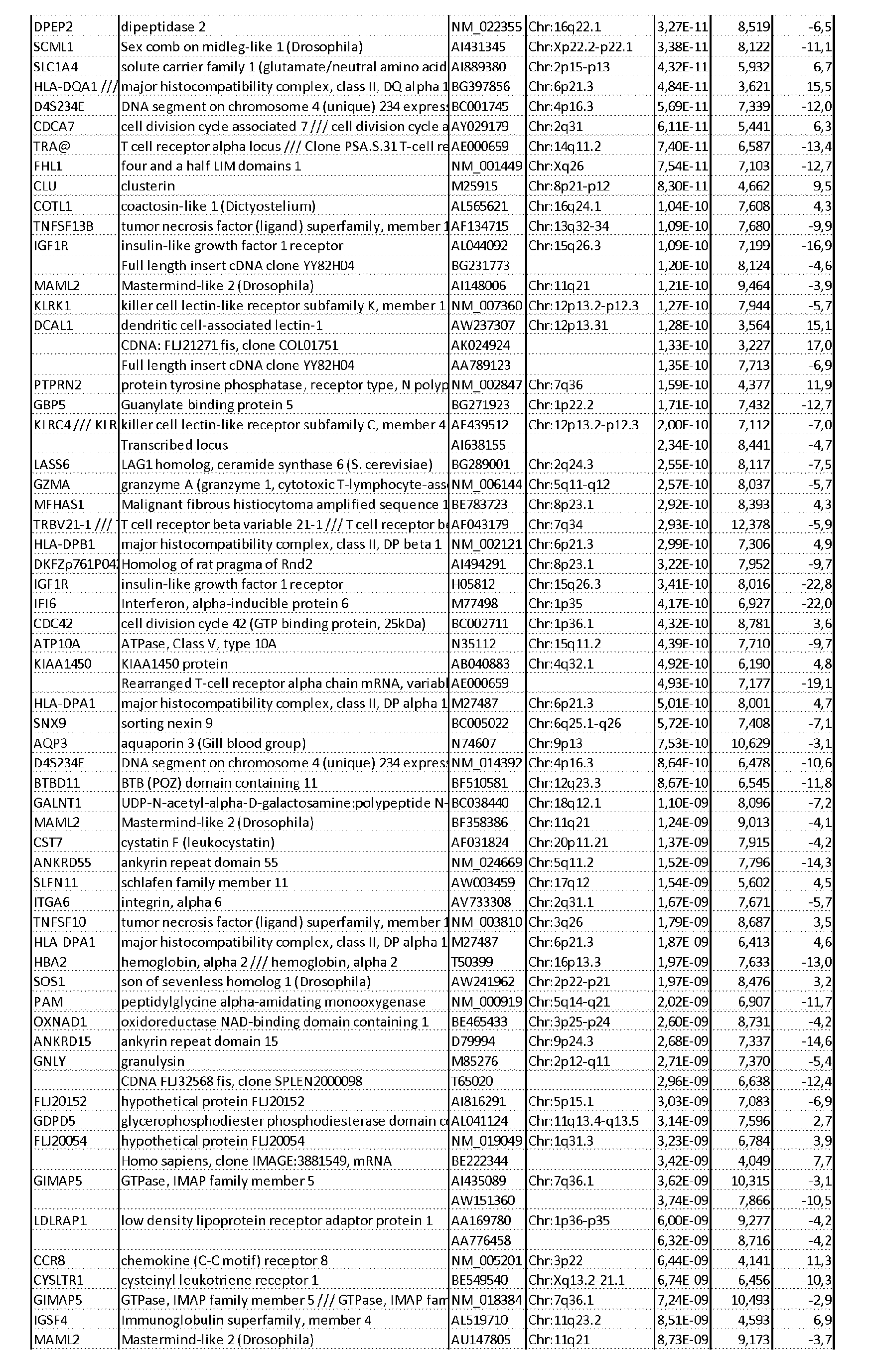

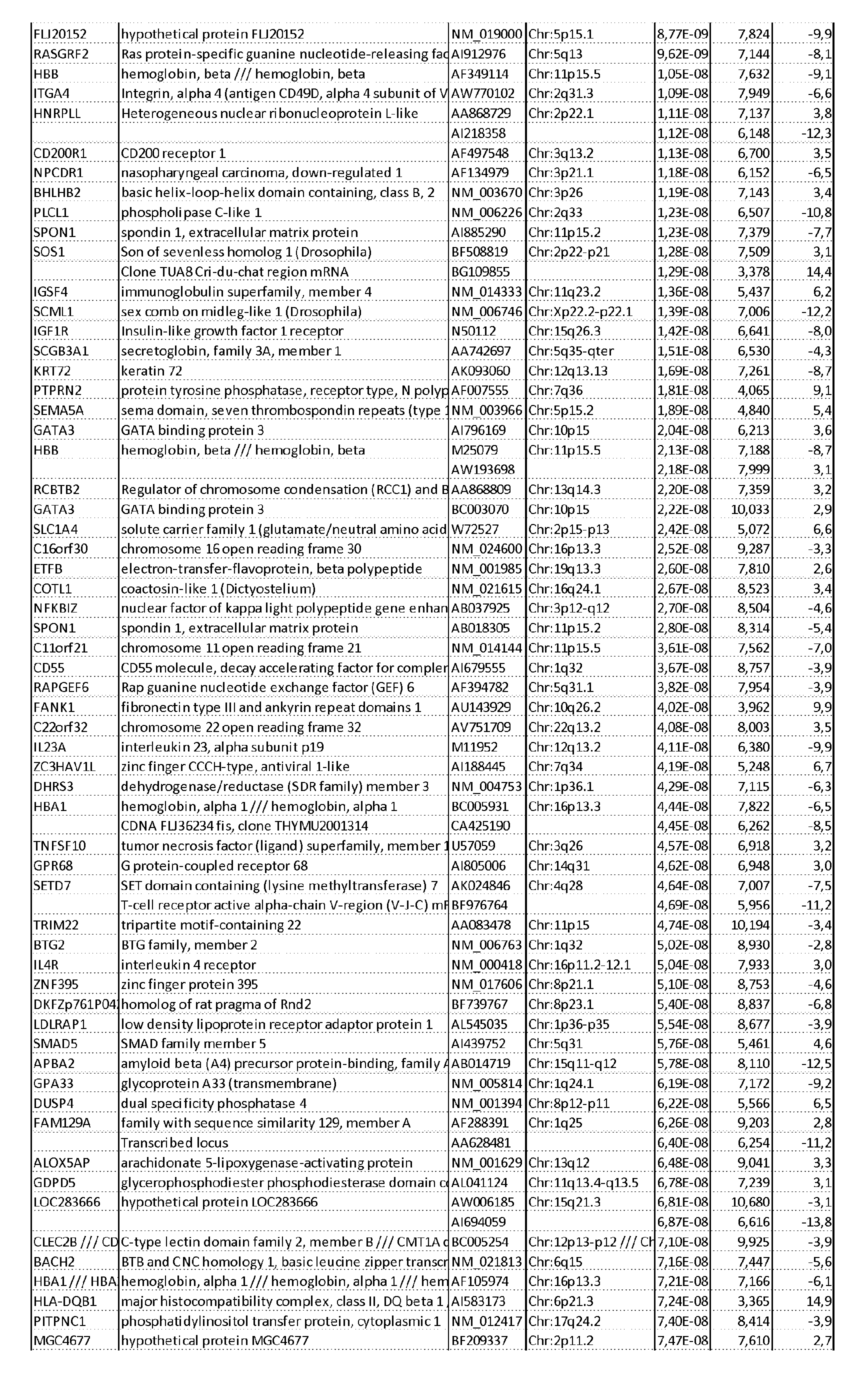

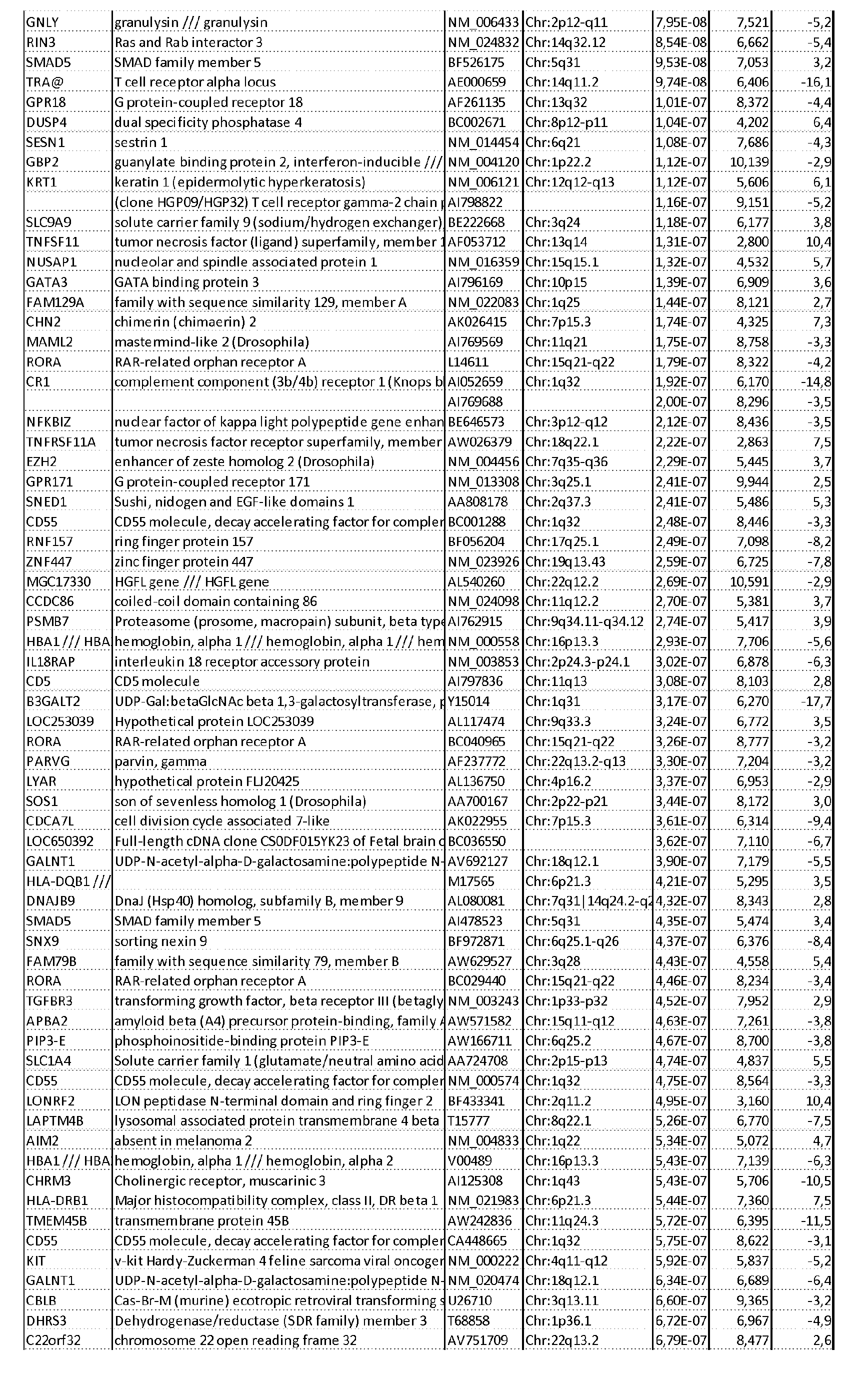

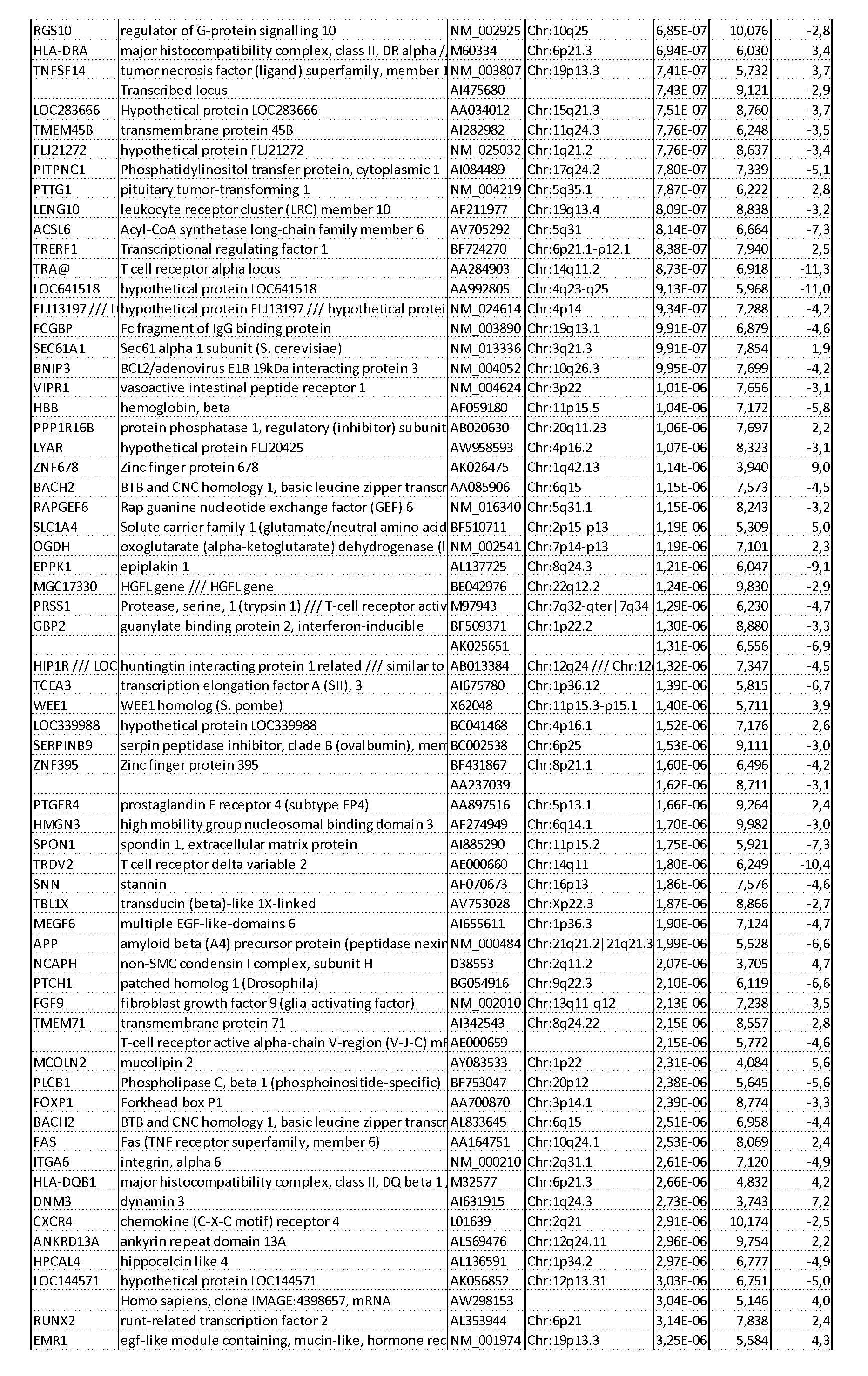

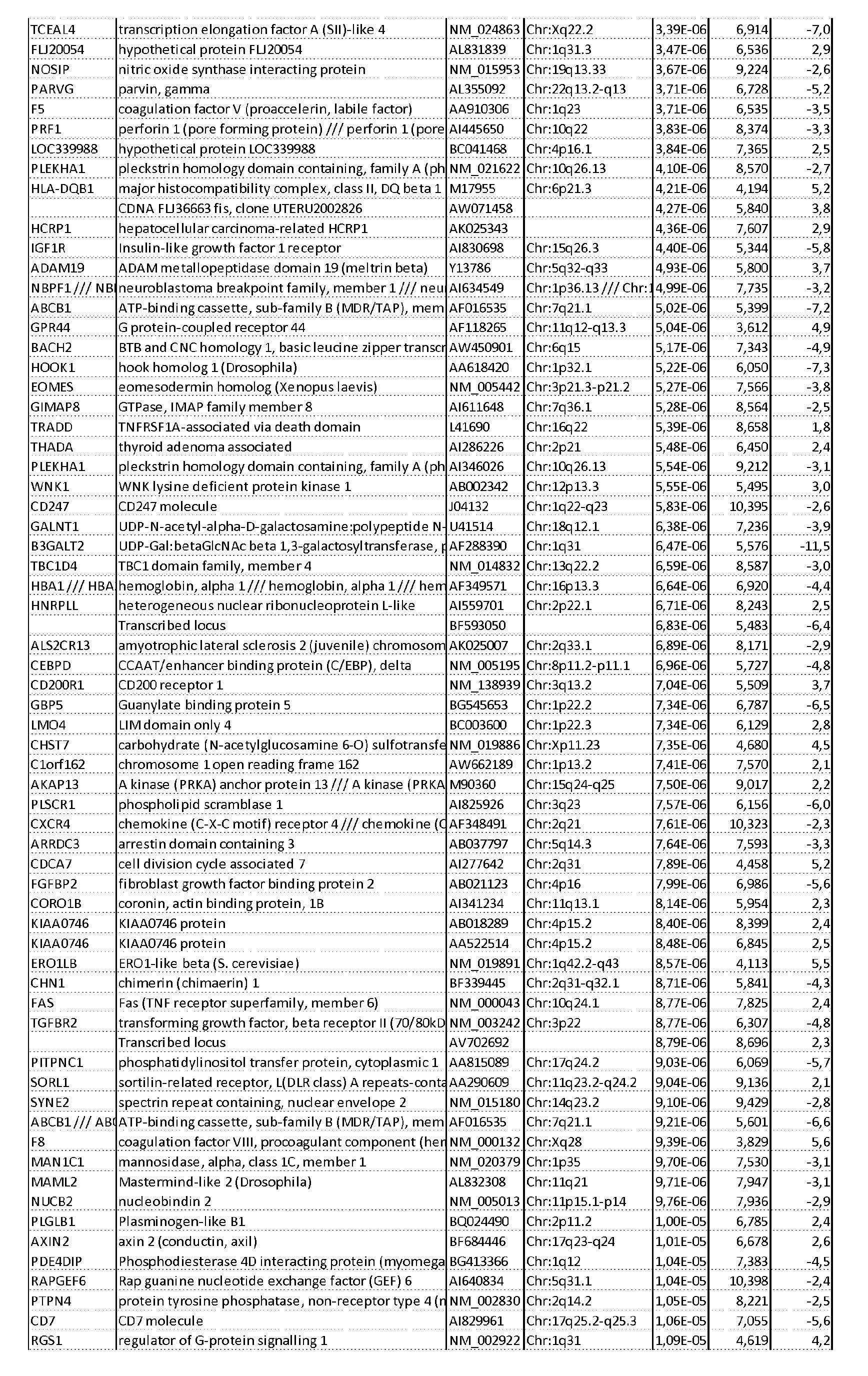

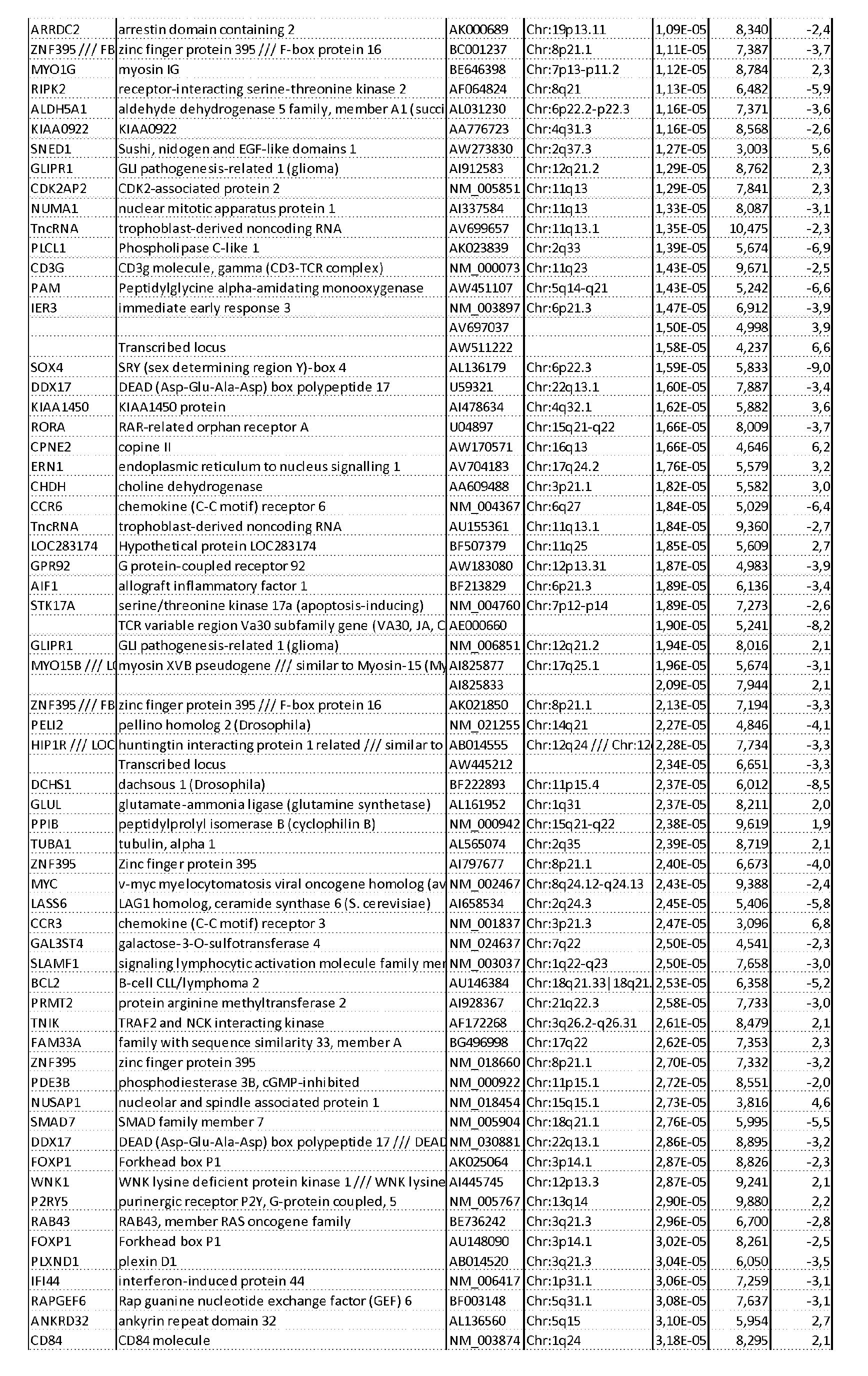

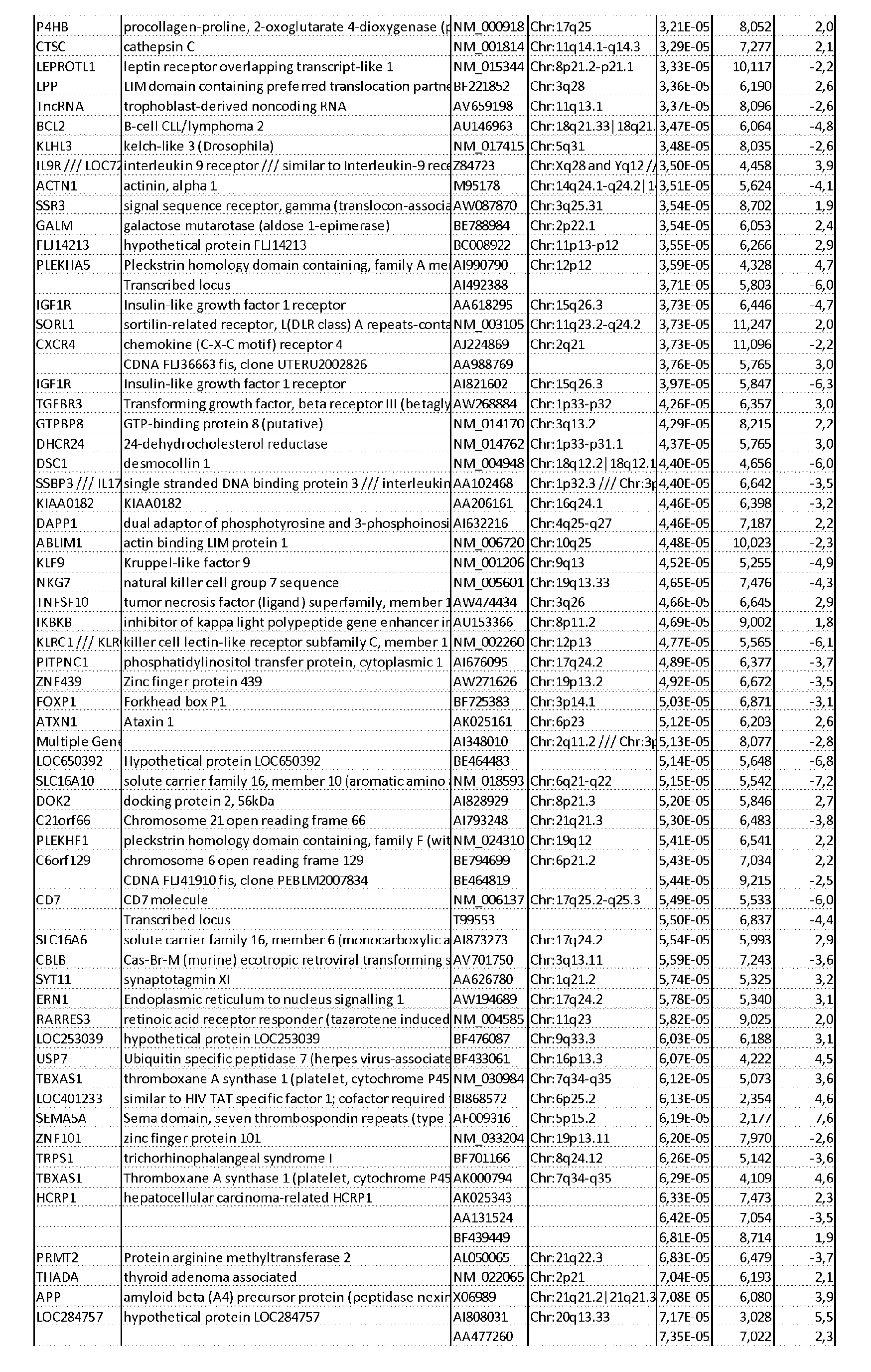

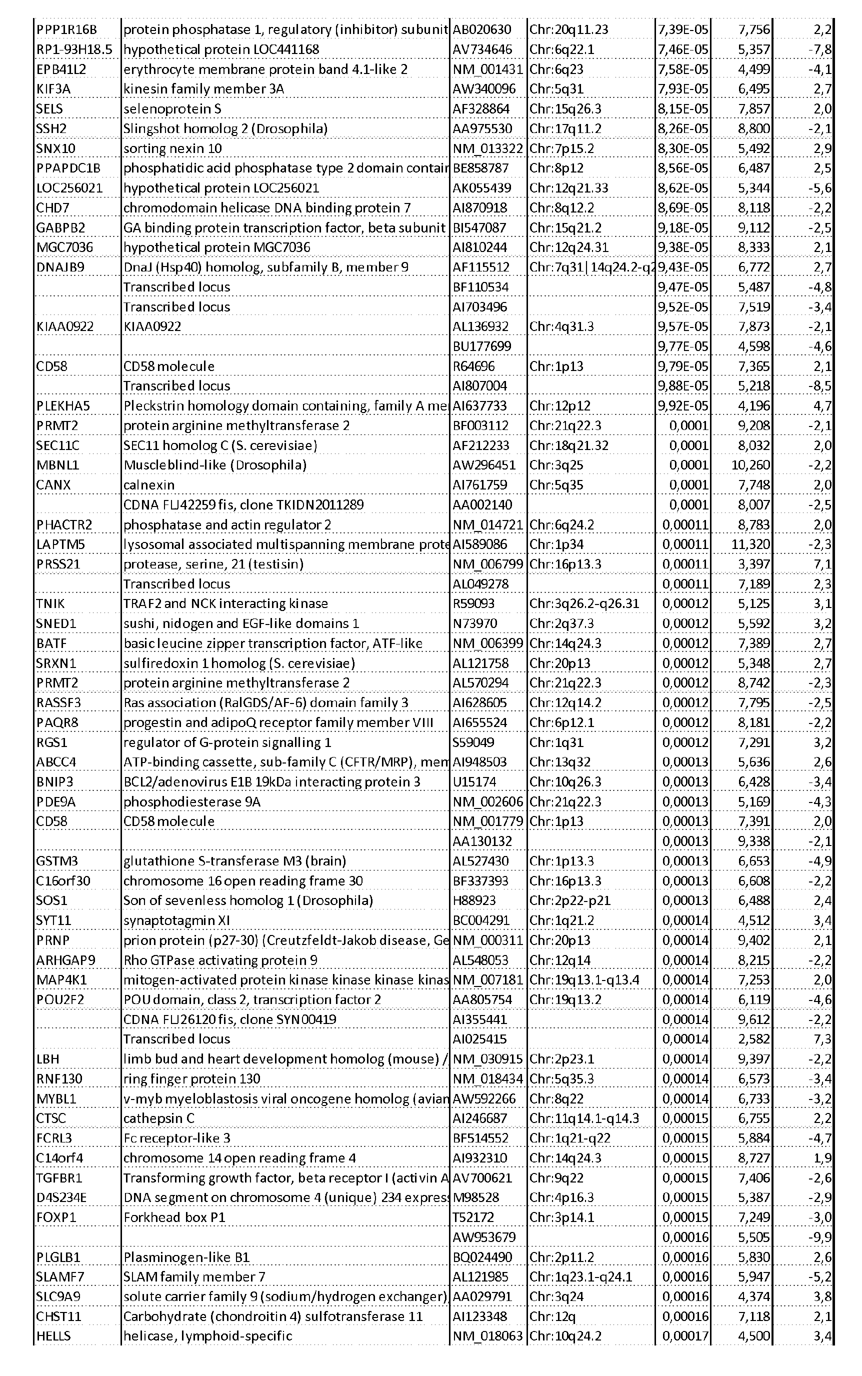

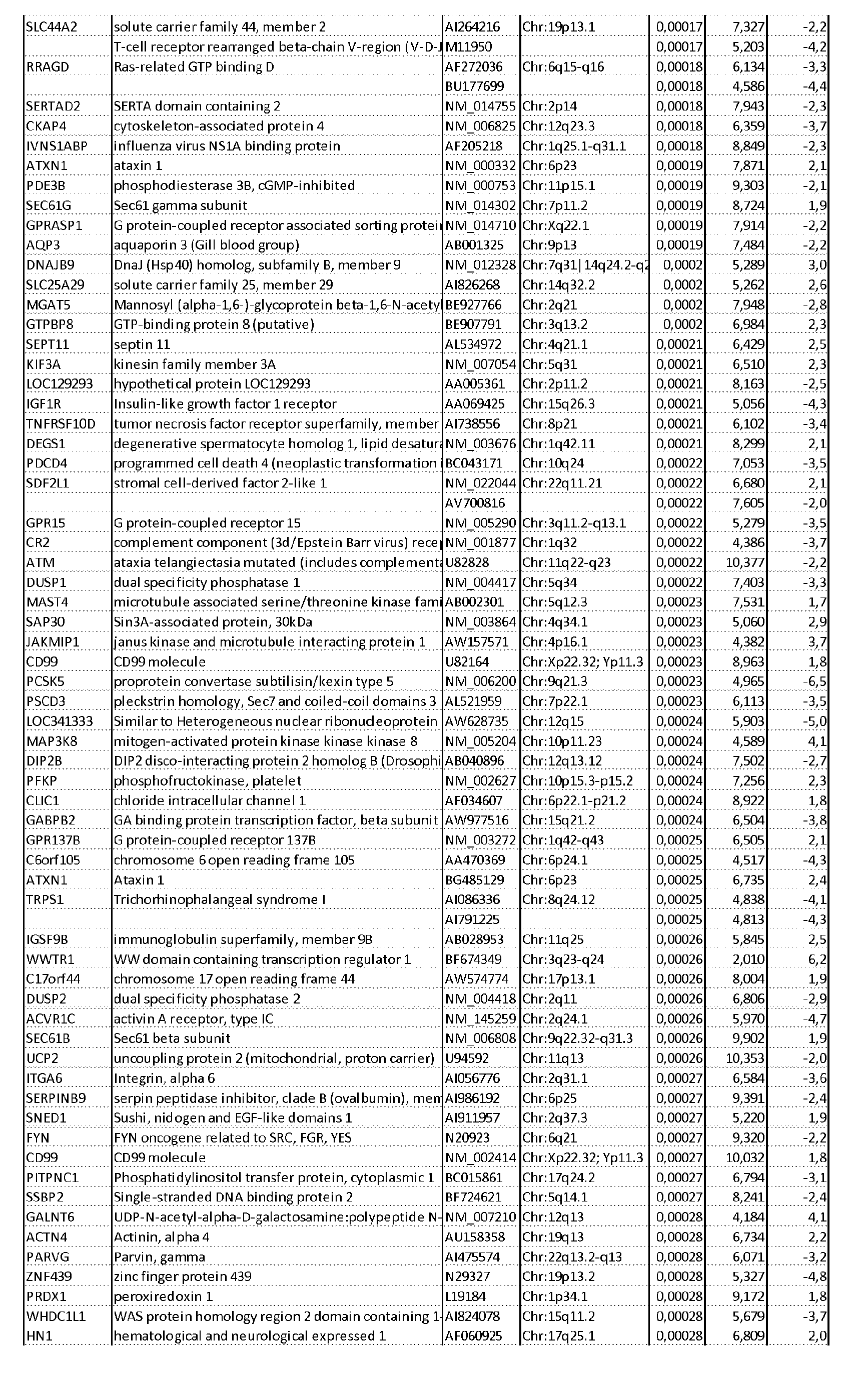

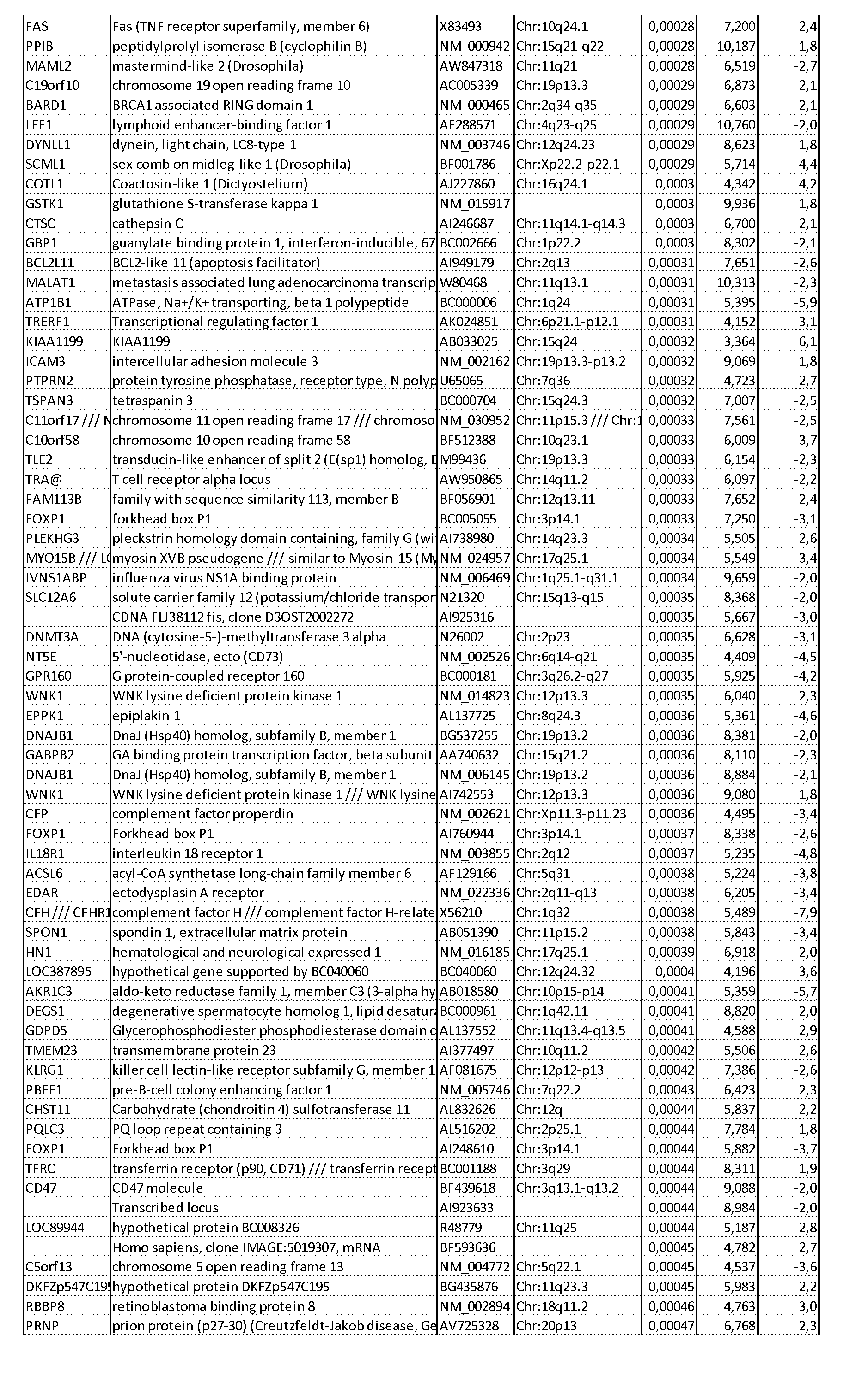

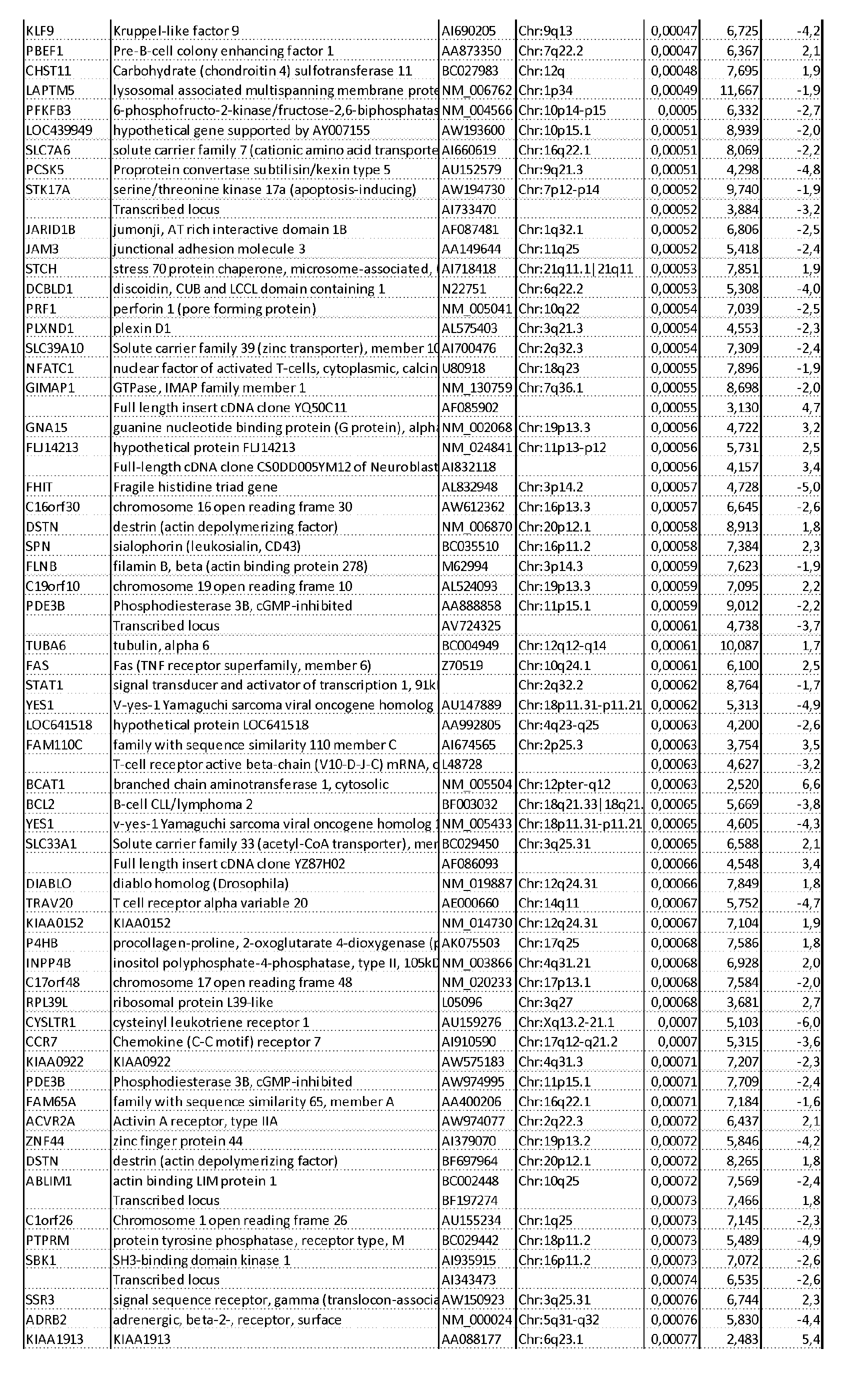

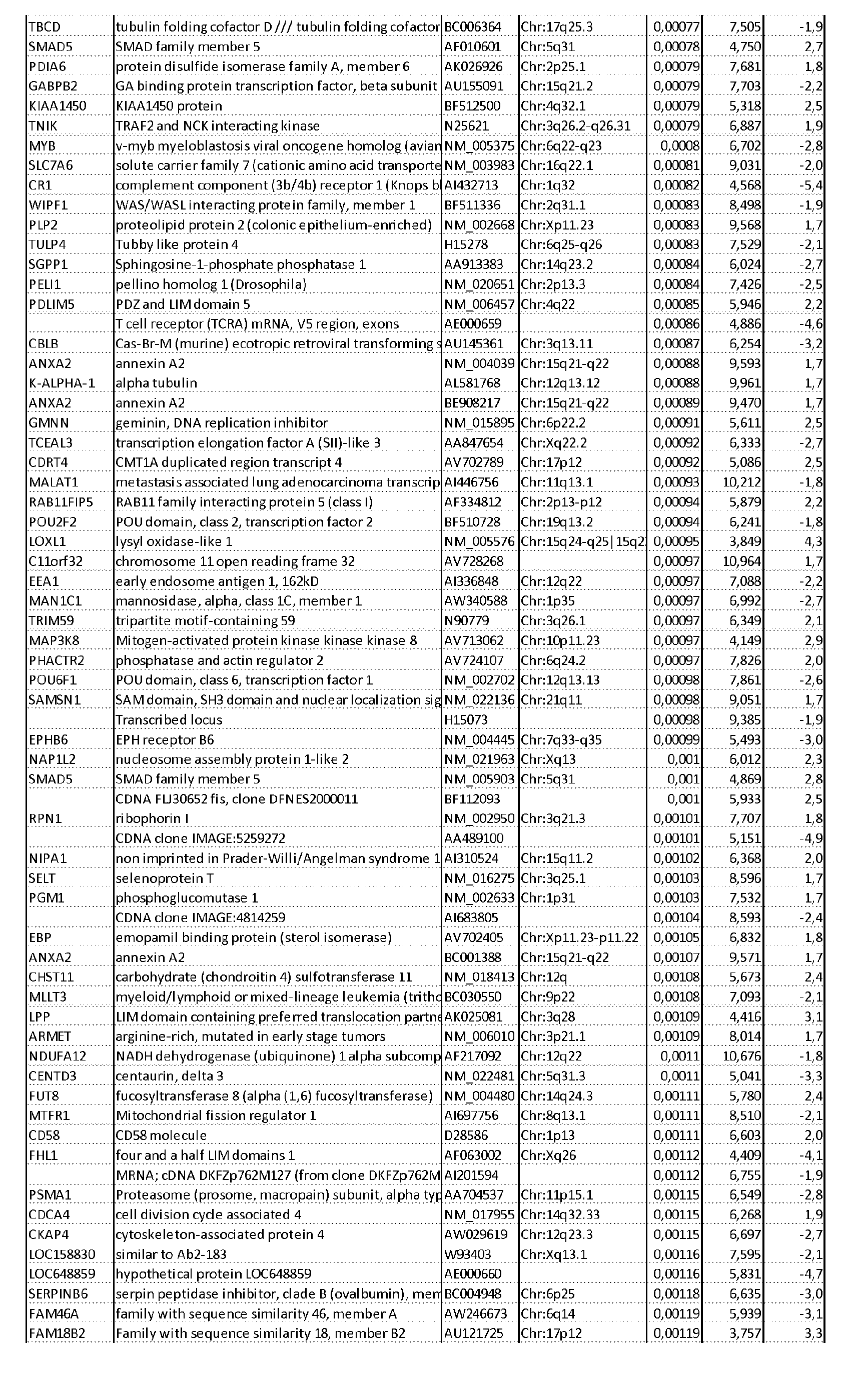

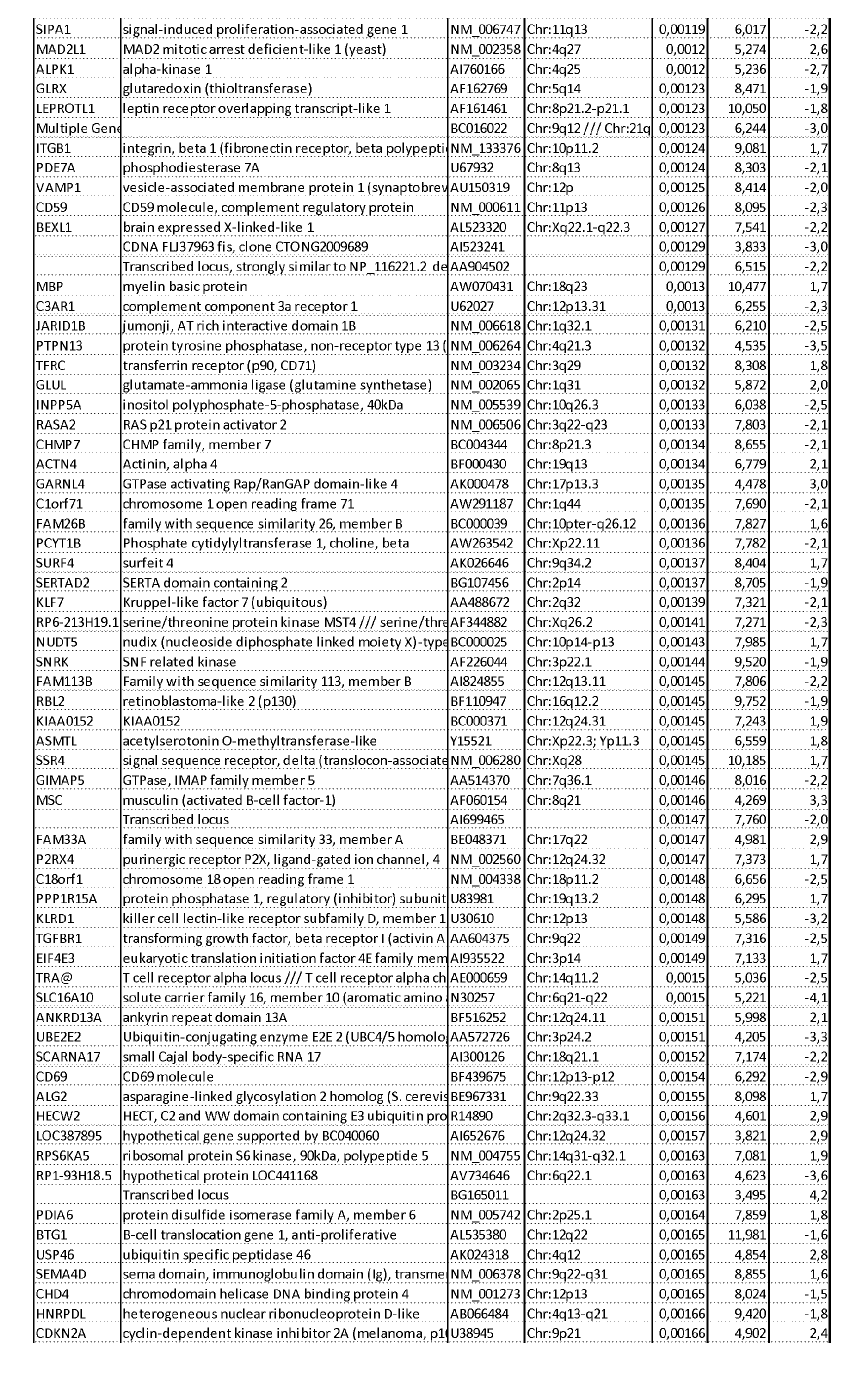

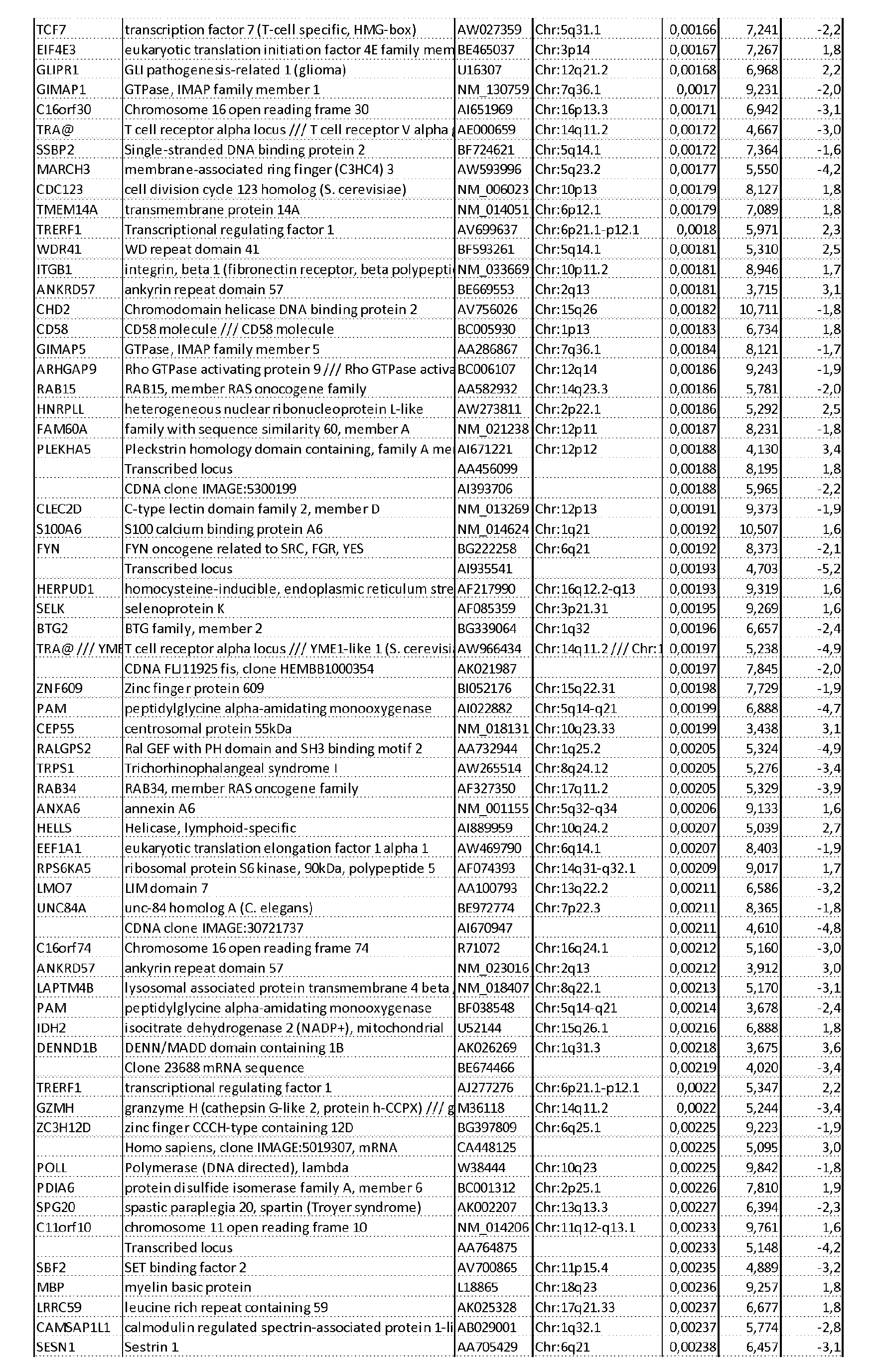

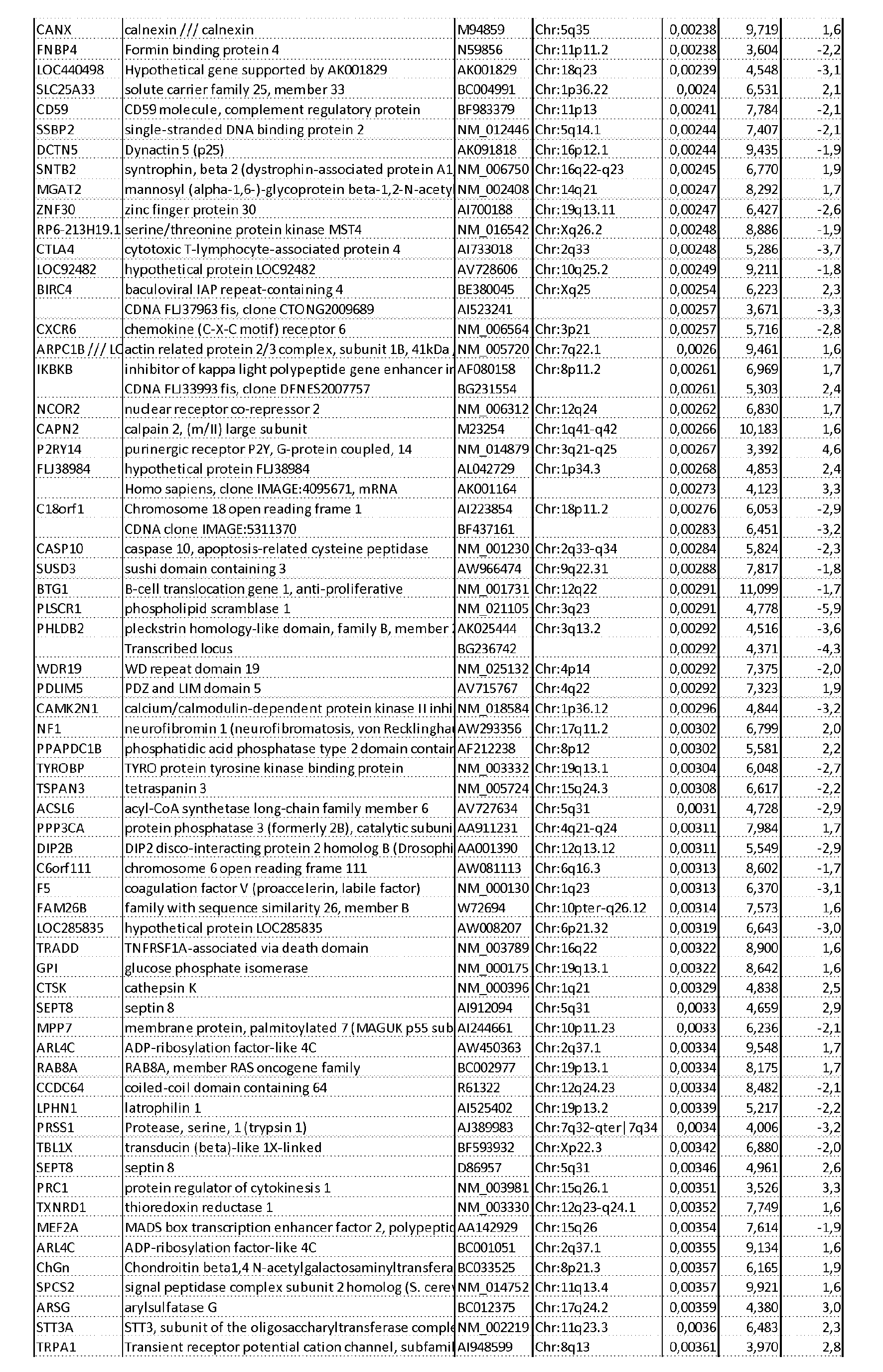

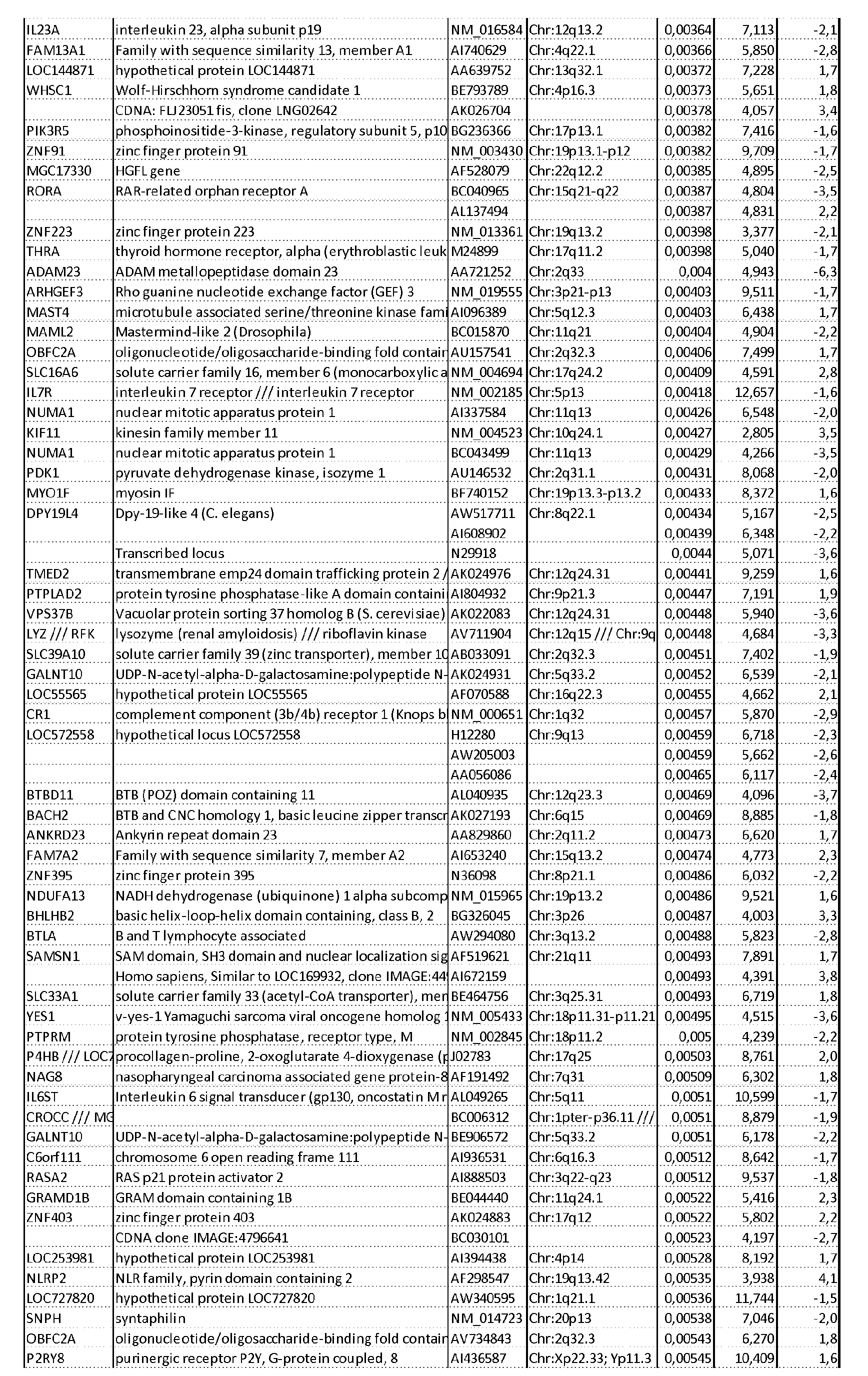

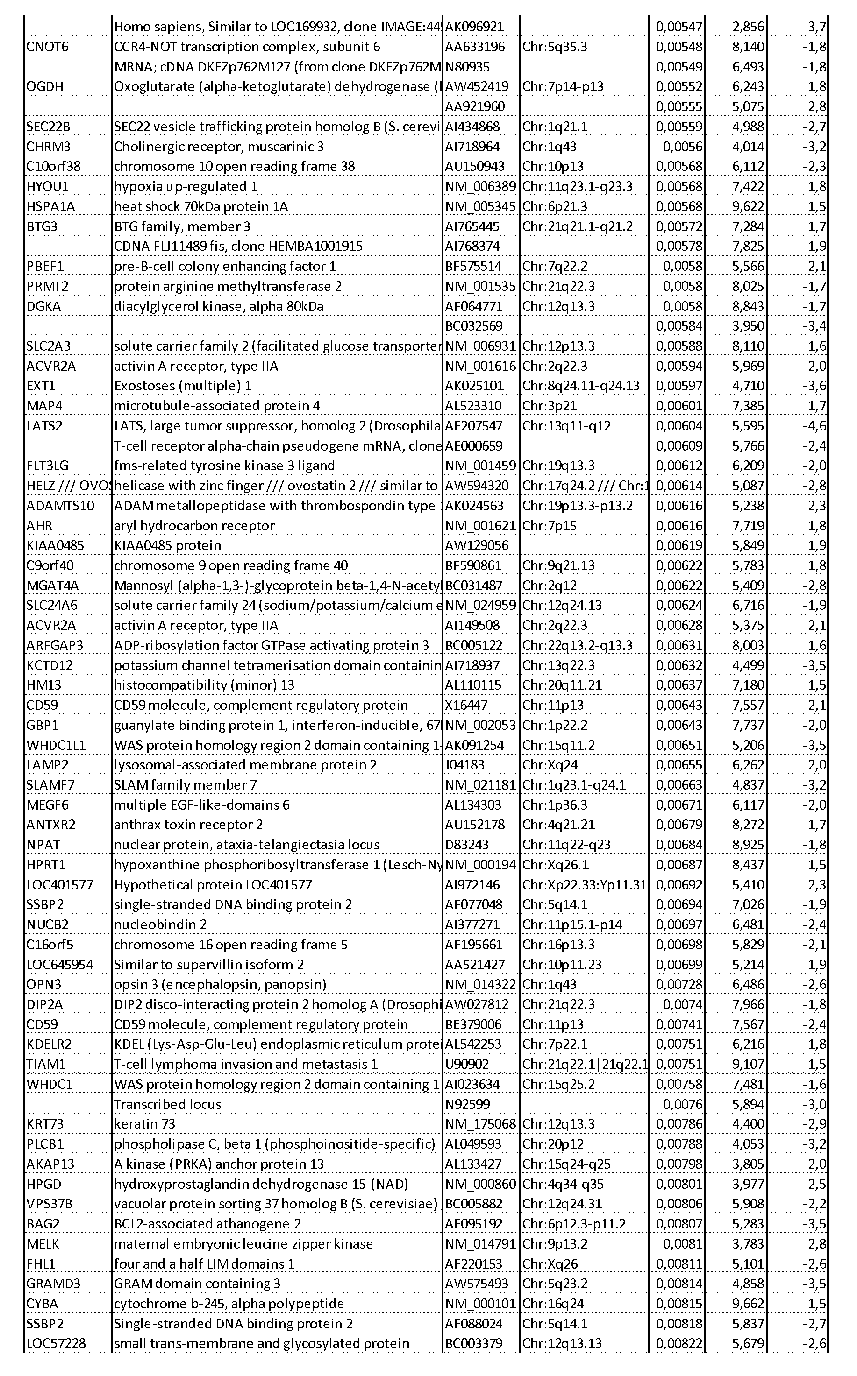

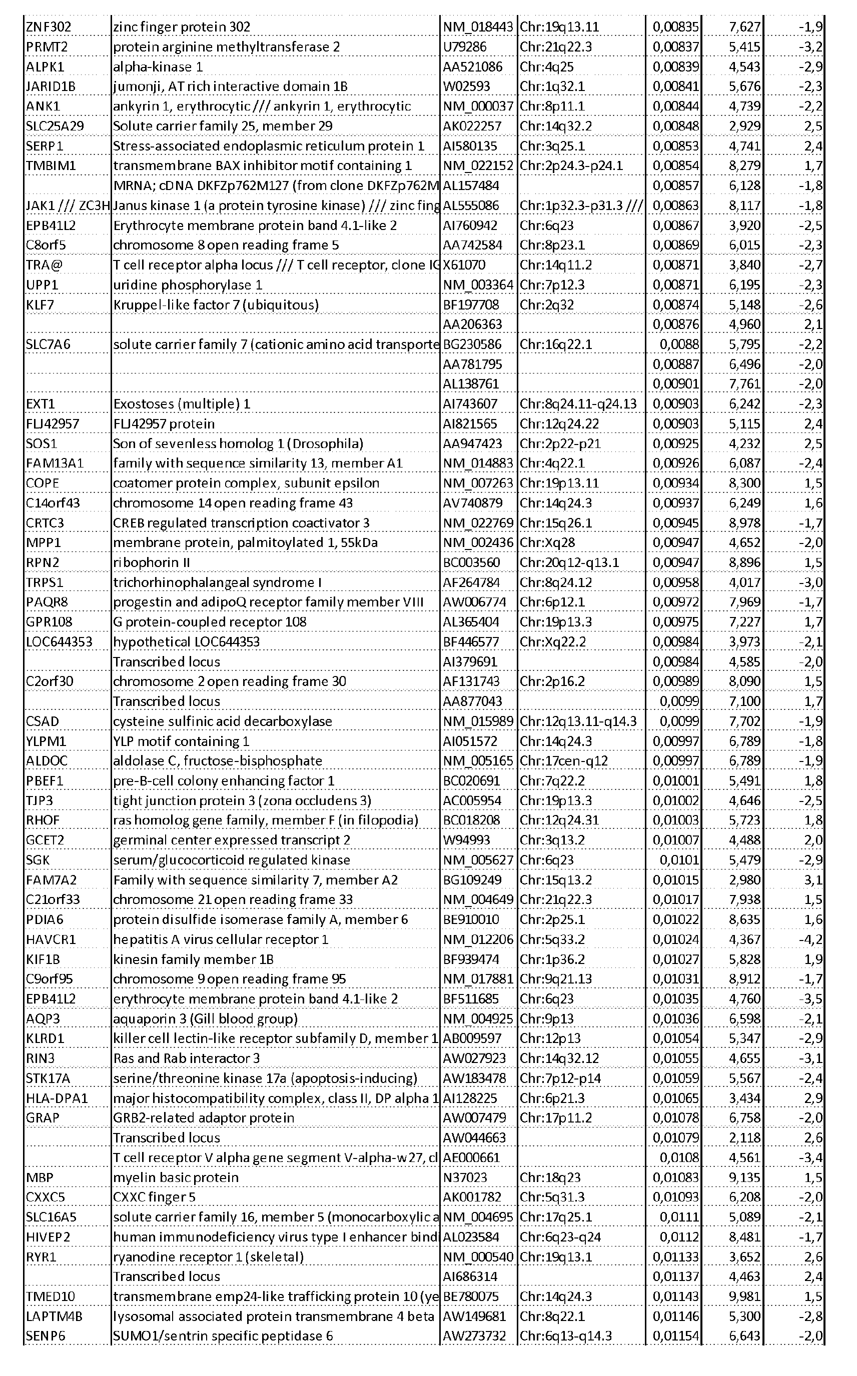

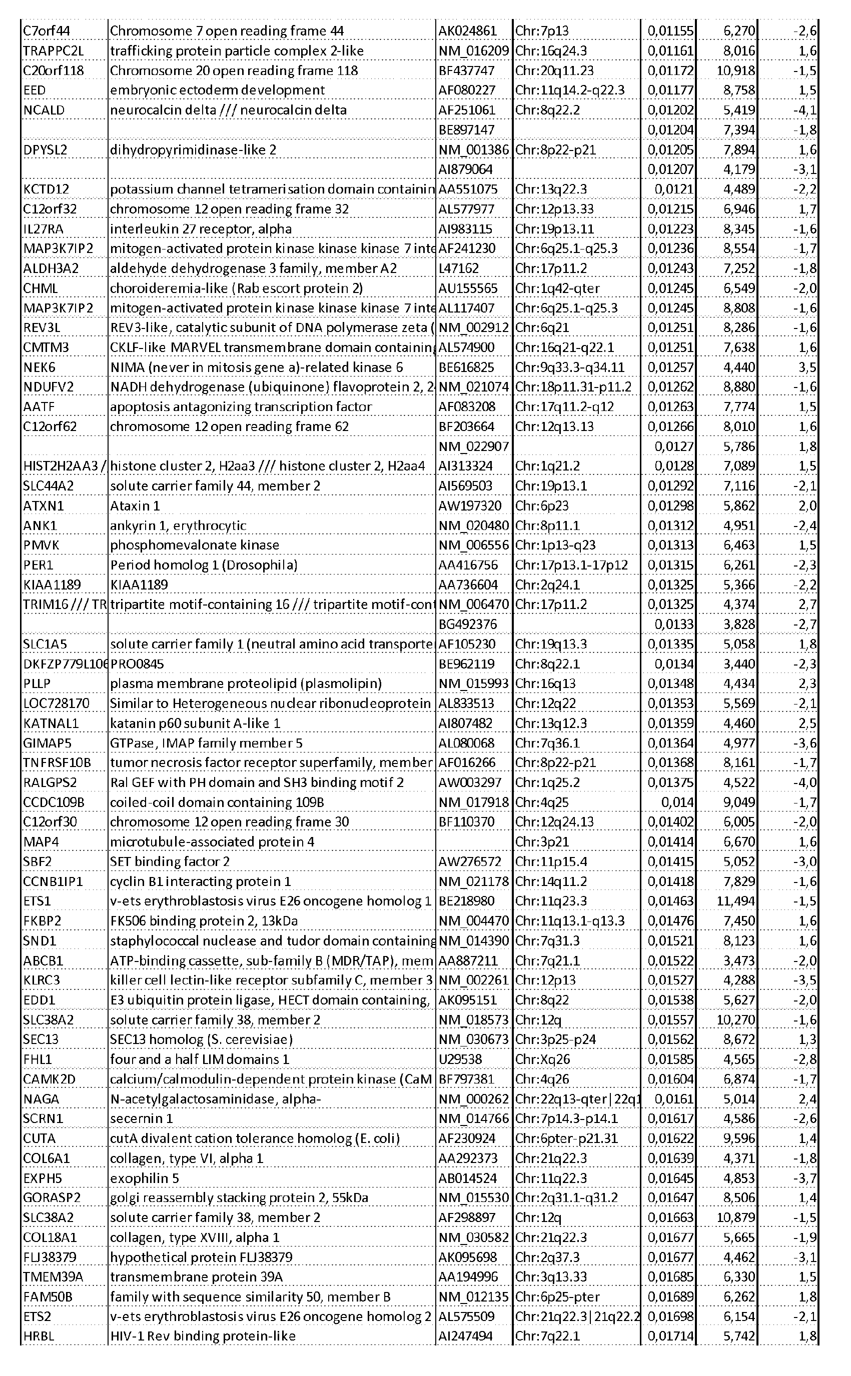

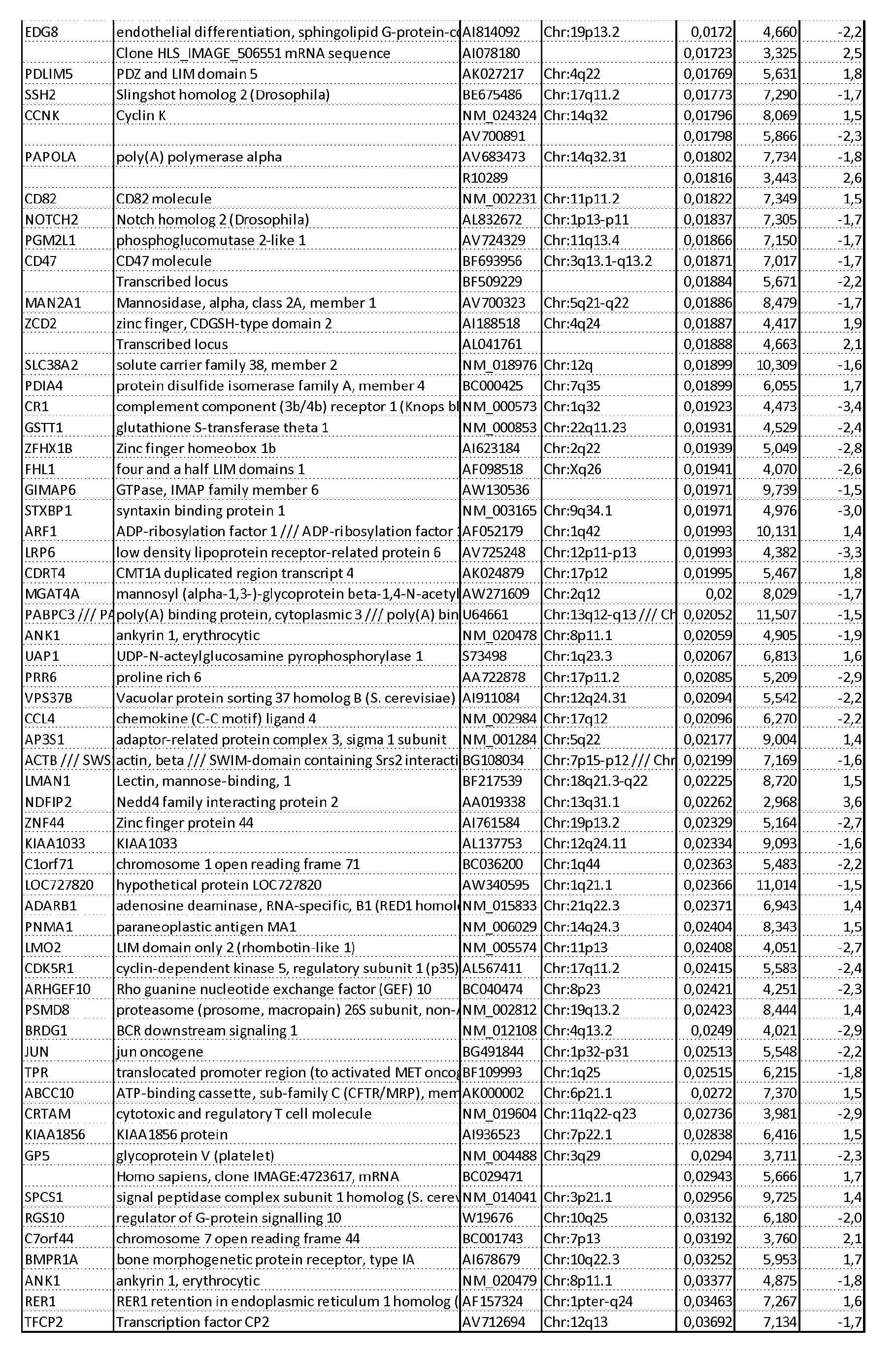

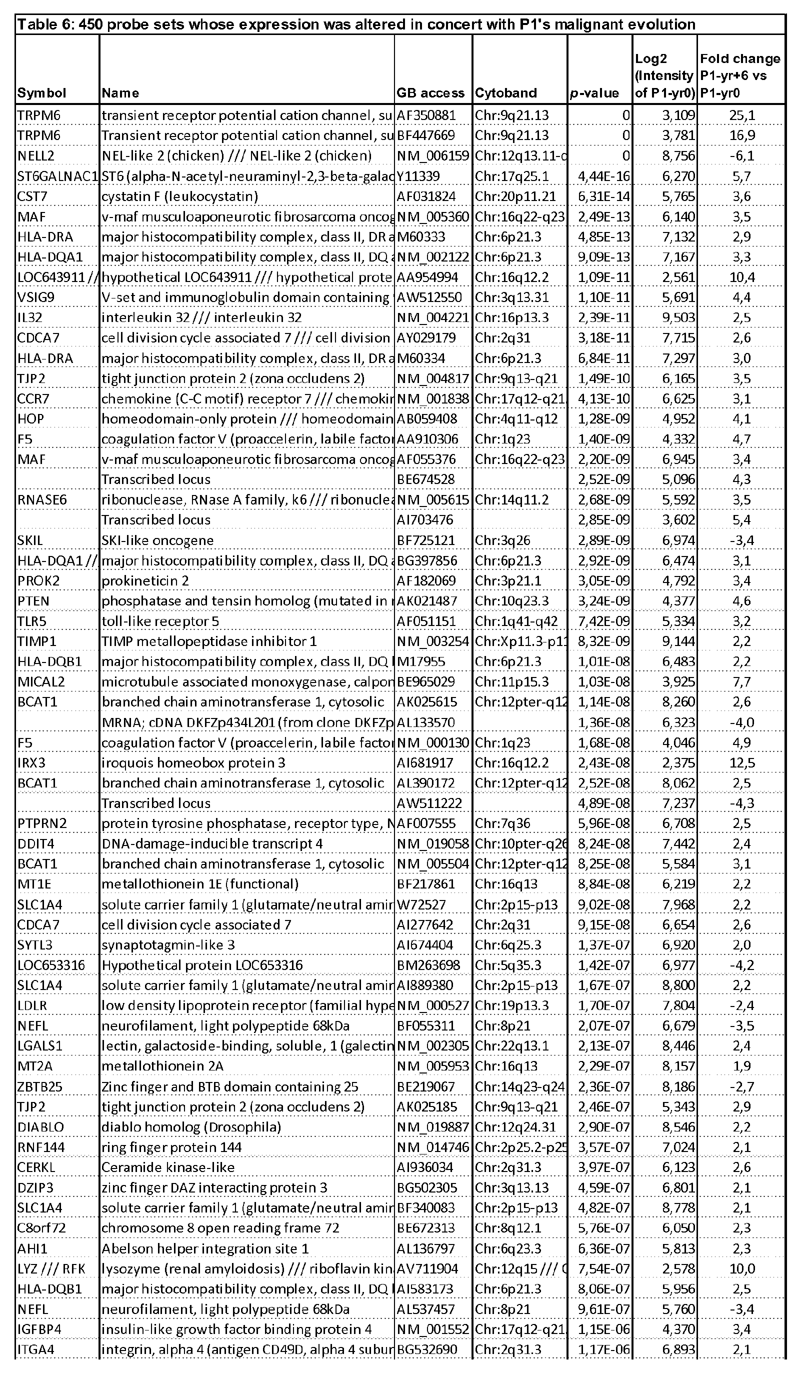

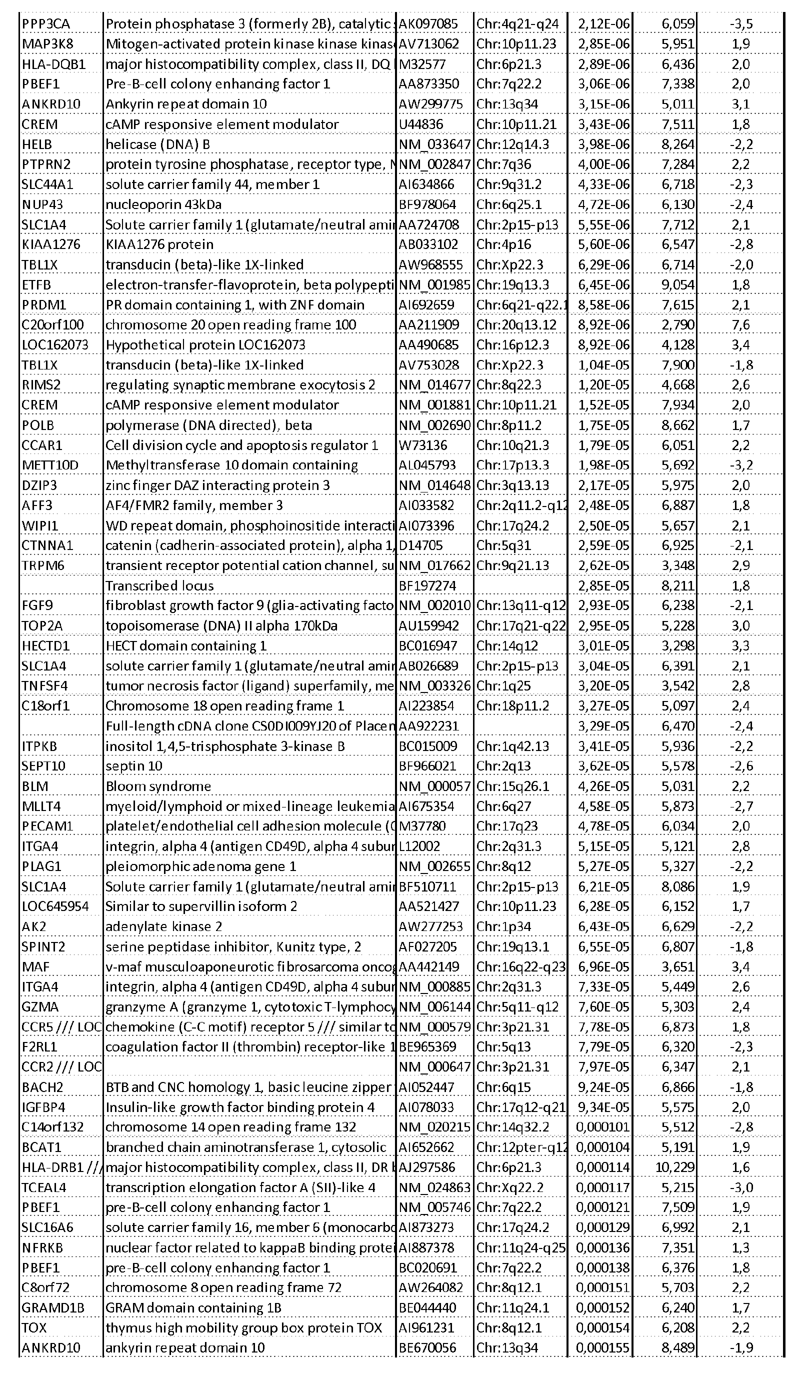

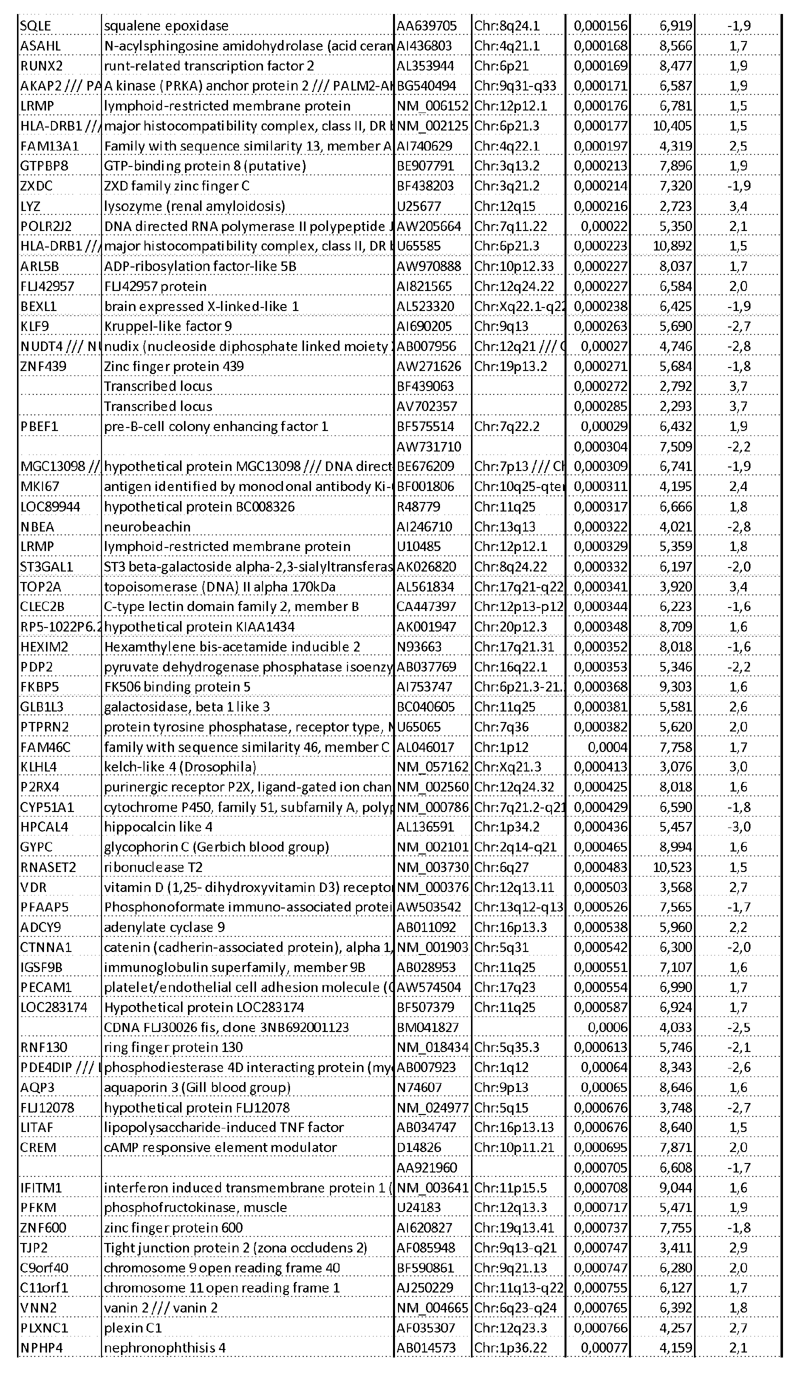

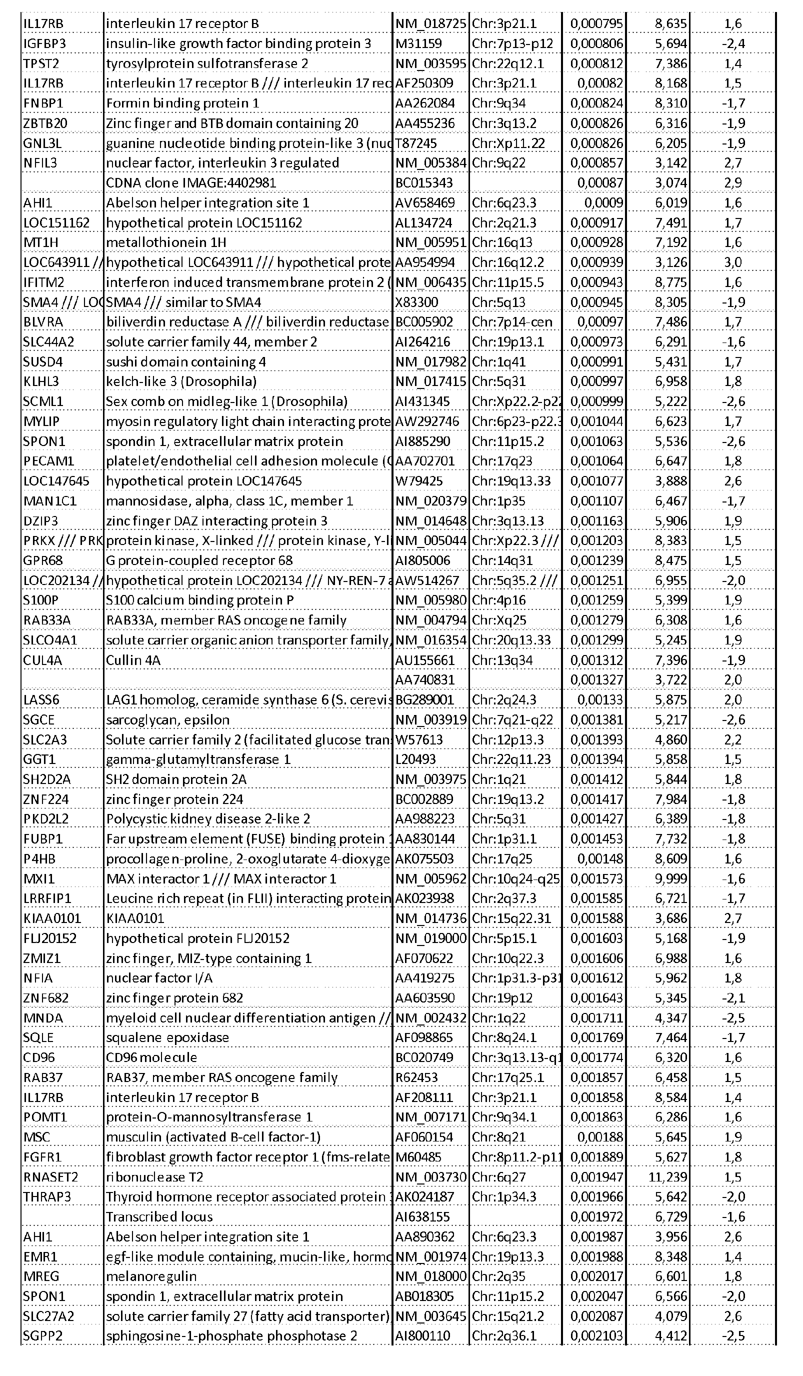

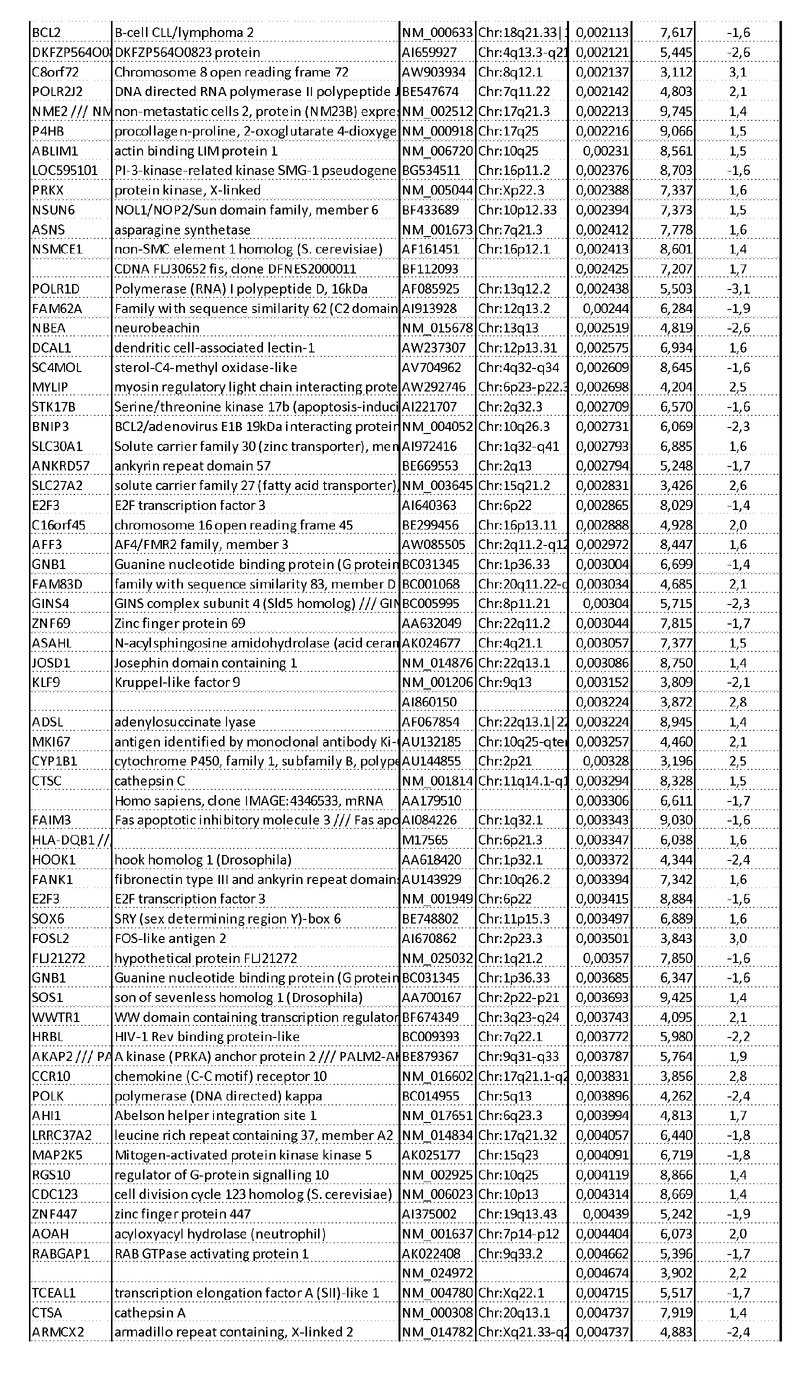

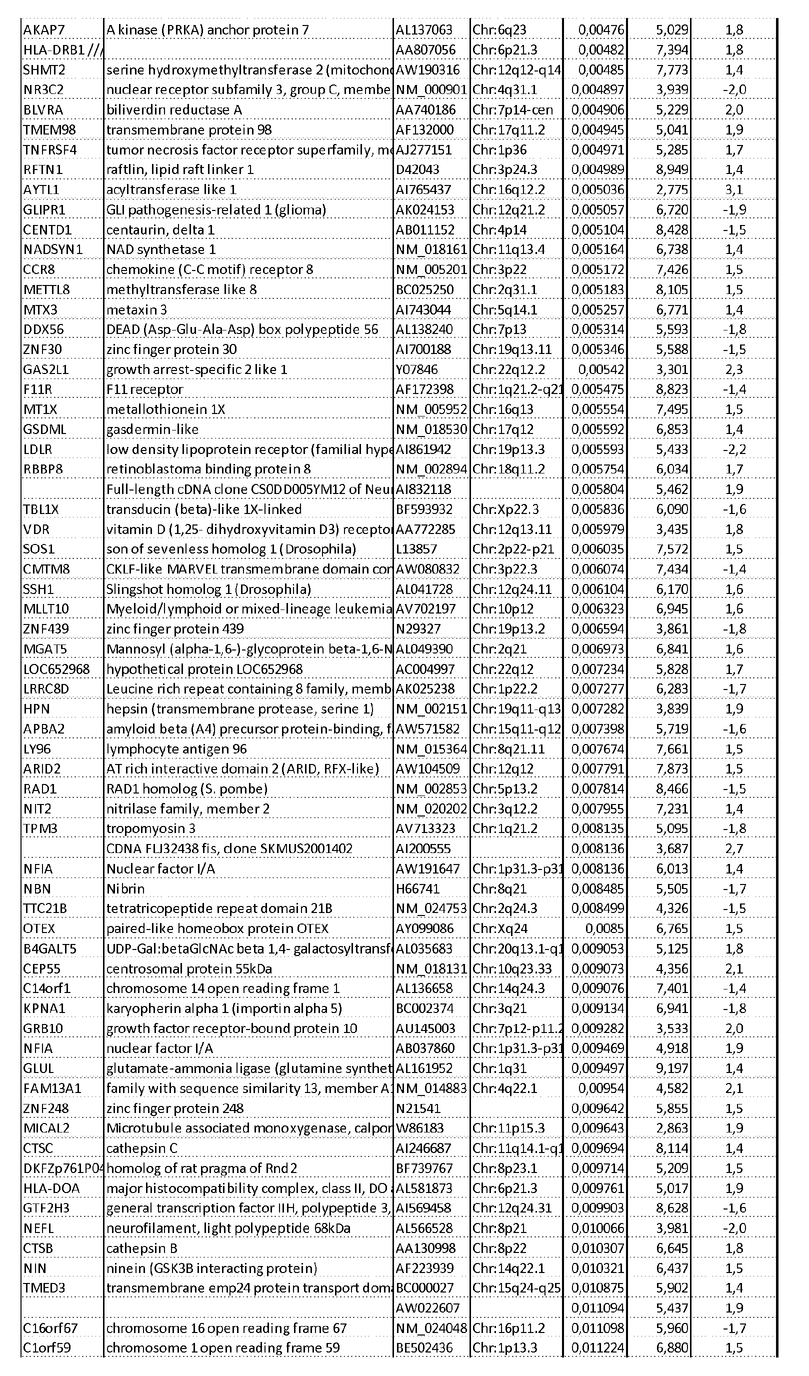

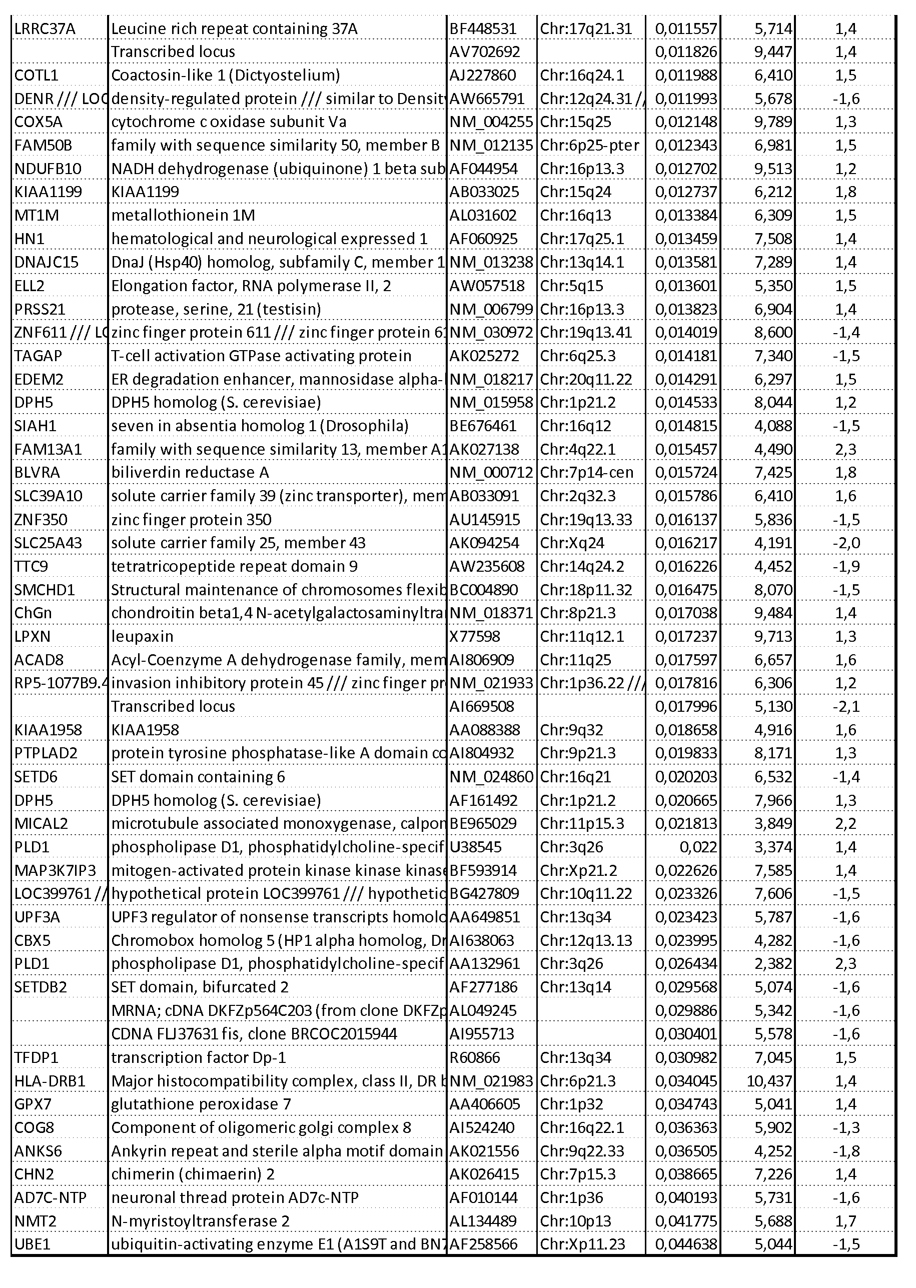

### Gene expression changes in CD3⁻CD4⁺ T-cells from L-HES patients compared with CD3⁺CD4⁺ T-cells from controls

Immunophenotype: Based on the microarray data, a comprehensive immunophenotype of the CD3⁻CD4⁺ cells was obtained. A reduced or lost CD3 (CD3γ,CD3ξ), CD7, CD27 (TNFRSF7) and CD69 mRNA transcripts and increased CD5, CD95 (FAS) and HLA class II antigen mRNA transcripts was observed.

The inventors have determined whether altered mRNA expression corresponded to increased surface protein expression for numerous previously unexplored immunophenotypic markers using flow cytometry (Table 2) and an enlarged patient group (P1-P7; Table 1). Some gene expression changes can be attributed to the clonal Th2 nature of the patient's CD3⁻CD4⁺ T-cells compared with the heterogeneous CD3⁺CD4⁺ T-cell population from controls, particularly in relation to polarization and activation status. These include upregulation of the established Th2 genes IL-4R, CCR3, CCR8, GATA3, CRTH2 (CD294, GPR44) and IL-17RB and downregulation of the Th1 genes BTLA, CCL5 (RANTES), IL-18R (IL18R1 and IL18RAP), NOTCH2, JUN, SLAMF7 (CD319) and integrin α6 (ITGA6) in the abnormal T-cells. Although a previous study (de Lavareille A et al. (2001)) did not detect surface CCR3 on CD3⁻CD4⁺ T-cells from P1&P2, this study found modest increases in CCR3 mRNA in P1&P2 and moderate increases in P3 compared with its absence in the controls, which were confirmed for protein by flow cytometry.

Increased expression of IL-17RB (IL-25/IL-17E receptor) was detected not only in all patients CD3⁻CD4⁺ T-cells during chronic L-HES but also further increased on P1's T-lymphoma cells (Table 7) and after CD2/CD29 co-stimulation of P1-P3's CD3⁻CD4⁺ T-cells (Table 8). Flow cytometry detected a significantly higher proportion of P3's CD3⁻CD4⁺ CD45RO⁺ T-cells expressing membrane IL-17RB compared to CD3⁺CD4⁺CD45RO⁺ or CD45RO⁻ T-cells from controls (Figure 1A). P3's CD3⁻CD4⁺ T-cells cultured with rhIL-25 induced a dose response increase in the production of IL-5 and IL-13 but not IFNγ and enhanced their proliferation in response to phorbol ester/anti-CD28 (Figure 1B). This might reflect the functional role of IL-25 in inflammatory responses where eosinophil infiltration (IL-25 producers) and Th2 memory cells (IL-25 responders) have important activities.

mRNA expression for the membrane complement regulatory proteins CR1 (CD35), CR2 (CD21), CD55 (DAF) and CD59 were decreased in the patient's CD3⁻CD4⁺ T-cells. Decreases in CR1, CD55 and CD59 expression have been associated with various autoimmune and/or inflammatory diseases in humans, and studies in CD55 or CD59 deficient mice indicate that these receptors play a regulatory role in T-cell activation and affect cytokine expression.

Decreased expression of other genes involved in negatively regulating T-cell responses were also detected in the abnormal cells including CTLA4 (CD152) and IL27RA, which have been shown in mice to play critical roles in Th2 cell homeostasis. Additional immune genes that decreased in the CD3⁻CD4⁺ T-cells included CCL4, CCR6, CCR7, CXCR4, CXCR6, CD47, CLEC2B, CLEC2D, CRTAM, CYSLTR1, FLT3LG, IGF1R (CD221), IL6ST, IL7R, IL23A, ITGA4 (CD49D), granzyme (GZMA, GZMH, GZMK), and SLAMF1 (CD150). Because many of these receptors play a role in modulating T-cell activities their downmodulation is potentially an important prerequisite, along with loss of TCR/CD3 surface expression, for persistence and expansion of the CD3⁻CD4⁺ T-cell clone.

Increases in a number of immunoregulatory receptors were also detected, including ICAM3 (CD50), LFA3 (CD58), CD82, SLAMF5 (CD84), DCAL1 (CLECL1), CD71 (TFRC), CD99, CD200R1, IL9R, and ITGB1 gene expression and perhaps provide the more pertinent characterization of the CD3⁻CD4⁺ T-cells. SLAMF5 functions as an inhibitory receptor for the high affinity IgE receptor. DCAL1 is a type II transmembrane, C-type lectin-like protein expressed on dendritic cells and B cells (no previous reports of T-cell expression) whose interaction with T-cells has been shown to enhance their IL-4 production. CD200R1 is an inhibitory receptor that regulates the activation threshold of inflammatory immune responses and thus could also affect CD3⁻CD4⁺ T-cell activation.

CD71 upregulation on activated T-cells and in human malignancies has been extensively described whereas IL-9 receptor upregulation on the CD3⁻CD4⁺ T-cells from L-HES patients is a novel observation. IL-9R expression has been shown to increase in mice over-expressing IL-9 that develop thymomas and was detected in some Hodgkin lymphomas; however, the inventors did not observe further upregulation on P1-yr+6 commensurate with T-lymphoma.

**Table 7: Gene expression change in CD3-CD4+ T cells from chronic L-HES patients relative to controls and in association with P1's evolution to T lymphoma**

| **Symbol** | **Name** | **Probe set** | **P1-P3 vs C^{a,b}** | **P1 yr+6 vs P1-yr0^{b,c}** |
|---|---|---|---|---|
| **ABLIM1** | actin binding LIM protein 1 | 200965_s_at | -2,26 | 1,48 |
| | | 210461_s_at | -2,44 | |
| **ANKRD57** | ankyrin repeat domain 57 | 227034_at | 3,13 | -1,69 |
| | | 219496_at | 2,96 | |
| **APBA2** | amyloid beta (A4) precursor protein-binding, family A, | 209870_s_at | -3,81 | -1,56 |
| | member 2 (X11-like) | 209871_s_at | -12,51 | |
| **AQP3** | aquaporin 3 (Gill blood group) | 39248_at | -3,07 | 1,56 |
| | | 39249_at | -2,16 | |
| | | 203747_at | -2,14 | |
| **BACH2** | BTB and CNC homology 1, | 236796_at | -6,70 | -1,78 |
| | basic leucine zipper transcription factor 2 | 221234_s_at | -5,59 | |
| | | 1556451_at | -4,38 | |
| | | 236307_at | -4,53 | |
| | | 227173_s_at | -4,86 | |
| | | 215907_at | -1,77 | |
| **BCAT1** | branched chain aminotransferase 1, cytosolic | 225285_at | 26,07 | 2,58 |
| | | 226517_at | 27,21 | 2,52 |
| | | 214452_at | 6,56 | 3,11 |
| | | 214390_s_at | | 1,94 |
| **BCL2** | B-cell CLL/lymphoma 2 | 232210_at | -5,23 | |
| | | 232614_at | -4,83 | |
| | | 244035_at | -3,84 | |
| | | 203685_at | | -1,64 |
| **BEXL1** | brain expressed X-linked-like 1 | 215440_s_at | -2,19 | -1,90 |
| **BNIP3** | BCL2/adenovirus E1B 19kDa interacting protein 3 | 201849_at | -4,24 | -2,27 |
| | | 201848_s_at | -3,40 | |
| **C18orf1** | chromosome 18 open reading frame 1 | 242551_at | -2,90 | 2,38 |
| | | 207996_s_at | -2,47 | |
| **C9orf40** | chromosome 9 open reading frame 40 | 222781_s_at | 1,81 | 2,01 |
| **CCR7** | chemokine (C-C motif) receptor 7 | 206337_at | -15,39 | 3,13 |
| | | 243107_at | -3,58 | |
| **CCR8** | chemokine (C-C motif) receptor 8 | 208059_at | 11,33 | 1,50 |
| **CDCA7** | cell division cycle associated 7 | 230060_at | 5,24 | 2,63 |
| | | 224428_s_at | 6,32 | 2,61 |
| **CEP55** | centrosomal protein 55kDa | 218542_at | 3,07 | 2,08 |
| **ChGn** | chondroitin β1,4 N-acetylgalactosaminyltransferase | 1569387_at | 1,93 | |
| | | 219049_at | | 1,36 |
| **CLEC2B** | C-type lectin domain family 2, member B | 209732_at | -3,88 | |
| | | 1556209_at | | -1,59 |
| **COTL1** | coactosin-like 1 (Dictyostelium) | 1556346_at | 4,25 | 1,51 |
| | | 224583_at | 4,28 | |
| | | 221059_s_at | 3,39 | |
| **CST7** | cystatin F (leukocystatin) | 210140_at | -4,21 | 3,61 |
| **CTSC** | cathepsin C | 201487_at | 2,09 | 1,45 |
| | | 225647_s_at | 2,17 | 1,43 |
| | | 225646_at | 2,13 | |
| **DCAL1** | CLECL1, dendritic cell-associated lectin-1 | 244413_at | 15,14 | 1,56 |
| **DIABLO** | diablo homolog (Drosophila) | 219350_s_at | 1,79 | 2,18 |
| **DKFZp761P0423** | homolog of rat pragma of Rnd2 | 235085_at | -6,77 | 1,49 |
| | | 240690_at | -9,70 | |
| **EMR1** | egf-like module containing, mucin-like, 1 | 207111_at | 4,35 | 1,36 |
| **ETFB** | electron-transfer-flavoprotein, beta polypeptide | 202942_at | 2,58 | 1,76 |
| **F5** | coagulation factor V (proaccelerin, labile factor) | 204713_s_at | -3,53 | 4,70 |
| | | 204714_s_at | -3,06 | 4,90 |
| **FAIM3** | Fas apoptotic inhibitory molecule 3 | 221601_s_at | -4,61 | -1,62 |
| | | 221602_s_at | -5,13 | |
| **FAM13A1** | family with sequence similarity 13, member A1 | 232628_at | -2,75 | 2,53 |
| | | 202973_x_at | -2,43 | 2,10 |
| | | 217047_s_at | | 2,29 |
| **FAM50B** | family with sequence similarity 50, member B | 205775_at | 1,82 | 1,53 |
| **FANK1** | fibronectin type III and ankyrin repeat domains 1 | 232968_at | 9,90 | 1,58 |
| **FGF9** | fibroblast growth factor 9 (glia-activating factor) | 206404_at | -3,48 | -2,10 |
| **FLJ20152** | FLJ20152 | | | |
| | hypothetical protein | 218532_s_at 218510_x_at | -9,93 -6,90 | -1,91 |
| **FLJ21272** | hypothetical protein FLJ21272 | 220467_at | -3,35 | -1,57 |
| **FLJ42957** | FLJ42957 protein | 237591_at | 2,40 | 2,04 |
| **GLIPR1** | GLI pathogenesis-related 1 (glioma) | 204222_s_at | 2,13 | |
| | | 214085_x_at | 2,27 | |
| | | 204221_x_at | 2,24 | |
| | | 233515_at | | -1,89 |
| **GLUL** | glutamate-ammonia ligase (glutamine synthetase) | 215001_s_at | 1,97 | 1,38 |
| | | 200648_s_at | 2,02 | |
| **GPR68** | G protein-coupled receptor 68 | 229055_at | 3,02 | 1,50 |
| **GTPBP8** | GTP-binding protein 8 (putative) | 223486_at | 2,29 | 1,93 |
| | | 221046_s_at | 2,19 | |
| **GZMA** | granzyme A (granzyme 1) | 205488_at | -5,70 | 2,40 |
| **HLA-DQA1/2** | major histocompatibility complex, class II, DQ_{α}1/2 | 212671_s_at | 15,46 | 3,07 |
| **HLA-DQB1** | major histocompatibility complex, class II, DQβ1 | 212998_x_at | 14,90 | 2,49 |
| | | 211656_x_at | 4,23 | 2,02 |
| | | 209823_x_at | 5,17 | 2,17 |
| | | 211654_x_at | 3,54 | 1,60 |
| **HLA-DRA** | major histocompatibility complex, class II, DR_{α} | 208894_at | 3,45 | 3,01 |
| **HLA-DRB1** | major histocompatibility complex, class II, DRβ1 | 215193_x_at | 8,60 | 1,64 |
| | | 208306_x_at | 7,51 | 1,41 |
| | | 209312_x_at | 8,34 | 1,52 |
| | | 204670_x_at | 7,01 | 1,52 |
| **HN1** | hematological and neurological expressed 1 | 222396_at | 2,05 | 1,36 |
| | | 217755_at | 2,00 | |
| **HPCAL4** | hippocalcin like 4 | 219671_at | -4,90 | -3,01 |
| **HRBL** | HIV-1 Rev binding protein-like | 222126_at | 1,77 | |
| | | 1554618_at | | -2,23 |
| **IGSF9B** | immunoglobulin superfamily, member 9B | 215255_at | 2,48 | 1,64 |
| **IL17RB** | interleukin 17 receptor B | 219255_x_at | 22,24 | 1,55 |
| | | 224156_x_at | 22,37 | 1,45 |
| | | 224361_s_at | 16,85 | 1,47 |
| **ITGA4** | integrin, alpha 4 (antigen CD49D, | 244599_at | -6,56 | |
| | alpha 4 subunit of VLA-4 receptor) | 213416_at | | 2,08 |
| | | 205884_at | | 2,55 |
| | | 205885_s_at | | 2,78 |
| **KIAA1199** | KIAA1 199 | 212942_s_at | 6,10 | 1,79 |
| **KLF9** | Kruppel-like factor 9 | 203543_s_at | -4,86 | -2,12 |
| | | 203542_s_at | -4,19 | -2,66 |
| **KLHL3** | kelch-like 3 (Drosophila) | 221221_s_at | -2,64 | 1,77 |
| **LASS6** | LAG1 longevity assurance homolog 6 (S. cerevisiae) | 212442_s_at | -7,49 | 2,01 |
| | | 212446_s_at | -5,80 | |
| **LOC283174** | hypothetical protein LOC283174 | 229734_at | 2,71 | 1,71 |
| **LOC89944** | hypothetical protein BC008326 | 213713_s_at | 2,80 | 1,77 |
| **LYZ /// LILRB1** | lysozyme (renal amyloidosis) /// leukocyte Ig-like | 213975_s_at | -3,26 | 10,01 |
| **LYZ** | receptor, subfamily B, member 1 | 1555745_a_at | | 3,41 |
| **MAN1C1** | mannosidase, alpha, class 1C, member 1 | 218918_at | -3,14 | -1,66 |
| | | 214180_at | -2,75 | |
| **MAP3K8** | mitogen-activated protein kinase kinase kinase 8, | 235421_at | 2,90 | 1,90 |
| | COT, TPL2 | 205027_s_at | 4,10 | |
| **MGAT5** | mannosyl (alpha-1,6-)-glycoprotein e | 241893_at | -2,75 | |
| | beta-1,6-N-acetyl-glucosaminyltransferase | 215528_at | | 1,56 |
| **MSC** | musculin (activated B-cell factor-1) | 209928_s_at | 3,28 | 1,95 |
| **NELL2** | NEL-like 2 (chicken) | 203413_at | -19,57 | -6,10 |
| **P2RX4** | purinergic receptor P2X, ligand-gated ion channel, 4 | 204088_at | 1,75 | 1,64 |
| **P4HB** | procollagen-proline, 2-oxoglutarate 4-dioxygenase | 200656_s_at | 2,05 | 1,46 |
| | (proline 4-hydroxylase), beta polypeptide | 1564494_s_at | 1,81 | 1,59 |
| | | 200654_at | 2,04 | |
| **PBEF1** | pre-B-cell colony enhancing factor 1 | 243296_at | 2,07 | 1,98 |
| | | 1555167_s_at | 1,76 | 1,82 |
| | | 217739_s_at | 2,33 | 1,93 |
| | | 217738_at | 2,08 | 1,88 |
| **PDE4DIP** | phosphodiesterase 4D interacting protein | 236704_at | -4,48 | |
| | (myomegalin) | 212390_at | | -2,59 |
| **PPP3CA** | protein phosphatase 3 (formerly2B), catalytic subunit, | 202457_s_at | 1,70 | |
| | alpha isoform (calcineurin A alpha) | 1562467_at | | -3,53 |
| **PRSS21** | protease, serine, 21 (testisin) | 220051_at | 7,10 | 1,44 |
| **PTPLAD2** | protein tyrosine phosphatase-like A domain 2 | 244050_at | 1,85 | 1,34 |
| **PTPRN2** | protein tyrosine phosphatase, receptor type, | 203029_s_at | 11,85 | 2,20 |
| | N polypeptide 2 | 203030_s_at | 9,14 | 2,49 |
| | | 211534_x_at | 2,68 | 1,99 |
| **RBBP8** | retinoblastoma binding protein 8 | 203344_s_at | 3,02 | 1,73 |
| **RGS10** | regulator of G-protein signalling 10 | 204319_s_at | -2,81 | 1,40 |
| | | 204316_at | -2,02 | |
| **RNF130** | ring finger protein 130 | 217865_at | -3,41 | -2,08 |
| **RUNX2** | runt-related transcription factor 2 | 232231_at | 2,41 | 1,86 |
| **SCML1** | sexcomb on midleg-like 1 (Drosophila) | 235652_at | -11,11 | -2,61 |
| | | 218793_s_at | -12,21 | |
| | | 222747_s_at | -4,39 | |
| **SLC16A6** | solute carrier family 16, member 6 | 230748_at | 2,86 | 2,10 |
| | (monocarboxylic acid transporter 7) | 207038_at | 2,76 | |
| **SLC1A4** | solute carrier family 1, member 4 | 212811 x_at | 6,71 | 2,20 |
| | (glutamate/neutral amino acid transporter) | 209610_s_at | 9,11 | 2,07 |
| | | 212810_s_at | 6,62 | 2,18 |
| | | 244377_at | 5,53 | 2,05 |
| | | 235875_at | 5,03 | 1,89 |
| | | 209611_s_at | | 2,14 |
| **SLC2A3** | solute carrier family 2, member 3 | 202499_s_at | 1,62 | |
| | (facilitated glucose transporter) | 236180_at | | 2,16 |
| **SLC39A10** | solute carrier family 39 (zinc transporter), member 10 | 225295_at | -1,93 | 1,60 |
| | | 226444_at | -2,38 | |
| **SLC44A2** | solute carrier family 44, member 2 | 224609_at | -2,17 | -1,65 |
| | | 225175_s_at | -2,12 | |
| **SOS1** | son of sevenless homolog 1 (Drosophila) | 212780_at | 2,97 | 1,45 |
| | | 229261_at | 3,08 | |
| | | 230337_at | 3,23 | |
| | | 232883_at | 2,37 | |
| | | 242018_at | 2,53 | |
| | | 212777_at | | 1,51 |
| **SPON1** | spondin 1, extracellular matrix protein | 213994_s_at | -7,71 | -2,57 |
| | | 209436_at | -5,41 | -2,03 |
| | | 213993_at | -7,27 | |
| | | 209437_s_at | -3,37 | |
| **TBL1X** | transducin (beta)-like 1X-linked | 213400_s_at | -2,68 | -1,80 |
| | | 201869_s_at | -2,01 | -1,57 |
| | | 201867_s_at | | -2,01 |
| **TCEAL4** | transcription elongation factor A(SII)-like 4 | 202371_at | -6,95 | -3,00 |
| **WWTR1** | WW domain containing transcription regulator 1 | 202133_at | 6,17 | 2,15 |
| **ZNF30** | zinc finger protein 30 | 232014_at | -2,61 | -1,53 |
| **ZNF439** | zinc finger protein 439 | 236562_at | -4,82 | -1,80 |
| | | 237441_at | -3,46 | -1,76 |
| **ZNF447** | zinc finger protein 447 | 218312_s_at | -7,77 | |
| | | 217593_at | | -1,89 |

| | | | | |
|---|---|---|---|---|
| ^{a}: Fold-change comparing the mean expression of duplicate arrays from P1, P2 and P3 with the mean expression from 4 controls; data from Table 5 ^{b}: The last two columns of this table especially highlight several consistently upregulated genes (e.g. BCAT1) and several consistently downregulated genes (e.g. APBA2) ^{c}: Fold-change comparing the mean expression of triplicate arrays from P1-yr0 and P1-yr+6; data from Table 6. | | | | |

The lack of TCR/CD3 expression on the abnormal T-cell surface dictates the potential loss of this important signaling pathway, although the CD3⁻CD4⁺ T-cells do remain responsive to co-stimulatory signals. Some critical TCR/CD3 downstream signals were found decreased in non-stimulated CD3⁻CD4⁺ T-cells, including inhibitory receptors, PI3K-associated or family proteins, tyrosine and MAP3 kinases and activation responsive transcription factors. Interestingly, gene expression analysis of the abnormal T-cells after CD2/CD28 co-stimulation revealed that relatively few of the gene expression changes detected in "quiescent" CD3⁻CD4⁺ T-cells isolated from patient blood involved genes whose expression was affected by activation (Table 8, Table 5). Thus, induction of the Th2 cytokine genes IL5, IL-13 and other immune response genes by co-stimulation *in vitro* apparently induces transient signals that may be elicited by a sustained stimulus present in local immune microenvironments *in vivo*.

**Table 8: Changes in the expression of immune response genes associated with anti-CD2/CD28 activation of the CD3-CD4+ T cells from P1-P3^{a}**

| **Symbol** | **Name** | **Probe set ID** | **Mn fold chg: P1-P3 vs C^{b}** | **Mn fold chg: I P1 yr+6 vs yr0^{c}** | **Mn fold chg: P1 P3 S vs NS^{d}** |
|---|---|---|---|---|---|
| **ATF4** | activating transcription factor 4 | 200779_at | nc^{f} | nc | 1,55 |
| **BCL2** | B-cell CLL/lymphoma 2 | 232210_at | -5,23 | nc | 18,44 |
| | | 203685_at | nc | -1,64 | 8,91 |
| **BCL2L1** | BCL2-like 1 | 212312_at | nc | nc | 1,70 |
| **BCL2L11** | BCL2-like 11 (apoptosis facilitator) | 225606_at | -2,62 | nc | nc |
| **CCL5** | chemokine (C-C motif) ligand 5 (RANTES) | 1405_i_at | -26,17 | nc^{g} | nc |
| **CCR2^{e}** | chemokine (C-C motif) receptor 2 (CD192) | 206978_at | nc | 2,14 | 2,01 |
| **CCR3** | chemokine (C-C motif) receptor 3 (CD193) | 208304_at | 6,79 | nc | nc |
| **CCR5** | chemokine (C-C motif) receptor 5 (CD195) | 206991_s_at | nc | 1,80 | nc |
| **CCR6** | chemokine (C-C motif) receptor 6 (CD196) | 206983_at | -6,40 | nc | nc |
| **CCR7** | chemokine (C-C motif) receptor 7 (CD197) | 206337_at | -15, 39 | 3,13 | nc |
| | | 243107_at | -3,58 | nc | nc |
| **CCR8** | chemokine (C-C motif) receptor 8 (CD198) | 208059_at | 11,33 | 1,50 | nc |
| **CCR10** | chemokine (C-C motif) receptor 10 | 220565_at | nc | 2,81 | nc |
| **CD27** | CD27 molecule | 206150_at | -42, 39 | nc | nc |
| **CD3G** | CD3g molecule, gamma (TCR/CD3) | 206804_at | -2,47 | nc | nc |
| **CD47** | CD47 molecule | 227259_at | -1,99 | nc | nc |
| **CD5** | CD5 molecule | 230489_at | 2,78 | nc | nc |
| **CD53** | CD53 molecule | 203416_at | nc | nc | 1,38 |
| **CD55** | CD55 molecule, DAF for complement | 243395_at | -3,93 | nc | nc |
| **CD58** | CD58 molecule | 243931_at | 2,10 | nc | nc |
| **CD59** | CD59 molecule, complement regulatory | 212463_at | -2,43 | nc | nc |
| **CD69** | CD69 molecule | 209795_at | nc | nc | 2,36 |
| | | 237009_at | -2,88 | nc | nc |
| **CD7** | CD7 molecule | 214551_s_at | -6,03 | nc | nc |
| **CD74** | CD74 molecule, MHC, class II invariant | 209619_at | nc | nc | 2,73 |
| **CD80** | CD80 molecule | 1554519_at | nc | nc | 3,33 |
| **CD82** | CD82 molecule | 203904_x_at | 1,53 | nc | nc |
| **CD84** | CD84 molecule | 205988_at | 2,11 | nc | nc |
| **CD96** | CD96 molecule | 1555120_at | nc | 1,62 | nc |
| **CD99** | CD99 molecule | 201028_s_at | 1,78 | nc | nc |
| **CD200R1** | CD200 receptor 1 | 1552875_a_at | 3,69 | nc | nc |
| **CD247** | CD247 molecule | 210031_at | -2,56 | nc | nc |
| **CIITA** | class II, MHC, transactivator | 205101_at | nc | nc | 2,87 |
| **CISH** | cytokine inducible SH2-containing protein | 223961_s_at | nc | nc | 11,83 |
| **DUSP1** | dual specificity phosphatase 1 | 201041_s_at | -3,25 | nc | nc |
| **DUSP2** | dual specificity phosphatase 2 | 204794_at | -2,86 | nc | nc |
| **DUSP4** | dual specificity phosphatase 4 | 204014_at | 6,51 | nc | nc |
| **DUSP5** | dual specificity phosphatase 5 | 209457_at | nc | nc | 5,76 |
| **DUSP6** | dual specificity phosphatase 6 | 208891_at | nc | nc | 6,93 |
| **DUSP16** | dual specificity phosphatase 16 | 224336_s_at | nc | nc | -2,14 |
| **EGR1** | early growth response 1 | 227404_s_at | nc | nc | 7,82 |
| **EGR2** | early growth response 2 | 205249_at | nc | nc | 8,02 |
| **EGR3** | early growth response 3 | 206115_at | nc | nc | 6,54 |
| **EPHA4** | EPH receptor A4 | 206114_at | nc | nc | -1,75 |
| **EPHB6** | EPH receptor B6 | 204718_at | -2,98 | nc | nc |
| **FAIM3^{e}** | Fas apoptotic inhibitory molecule 3 | 221601_s_at | -4,61 | -1,62 | -1,94 |
| | | 221602_s_at | -5,13 | nc | -1,83 |
| **FLT3LG** | fms-related tyrosine kinase 3 ligand | 206980_s_at | -2,03 | nc | 3,36 |
| | | 210607_at | nc | nc | 4,01 |
| **FYB** | FYN binding protein (FYB-120/130) | 227266_s_at | nc | nc | -1,44 |
| **FYN** | FYN oncogene related to SRC, FGR, YES | 212486_s_at | -2,18 | nc | nc |
| **HLA-DMA** | MHC, class II, DM alpha | 217478_s_at | nc | nc | 2,42 |
| **HLA-DMB** | MHC, class II, DM beta | 203932_at | nc | nc | 2,31 |
| **HLA-DPA1** | MHC, class II, DP alpha 1 | 211990_at | 4,68 | nc | nc |
| **HLA-DPB1** | MHC, class II, DP beta 1 | 201137_s_at | 4,89 | nc | nc |
| **HLA-DQA1³** | MHC, class II, DQ alpha 1 | 212671_s_at | 15,46 | 3,07 | 2,49 |
| **HLA-DQB1^{e}** | MHC, class II, DQ beta 1 | 212998_x_at | 14,90 | 2,49 | 2,03 |
| **HLA-DRA^{e}** | MHC, class II, DR alpha | 208894_at | 3,45 | 3,01 | 4,17 |
| **IFI44** | interferon-induced protein 44 | 214453_s_at | -3,08 | nc | nc |
| **IFI6** | Interferon, alpha-inducible protein 6 | 224533_s_at | -22,00 | nc | nc |
| **IFITM1^{e}** | interferon induced transmembrane 1 | 201601_x_at | nc | 1,57 | 2,17 |
| | | 214022_s_at | nc | nc | 2,10 |
| **IFITM2** | interferon induced transmembrane 2 | 201315_x_at | nc | 1,59 | nc |
| **IL2RA** | interleukin 2 receptor, alpha (CD25) | 211269_s_at | nc | nc | 4,22 |
| **IL2RB** | interleukin 2 receptor, beta (CD122) | 205291_at | nc | nc | 1,73 |
| **IL4R** | interleukin 4 receptor (CD124) | 203233_at | 2,98 | nc | nc |
| **IL5** | interleukin 5 (CSF, eosinophil) | 207952_at | nc | nc | 6,82 |
| **IL6ST** | Interleukin 6 signal transducer (CD130) | 212195_at | -1,71 | nc | nc |
| **IL7R** | interleukin 7 receptor (CD127) | 205798_at | -1,59 | nc | -1,50 |
| **IL9R** | interleukin 9 receptor (CD129) | 214950_at | 8,81 | nc | nc |
| **IL13** | interleukin 13 | 207844_at | nc | nc | 7,34 |
| **IL17RB^{e}** | interleukin 17 receptor B | 219255_x_at | 22,24 | 1,55 | 2,97 |
| **IL18R1** | interleukin 18 receptor 1 (CD218a) | 206618_at | -4,85 | nc | nc |
| **IL18RAP** | interleukin 18 receptorAP (CD218b) | 207072_at | -6,27 | nc | nc |
| **IL23A** | interleukin 23, alpha subunit p19 | 234377_at | -9,93 | nc | nc |
| **IL27RA** | interleukin 27 receptor, alpha | 222062_at | -1,61 | nc | nc |
| **IL32** | interleukin 32 | 203828_s_at | nc | 2,50 | nc |
| **INPP4B^{e}** | inositol polyphosphate-4-phosphatase | 205376_at | 1,99 | nc | 1,78 |
| **IRF4** | interferon regulatory factor 4 | 204562_at | nc | nc | 6,12 |
| **ISGF3G** | interferon-stimulated transcript. factor 3 | 203882_at | nc | nc | 1,83 |
| **ITGA4** | integrin, alpha 4 (CD49D, VLA-4) | 205885_s_at | nc | 2,78 | nc |
| | | 213416_at | nc | nc | 2,05 |
| | | 244599_at | -6,56 | nc | nc |
| **ITGA6** | integrin, alpha 6 | 215177_s_at | 5,67 | nc | nc |
| **ITGB1** | integrin, beta 1 (CD29) | 1553678_a_at | 1,70 | nc | nc |
| **LGALS1** | lectin, galactoside-binding 1 (galectin 1) | 201105_at | nc | 2,40 | nc |
| **LGALS3** | lectin, galactoside-binding 3 (galectin 3) | 208949_s_at | -14,53 | nc | nc |
| **LIF^{e}** | leukemia inhibitory factor | 205266_at | nc | nc | 2,13 |
| **LMAN1** | lectin, mannose-binding, 1 | 224629_at | 1,49 | nc | 1,46 |
| **LMNA** | lamin A/C | 1554600_s_at | nc | nc | 2,13 |
| **LMNB1** | lamin B1 | 203276_at | nc | nc | 1,76 |
| **LTA** | lymphotoxin alpha (TNFSF1) | 206975_at | nc | nc | 3,33 |
| **LTB** | lymphotoxin beta (TNFSF3) | 207339_s_at | nc | nc | 1,58 |
| **MAL** | mal, T-cell differentiation protein | 204777_s_at | nc | nc | 2,19 |
| **MAP2K5** | mitogen-activated protein kinase kinase 5 | 216765_at | nc | -1,75 | nc |
| **MAP3K7IP2** | mitogen-activated protein 3x kinase 7 IP2 | 212184_s_at | -1,72 | nc | 1,68 |
| **MAP3K7IP3** | mitogen-activated protein 3x kinase 7 IP3 | 227357_at | nc | 1,45 | nc |
| **MAP3K8** | mitogen-activated protein 3x kinase 8 | 205027_s_at | 4,07 | nc | nc |
| | | 235421_at | 2,91 | 1,93 | nc |
| **MAPKAPK3** | mitogen-activated protein kinase-act 3 | 202788_at | nc | nc | 1,54 |
| **MAP4K1** | mitogen-activated protein 4x kinase 1 | 206296_x_at | 2,00 | nc | nc |
| **NDFIP2^{e}** | Nedd4 family interacting protein 2 | 224802_at | 3,62 | nc | 8,10 |
| | | 224799_at | nc | nc | 6,62 |
| **NFIA** | nuclear factor I/A | 224970_at | nc | 1,76 | nc |
| **NFIL3^{e}** | nuclear factor, interleukin 3 regulated | 203574_at | nc | 2,73 | 2,69 |
| **NFATC1** | NFAT, cytoplasmic 1 | 211105_s_at | -1,92 | nc | nc |
| **NFKBIZ** | NF-kB inhibitor, zeta | 223218_s_at | -4,56 | nc | nc |
| **OSM** | oncostatin M | 230170_at | nc | nc | 3,52 |
| **PAK1IP1** | PAK1 interacting protein 1 | 218886_at | nc | nc | 1,82 |
| **PBXIP1** | pre-B-cell leukemia TF interact. protein 1 | 214177_s_at | nc | nc | -1,62 |
| **PDE3B^{e}** | phosphodiesterase 3B, cGMP-inhibited | 214582_at | -2,12 | nc | -1,80 |
| | | 222330_at | -2,37 | nc | nc |
| **PDE4DIP** | phosphodiesterase 4D interacting | 236704_at | -4,48 | nc | nc |
| | | 212390_at | nc | -2,59 | nc |
| **PDE7A** | phosphodiesterase 7A | 1552343_s_at | nc | nc | -1,57 |
| | | 224046_s_at | -2,06 | nc | nc |
| **PDE9A** | phosphodiesterase 9A | 205593_s_at | -4,32 | nc | nc |
| **PIM1** | pim-1 oncogene | 209193_at | nc | nc | 2,22 |
| **PIM2** | pim-2 oncogene | 204269_at | nc | nc | 2,25 |
| **PMAIP1** | PMA-induced protein 1 | 204285_s_at | nc | nc | 5,69 |
| **PPIB** | peptidylprolyl isomerase B (cyclophilin B) | 200968_s_at | 1,91 | nc | nc |
| **PPIF** | peptidylprolyl isomerase F (cyclophilin F) | 201490_s_at | nc | nc | 1,66 |
| **PPIL1** | peptidylprolyl isomerase, cyclophilin-like 1 | 222500_at | nc | nc | 1,72 |
| **PRDMI^{e}** | PR domain containing 1, with ZNF domain | 228964_at | nc | 2,07 | 1,98 |
| **PTGER2** | prostaglandin E receptor 2 (subtype EP2) | 206631_at | nc | nc | 4,05 |
| **PTGER4** | prostaglandin E receptor 4 (subtype EP4) | 204897_at | 2,37 | nc | nc |
| **RGS1** | regulator of G-protein signalling 1 | 202988_s_at | 4,21 | nc | nc |
| **RGS10** | regulator of G-protein signalling 10 | 204319_s_at | -2,81 | 1,40 | nc |
| **RGS10** | regulator of G-protein signalling 10 | 204316_at | -2,02 | nc | nc |
| **RGS16** | regulator of G-protein signalling 16 | 209324_s_at | nc | nc | 2,29 |
| **RUNX1** | runt-related transcription factor 1 | 209360_s_at | nc | nc | 1,35 |
| **RUNX2** | runt-related transcription factor 2 | 232231_at | 2,41 | 1,86 | nc |
| **SNFT** | Jun dimerization protein p21SNFT | 220358_at | nc | nc | 2,28 |
| **SOCS1** | suppressor of cytokine signaling 1 | 210001_s_at | nc | nc | 5,15 |
| **SOCS2** | suppressor of cytokine signaling 2 | 203373_at | nc | nc | 4,44 |
| **SOCS3** | suppressor of cytokine signaling 3 | 227697_at | nc | nc | 4,49 |
| **SORL1** | sortilin-related receptor, L(DLR class) | 230707_at | 2,07 | nc | -1,85 |
| **SOS1^{e}** | son of sevenless homolog 1 | 212780_at | 2,97 | 1,45 | 1,65 |
| **SOX4** | SRY (sex determining region Y)-box 4 | 201417_at | -9,01 | nc | nc |
| **SOX6** | SRY (sex determining region Y)-box 6 | 235526_at | nc | 1,59 | nc |
| **STAT1** | signal transducer & act. transcription 1 | AFFX-HUMIS C | -1,67 | nc | 4,44 |
| | | 209969_s_at | nc | nc | 6,74 |
| **STAT3** | signal transducer & act. transcription 3 | 208992_s_at | nc | nc | 1,61 |
| **TFRC^{e}** | transferrin receptor (CD71) | 208691_at | 1,89 | nc | 1,84 |
| **TNF** | TNF superfamily, member 2 | 207113_s_at | nc | nc | 5,16 |
| **TNFAIP3** | TNF, alpha-induced protein 3 | 202644_s_at | nc | nc | -1,58 |
| **TNFRSF4^{e}** | TNF receptor superfamily, member 4 | 214228_x_at | nc | 1,65 | 3,30 |
| **TNFRSF10B** | TNF receptor superfamily, member 10b | 209295_at | -1,71 | nc | nc |
| **TNFRSF10D** | TNF receptor superfamily, member 10d | 227345_at | -3,39 | nc | nc |
| **TNFRSF11A** | TNF, member 11a, NFKB activator | 238846_at | 7,45 | nc | nc |
| **TNFSF4** | TNF (ligand) superfamily, member 4 | 207426_s_at | nc | 2,77 | nc |
| **TNFSFB** | TNF (ligand) superfamily, member 8 | 235735_at | nc | nc | 2,30 |
| **TNFSF10^{e}** | TNF (ligand) superfamily, member 10 | 214329_x_at | 2,92 | nc | 1,63 |
| | | 202688_at | 3,47 | nc | nc |
| **TNFSF11** | TNF (ligand) superfamily, member 11 | 210643_at | 10,35 | nc | nc |
| **TNFSF13B** | TNF (ligand) superfamily, member 13b | 223502_s_at | -9,87 | nc | nc |
| **TNFSF14** | TNF (ligand) superfamily, member 14 | 207907_at | 3,72 | nc | nc |
| **TNIK** | TRAF2 and NCK interacting kinase | 211828_s_at | 2,05 | nc | -1,62 |
| **TNIK** | TRAF2 and NCK interacting kinase | 213107 at | 3,06 | nc | nc |

| | | | | | |
|---|---|---|---|---|---|
| ^{a}One probe (greatest fold change) is shown for each gene except were differences were detected in individual probes to the same gene ^{b}Fold-change comparing the mean expression of duplicate arrays from P1, P2 and P3 non-stimulated with the mean expression from 4 controls; data from Table 5 ^{c}fold-changes detected in the mean expression from triplicate arrays of P1's non-stimulated CD3-CD4+ T cells from yr+6 compared with yr0; data from Table 6 ^{d}Fold-change comparing the mean expression of duplicate arrays from stimulated (S) versus non-stimulated (NS) CD3-CD4+ T cells from P1-P3. ^{e}Genes whose expression is similarly altered both after activation and in L-HES patient cells (either chronic disease or T lymphoma) ^{f}nc = no change ^{g}In P1, CCL5 levels are decreased yr0 vs C and yr +4 vs yr0 but then increase in yr+6 vs yr 4 (Table 6) | | | | | |

### G-protein coupled receptors

Numerous G-protein coupled receptors were altered on the CD3⁻CD4⁺ T-cells and include two of particular significance. A 19-fold decrease in cysteinyl leukotriene receptor 1 (CYSLTR1) gene expression was observed in the CD3⁻CD4⁺ T-cells with the greatest degree of downmodulation detected in Pl-yr0. This decrease was confirmed by Q-RT-PCR (P1-P5+P7; Figure 2A; Table 3) but revealed a disparity in expression between the patients (i.e. it is not expressed in P1 and low expression levels were detected in P2, P3 and P5). CYSLTR1 is normally expressed on myeloid cells, including eosinophils, and induced on CD4⁺ T-cells by type 2 cytokines and TCR/CD3-mediated activation. The CYSLTR1 ligand, leukotriene D4, is produced by eosinophils and other myeloid cells and plays an active role both in cell survival and leukocyte recruitment to inflamed tissues. In contrast to the decreased expression observed on L-HES CD3⁻CD4⁺ T-cells, CYSLTR1 is significantly upregulated and functional on CD4⁺ T-cells from mice carrying a LAT gene mutation. These mice develop a Th2 lymphoproliferative disorder characterized by marked infiltration of CD3^{lo}CD4⁺ T-cells in secondary lymphoid organs.

Lack of CYSLTR1 on patients CD3⁻CD4⁺ T-cells may render them less responsive to eosinophil derived survival signals and thereby contribute to the indolent nature of L-HES.

The prostaglandin D2 receptor CRTH2 (CD294, GPR44), a G-protein coupled receptor selectively expressed by Th2 cells, eosinophils and basophils, is currently considered the most reliable marker for memory Th2 cells. Two CRTH2 probes revealed a 5- and 22-fold increase in expression in the patients abnormal T-cells, which was confirmed by flow cytometry (Table 2). CRTH2 is involved in Th2 cell migration, GATA3 upregulation and the induction of Th2 cytokine production. These experiments found that GATA3 nuclear binding is upregulated in patients activated but not quiescent CD3⁻CD4⁺ T-cells; however, the arrays detected a 3.6-fold increase in GATA3 transcripts in the quiescent CD3⁻CD4⁺ T-cells. This is substantially greater than the lack of significant differences detected in quiescent human Th1 and Th2 cells in a published microarray study (Freishtat RJ et al. (2005)). Taken together, these data suggest that significant levels of GATA3 may be present in the cytoplasm waiting for activation signals that rapidly induce phosphorylation, nuclear translocation and cytokine gene upregulation. The gene expression profiles of unstimulated versus CD2/CD28 co-stimulated CD3CD4⁺ T-cells clearly show that their Th2 cytokine expression is dependent upon activation (Table 8). The inventors also found that production of Th2 cytokines *in vitro* by the CD3⁻CD4⁺ T-cells requires exogenous activating factors such as those provided by dendritic cells. The lack of differences in Th2 cytokine gene expression detected between patient and control blood derived T-cells suggests that despite high CRTH2 and GATA3 expression levels, activating signals from local microenvironments *in vivo* are required to bring the circulating cells out of standby.

### Apoptosis

The clonal CD3⁻CD4⁺ T-cells persist at relatively stable levels for many years *in vivo*, suggesting equilibrium between cell proliferation and apoptosis during chronic disease. The death domain containing TNF superfamily plays critical roles in controlling the induction and progression of cell death with altered expression of several genes observed in the CD3⁻CD4⁺ T-cells, including upregulation of the pro-apoptotic genes FAS, TNFSF10 (TRAIL), TRADD, TNFRSF10B (TRAILR2), TNFSF14 and the anti-apoptotic (pro-proliferation) genes PECAM1, BIRC4, TNFSF11 (RANKL) and DIABLO. The inventors also detected downregulation of the pro-apoptotic genes ATM, CD47, CASP10, BNIP3, TNFRSF7, STK17A and SMAD7 and the anti-apoptotic genes BCL2, BCL2L11, FYN, FAIM3, GALECTIN3, AATF, KIT (CD117), MYB, and TNFRSF10D (CD264). Upregulation of DIABLO and downregulaton of KIT and SMAD7 transcripts was confirmed by Q-RT-PCR (Figure 2A-B). Increased surface expression of FAS and a lack of CD27 in L-HES were reconfirmed in this cohort (Table 2). RANKL (TNFSF11; 10-fold increase) augments the co-stimulatory properties of antigen presenting cells and thus could be important for CD3-CD4⁺ T-cell activation *in vivo.* A 10-fold increase in RANKL mRNA has also been detected in microarrays of CD4⁺ T-cells from patients with Sezary Syndrome (van Doorn R et al. (2004)). There was no further increase in RANKL expression associated with P1's progression to lymphoma or upon co-stimulation. Increased RANKL expression in L-HES is especially interesting given the ongoing development of humanized monoclonal antibodies for clinical uses (Phases II and III). The altered expression in specific subsets of genes involved in programmed cell death observed in this study suggests there is a controlled balance that potentiates the increased survival and persistent expansion of the CD3⁻CD4⁺ T-cell clone.

### T-cell homeostasis and the TGF-beta family

Altered expression among the TGFβ superfamily [TGFβ, activins, inhibins, growth differentiation factors (GDF) and bone morphogenetic proteins (BMP)] has been described for a variety of epithelial-derived solid tumors and hematological malignancies. A recent microarray study revealed that TGFa is the major signaling pathway that constitutively keeps human CD4⁺ T-cells in a resting state. In this study, the inventors detected numerous changes in expression of TGFβ family genes expression in the L-HES CD3⁻CD4⁺ T-cells during chronic disease with a subset of these genes changing further during P1's evolution to T-lymphoma; in contrast, no additional changes were observed in the patients abnormal T-cells after CD2/CD28 co-stimulation. Decreased expression in the CD3⁻CD4⁺ T-cells of the type I TGFβ receptor genes, TGFBR1 (TGFβRI) and ACVRIC, and the type II receptor gene TGFBR2 (TGFβRII) was confirmed by qRT-PCR (Figure 2A). A previous study of CD4⁺ T-cell lines derived from T-lymphoma patients found decreased TGFβRI and TGFβRII expression was related to reduced responsiveness to TGFβ1-mediated growth inhibition while microarrays of Sezary T-cells detected TGFBR2 gene downregulation (van Doorn R et al. (2004)).

Studies have shown that a third TGFβ receptor, TFGBR3 (TGFβRIII, betaglycan) is frequently downregulated in solid tumors in contrast to B-chronic lymphocytic leukemia where upregulation of this gene has been reported. Increased TGFBR3 in the CD3⁻CD4⁺ T-cells was detected from L-HES patients with chronic disease. Corticosteroids can selectively stimulate TGFβRIII expression in hepatic stellate cells and since corticosteroids are standard therapy for symptomatic L-HES patients, this treatment could be responsible for the TGFβRIII upregulation observed because the fold-changes for P2 and P3 (treated; Table 1) were lower than P1 (untreated).

TGFβRIII binds all TGFβ isoforms and presents them to TGFβRII thereby initiating the recruitment and phosphorylation of TGFβRI leading to kinase activation. However, evidence indicates that TGFβRIII also functions independently from TGFβ ligand presentation by working as a co-receptor with type 2 activin receptors (ACVR2A and ACVR2B), which increased in CD3⁻CD4⁺ T-cells. Activins and inhibins are structurally related members of the TGFβ superfamily that act as antagonists, with the former providing positive and the latter negative intracellular signals. High affinity binding of inhibin by TGFβRIII is favored in cells co-expressing ACVR2A, thereby inhibiting the activin pathway.

The inventors also detected an increase in the BMP type I receptor, BMPRIA which can interact with ACVR2A to bind BMPs. Finally, noggin (NOG) was substantially decreased in the abnormal T-cells. Noggin acts as an antagonist for the TGFβ superfamily members, BMP2 and BMP4, both of which play a role in early thymocyte differentiation. Altogether, these alterations in gene expression reflect a shift in the balance of TGFβ superfamily-dependent intracellular pathways in the CD3⁻CD4⁺ T-cells, with uncontrolled signaling via the BMP pathway possibly disrupting homeostasis and favoring abnormal cell survival and growth.

This hypothesis is further substantiated by altered expression of Smad proteins, which transmit signals downstream from the TGFβ superfamily receptors. An increased receptor regulated SMAD5 gene expression was observed together with a decrease in the inhibitory SMAD7 gene, both confirmed by qRT-PCR. While their function in hematopoietic cells is not as well defined as Smad2, -3 and -4, Smad5 is involved in regulating BMP signaling while Smad7 negatively regulates receptor regulated Smad signaling and has been implicated in mature hematopoietic cell development. Receptor regulated Smad proteins specific for the BMP pathway, such as Smad5, interact with a variety of proteins, including Runx family transcription factors. The RUNX genes have been shown to function as tumor suppressors in a number of human cancers, although their overexpression in murine models also revealed an oncogenic role in the development of hematopoietic tumors including T-lymphomas. Runx2 mediates cellular responses to signaling pathways hyperactive in tumors, including TGFβ family pathways, by forming co-regulatory complexes with Smads and other co-activator and co-repressor proteins to regulate gene transcription. Runx2, better known for its role in bone development and maintenance, was upregulated in all patients and then again in P1 with her evolution to T-lymphoma (Table 7). RUNX2 and RANKL are targets of transcriptional regulation by the vitamin D receptor (VDR), and all three genes were upregulated in CD3⁻CD4⁺ T-cells from chronic disease. In addition, several target genes known to be induced by TGFβ were also decreased, including JUN, MYB, FLT3LG and CXCR4 (the latter confirmed by flow cytometry). The clusterin gene (CLU), which has been shown to interact with TGFβRII and modulate Smad signaling, was also significantly upregulated in the abnormal T-cells. Further investigation into the perturbations detected in the TGFβ superfamily signaling pathways and the role they play in the persistence of the CD3⁻CD4⁺ T-cell clone in L-HES are ongoing.

### Gene expression changes in the CD3⁻CD4⁺ T-cells associated with the evolution from chronic L-HES to T-lymphoma

Cryopreserved blood samples from P1 spanning her six year progression from chronic disease to T-lymphoma provided a very rare opportunity to assess changes in gene expression associated with malignant transformation *in vivo.* Previous studies found that over time the initial CD3⁻CD4⁺ T-cell clone spawned subclones containing two independent 6q deletions (6q11-6q23.1 and 6q13-6q22.1) with progressive outgrowth of the 6q13q22.1-deleted subclone detected in 91% of the malignant T-cells. Gene expression profiles of P1's CD3⁻CD4⁺ T-cells at diagnosis (yr0), during chronic disease (yr+4) and with T-lymphoma (yr+6) revealed 349 genes (=450 probe sets) that were differentially expressed in the malignant compared with the pre-malignant T-cells (Table 9; Table 6). Remarkably, approximately one-third of the probes (126/450), corresponding to 87/349 genes, were also initially altered in patients with chronic L-HES (Table 7). They included genes whose expression decreased or increased stepwise, first in patients with chronic disease and then with the development of T-lymphoma. Progressive decreases were observed in the apoptosis genes BACH2 (located in the 6q-deleted region), BCL2 and its interacting protein BNIP3 and the Fas inhibitory molecule FAIM3, the growth factors FGF9 and NELL2, the extracellular matrix protein SPON1 and the transcription factors KLF9 and TCEAL4. Progressive increases included the surface receptors CCR8, IL17RB, GPR68, MHC class II, and DCAL1, the growth factors MSC and PBEF1, the signaling proteins MAP3K8, PTPRN2, PTPLAD2 and SOS1 and the transcription factors RUNX2 and RBBP8.

Among the genes whose expression changed progressively from chronic to malignant L-HES only six genes (BCAT1, HLA-DQA1/2, HLA-DQB1, HLA-DRA, IL17RB and SOS1) also increased and only the FAIM3 gene decreased following in vitro activation (Table 8).

These data suggest that the genes whose expression is altered in the abnormal T-cells from chronic and malignant L-HES reflect genuine changes in CD3⁻CD4⁺ T-cell physiology and not a transient response to external stimuli such as changes in signaling molecules, surface receptors and cytokine production.

Some genes were newly altered with the development of T-lymphoma and present potential relevance for malignant transformation. They include increases at yr+4 and yr+6 in genes for the transcription factors CREM, FKBP5, MKI67 and the toll receptor TLR5 or yr+6 only in the receptors CD96, IGFBP4, IFITM2, PECAM1 and TNFSF4 (OX40), the growth factors LGALS1 and TNFRSF4 (OX40L), the transcription factors FOSL2, NFIL3, NFIA, RUNX2, SOX6, TOX and VDR and the signaling proteins MAP3K7IP3, PRKX, PTEN and S100P. A limited number of genes with decreased expression in the lymphoma cells were detected at yr+6 and included the signaling proteins IGFBP3, ITPKB, STK17B and TAGAP and the transcription factors MXI1 and SKIL.

Overall, these alterations reflect progressive activation, altered signaling and/or homing of the CD3⁻CD4⁺ T-cells to specific sites and/or their adaptation to a specific microenvironment. The stepwise modulated genes as well as those newly deregulated in the malignant T-cells of particular interest and relevance as potential therapeutic targets.

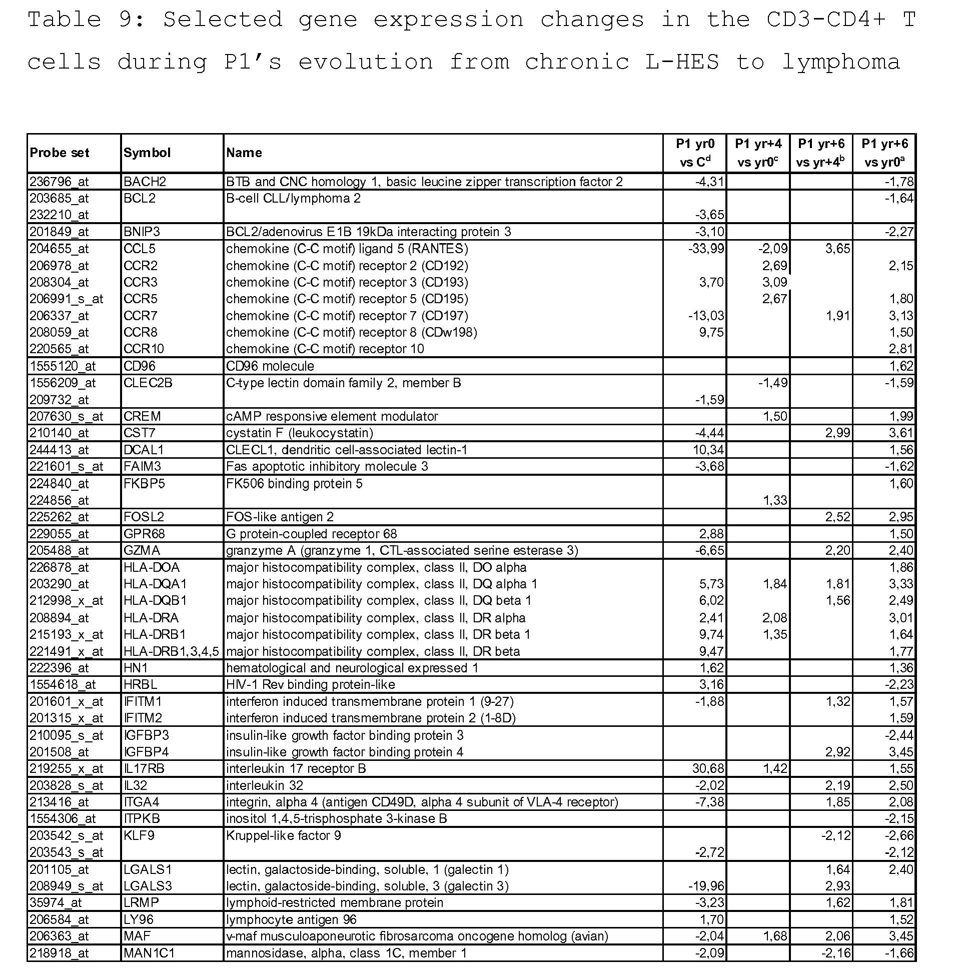

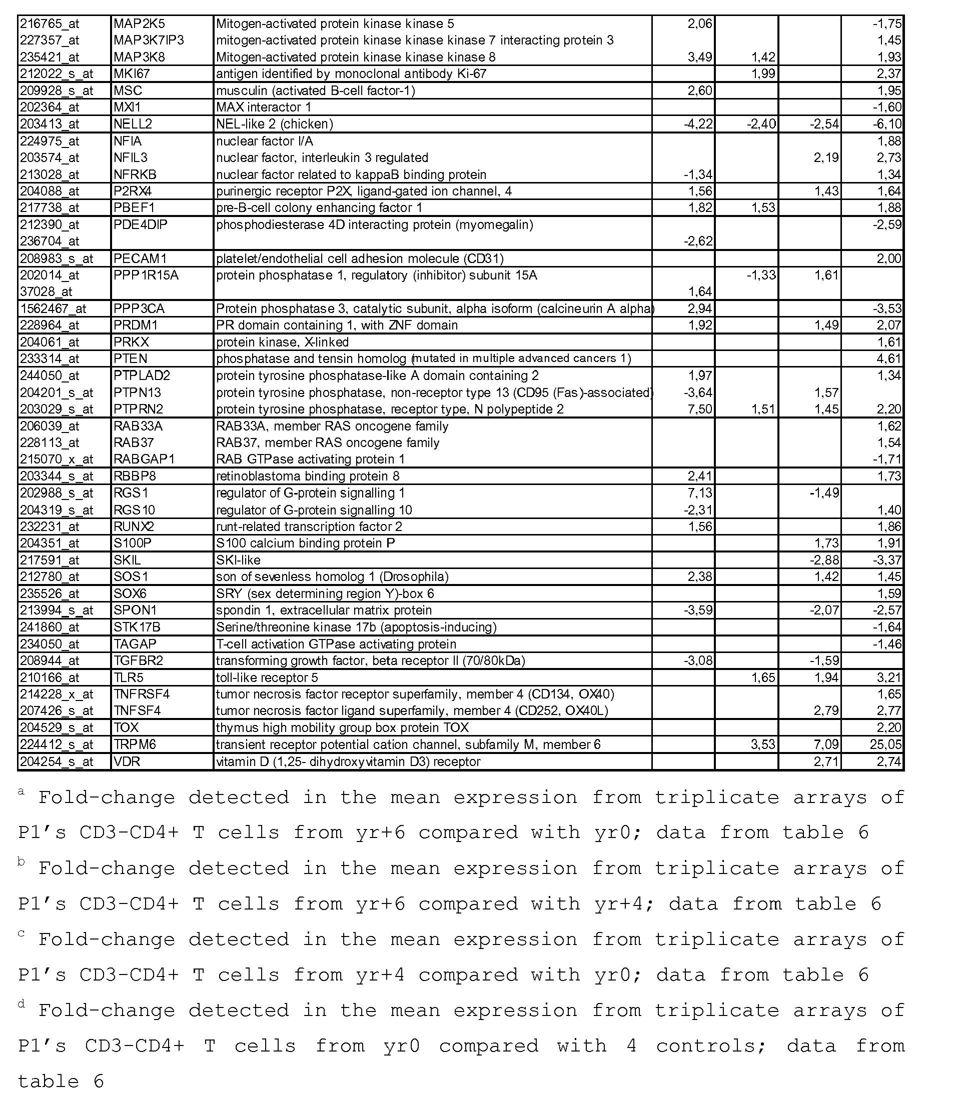

### T-Cell Trafficking and Migration

Leukocyte migration is mediated by a network of trafficking receptors expressed both on lymphoid and non-lymphoid tissues such that specific combinations of these adhesion and chemoattractant molecules act as traffic signals for directing extravasion and migration. Trafficking genes play distinct roles as leukocytes migrate through blood vessels. The initial step is mediated by selectins and flow cytometry revealed surface receptor upregulation on the CD3⁻CD4⁺ T-cells, which continued to increase as P1 progressed to T-lymphoma (Figure 2C). Rolling over endothelial cells exposes leukocytes to chemokines which in turn provoke conformational changes in integrins that increase their affinity. The α4β1 integrin (VLA-4) is composed of two subunits: CD49D (α4, ITGA4) and CD29 (β1, ITGB1), both required for VLA-4 surface expression. Downregulation of CD49D in association with a slight increase in CD29 was observed in the CD3⁻CD4⁺ T-cells during chronic disease (Figure 2; Tables 2 & 5). CD49D was re-expressed in concert with increased VLA-4 surface expression as P1 developed enlarged lymph nodes and progressed to T-lymphoma (Table 7, Figure 2C).

Changes in other trafficking receptor genes were detected both in chronic disease and during the evolution to T-lymphoma.

Downregulation of CXCR4 (CD184), CXCR6 (CD186), CCR6 and CCR7 were detected in patients with chronic disease with some CCR7 expression returning as P1 progressed to T-lymphoma (Tables 7 & 9).

Increases in CCR3, ICAM3 (CD50), LFA-3 (CD58), CD82 (KAI1), CD99 were observed in all patients with additional upregulation of CCR2 in P1-yr+4 and CCR5, CCR10, CD96 and PECAM1 in P1-yr+6 (Tables 7 & 8).
CCR8 expression levels increased stepwise, 10-fold in chronic patients and a further 1.5 fold in P1-yr+6. CCR8 binds 1-309 (CCL1), which like CCL17 can be induced in bronchial epithelial cells by the Th2 cytokines IL-4 and IL-13. Although serum CCL17 levels are extremely high in patients with L-HES, serum CCL1 levels and the functional role of CCR8 on CD3⁻CD4⁺ T-cells remain unknown.
The altered expression of trafficking receptors and ligands observed on the CD3⁻CD4⁺ T-cells likely directs their movement to specific sites during pre-malignant and malignant L-HES disease, exposing the cells to external activation signals and/or co-stimulatory cells present locally.

### Signaling

The lack of TCR/CD3 expression on the abnormal T-cell surface dictates the loss of this important signaling pathway leaving the CD3⁻CD4⁺ T-cells responsive to co-stimulatory signals alone. The microarrays revealed that some critical regulatory signals downstream from the TCR/CD3 receptor affecting co-stimulatory pathways were decreased in the patients CD3⁻CD4⁺ T-cells. They include the inhibitory receptor CTLA4, the PI3K associated or family proteins ATM, PIK3R5, PIP3E and PITPNC1, the tyrosine kinases FYN, JAK1 and TXK (TEC), the MAP3 kinase MAP37IP2, and the transcription factors NFATc1, LEF1 (TCF1α) and JUN (AP-1).

### Differential microRNA expression in the CD3⁻CD4⁺ T-cells

MicroRNAs are endogenously-expressed noncoding RNAs that regulate gene expression via mRNA degradation, mRNA destabilization or translation inhibition. There is growing evidence that deregulated microRNA expression contributes to oncogenesis with an increasing number of identified microRNAs targeting genes involved in immune development, proliferation and apoptosis. The molecular profile of L-HES was extended by using qRT-PCR to quantify changes in mature microRNA expression. Initially, the inventors compared the expression of 156 microRNAs in CD3⁻CD4⁺ T-cells from P1-yr.6 with control CD3⁺CD4⁺ T-cells (Table 10). Thirty eight microRNAs that decreased or increased greater than two-fold in two independent experiments were selected for further analysis in CD3⁻CD4⁺ T-cells from six chronic L-HES patients (P1-P5&P7) and CD3⁺CD4⁺ T-cells from the same four controls. Using the non-paired student t-test, 23 microRNAs were differentially expressed in the abnormal T-cells (Table 10). The majority (19/23) of the selected microRNAs were downregulated with increases found for only four microRNAs.

**Table 10 microRNAs that are differentially expressed in the CD3⁻CD4⁺ T cells from L-HES patients compared from CD3⁺CD4⁺ T cells from controls T-cells from P1 to P6 compared with CD3⁺CD4⁺ T cells from four controls**

| **miRNA name** | **Patients versus controls** | | **Chromosomal location^{c}** |
|---|---|---|---|
| | **p-value^{a}** | **Fold change^{b}** | |
| hsa-let-7b | 0,032 | 3,2 | 22q13.31 |
| hsa-miR-9 | 0,058 | 13,5 | 1q23.1 |
| hsa-miR-10a | 0,052 | -6,5 | 17q21.32 |
| hsa-miR-26a | 0,019 | -2,3 | 3p22.3 |
| hsa-miR-31 | 0,004 | -111,4 | 9p21.3 |
| hsa-miR-95 | 0,025 | -2,6 | 4p16.1 |
| hsa-miR-99a | 0,011 | -60,9 | 21q21.1 |
| hsa-miR-100 | 0,010 | -57,6 | 11q24.1 |
| hsa-miR-125a | 0,059 | -5,7 | 19q13.41 |
| hsa-miR-126 | 0,030 | -9,1 | 9q34.3 |
| hsa-miR-130a | 0,034 | -6,2 | 11q12.1 |
| hsa-miR-135b | 0,011 | -11,8 | 1q32.1 |
| hsa-miR-135a | 0,008 | -10,9 | 3p21.1 |
| hsa-miR-151 | 0,019 | -12,1 | 8q24.3 |
| hsa-miR-181a | 0,010 | -34,6 | 1q31.3 |
| hsa-miR-181b | 0,010 | -19,3 | 1q31.3 |
| hsa-miR-193a | 0,017 | -4,6 | 17q11.2 |
| hsa-miR-213 | 0,011 | -78,8 | 1q31.3 |
| hsa-miR-215 | 0,019 | -3,1 | 1q41 |
| hsa-miR-221 | 0,010 | 3,4 | Xp11.3 |
| hsa-miR-222 | 0,010 | 3,7 | Xp11.3 |
| hsa-miR-335 | 0,010 | -8,0 | 7q32.2 |
| hsa-miR-340 | 0,019 | -4,9 | 5q35.3 |

| | | | |
|---|---|---|---|
| ^{a} : P values were corrected using the False Discovery Calculation. ^{b} : Fold change in the L-HES patients CD3⁻CD4⁺ T cells (P1-P7) relative to controls (4). ^{c}: Chromosomal locations were obtained from Ensembl. | | | |

One effect of the interaction between a microRNA and its target mRNA can be transcript cleavage and degradation. Ingenuity Pathways Analysis® was used to assess the potential biological importance of the predicted target genes as a group and determined that the best scored functional networks included the cell cycle, cell death and hematological system development and function. Individual microRNAs and their putative gene targets were generated using MirBase and included some of notable interest and potential relevance. The expression of three Th2 genes in the CD3⁻CD4⁺ T-cells inversely paralleled several microRNAs predicted to target them including increases in GATA3 with decreases in miR-10a, miR-95 and miR-130a, IL4R increases in concert with decreased miR-126 and miR-340 and increased CCR3 in parallel with decreased miR-181a, miR-181b and miR-335. Genes whose mRNA expression changed in the abnormal T-cells that were also predicted targets of two or three altered microRNAs included: IL-18RAP (let7b, miR-221), CD99 (miR-31, miR-95, miR-135a), TRADD (miR-31, miR-125a), CD58 (miR-95, miR-135b), PPP3CA (miR-99a, miR-100), TNFSF11 (miR-126, miR-335), DMN3 (miR-126, miR-151), RGS1 (miR-130a, miR-335) and PRMT2 (miR-221, miR222).

Perhaps of greatest potential biological significance were three genes whose expression increased in patients with chronic disease (RBBP8, CLU and MAP3K8) with further increases associated with P1's evolution to T-lymphoma (RBBP8 and MAP3K8) that were also predicted targets of four different downregulated microRNAs. Retinoblastoma binding protein 8 (RBBP8) is a predicted target of the downregulated miR-31, miR-126, miR-130a and miR-335. The protein encoded by this gene is thought to function as a tumor suppressor in conjunction with the transcriptional co-repressor CTBP and BRCA1. Clusterin (CLU) is a calcium regulated protein whose expression has been associated with tumorigenesis and malignant progression, perhaps in part by modulating TGFβRII signaling. The nuclear form is pro-apoptotic and the secretory form is anti-apoptotic with both forms involved in DNA repair and cell cycle regulation. Clusterin expression was significantly upregulated in the CD3⁻CD4⁺ T-cells in concert with the downregulation of miR-99a, miR-100, miR-126 and miR-335. Thus, miR-126 and miR-335 potentially target both RBBP8 and CLU. A recent study of breast cancer found that miR-126 expression reduces tumor growth while miR-335 suppresses lung and bone metastasis. The loss of miR-335 leads to the activation of SOX4 and tenascin C (TNC), which are implicated in the acquisition of metastatic properties. A 9-fold decrease in SOX4 (no change in TNC) paralleled to a 9-fold decrease in miR-335 suggests that this microRNA targets other critical genes in T-cells.

Many of the gene changes detected in patients relative to controls and again during P1's evolution to T-lymphoma are involved in cell signaling. MAP3K8 (Tp12/Cot) oncogene expression increased stepwise first in patients during the chronic disease phase and again and during P1's evolution to T-lymphoma. The MAP3K8 oncogene is a predicted target of miR-135a, miR-135b, miR-181a and miR-181b, which were all decreased in the CD3⁻CD4⁺ T-cells. While little is known about the miR-135 family, studies have shown that decreased expression of miR-181b in B-CLL patients was associated with upregulation of the TCL1 oncogene. The MAP3K8 oncogene is of particular interest since it has been shown it is differentially regulated in hematopoietic cells and plays a role in tumor development. Overexpression and truncation of MAP3K8 leads to the activation of a number of T-cell signaling pathways and has been associated with large granular lymphocyte proliferative disorders. miR-181a also positively modulates TCR/CD3 sensitivity and affinity by suppressing phosphatases involved in negatively regulating TCR/CD3 signaling. The miR-181 family is involved in controlling hematopoietic cell differentiation and maturation with miR-181a levels fluctuating during thymopoiesis with its repression shown to diminish T-cell sensitivity in both primed and stimulated naive T-cells. MiR-181a has also been shown to inhibit CD69, BCL2 and TCRα gene transcription. Intriguingly, these data revealed a substantial decrease in miR-181a and miR-181b associated with a low CD69, BCL2 and TCR/CD3 expression levels in the CD3⁻CD4⁺ T-cells, suggesting that the complex interactions between the TCR/CD3 signaling genes and the miR-181 family require further analysis.

Experiments designed to approach the functional relevance of decreased expression of miR-135 family members, about which little is known, were accomplished by transfecting miR-135a and miR-135b mimics together in the CD4⁺ Jurkat T-cell line. These data indicate that the miR-135 mimics decrease MAP3K8 (-2.6 fold) and SMAD5 (-2.3 fold) expression compared to irrelevant sequence controls.

### Changes in microRNA expression during P1's clinical evolution

Expression of the same 38 microRNAs in association with P1's evolution to T-lymphoma has found that only miR-125a changed significantly. miR-125a levels were 5.7-fold lower (p=0.059) in the L-HES patient cohort relative to controls (Table 10) and as P1 evolved to T-lymphoma expression of miR-125a progressively decreased with an additional 2.8-fold drop (p=0.0033) detected at yr+6. Predicted gene targets of miR-125a were generated using MirBase and compared with the mRNA expression profiles of P1's CD3⁻CD4⁺ T-cells (Table 10).

The upregulated target genes included another gene involved in signaling, PTPRN2, which is a member of the receptor-type protein tyrosine phosphatase family. PTPRN2 expression increased in parallel with the progressive decrease of miR-125a expression in the CD3⁻CD4⁺ T-cells from chronic and malignant disease. PTPRN2 (IA-2β) is a pancreatic β-cell autoantigen for type 1 diabetes and although its function is largely unknown, its role in L-HES warrants further investigation. A second miR-125a target gene, the Abelson helper integration site 1 (AHI1) was significantly upregulated in the latter stages of P1's evolution to T-lymphoma. AHI1 has been implicated in the development of T and B cell malignancies with increased expression detected in CD4⁺CD7⁻ T-cells from Sezary syndrome patients. While the function of miR-125a remains unknown its homolog miR-125b has been shown to post-trancriptionally target TNFα and decrease cell proliferation. The stepwise downregulation of miR-125a detected in L-HES disease suggests a potential role for this microRNA in the persistence and progressive transformation of the CD3⁻CD4⁺ T-cells.

The global gene expression study was undertaken in the present invention to identify, in an unbiased manner, the specific genes and cellular pathways involved in the complex interplay between persistence and control of the clonal CD3⁻CD4⁺ T-cell population in chronic L-HES and in association with progression to full-blown malignancy.

Identification of functional networks operating in the CD3-CD4+ cells has potential clinical relevance as a first step toward developing targeted therapy for this HES variant, and for distinction of patients with T cell mediated HES (or L-HES) among those fulfilling the diagnostic criteria for HES (ie development of a more standardised diagnostic tool). Also, despite the rarity of this disease, these patients afford an uncommon opportunity to assess gene expression changes acquired by abnormal T-cells *in vivo.*

The microarray analysis of patients with chronic disease provides a detailed immunophenotype/genotype confirming the Th2 nature of the abnormal T-cell clone and offering insight on activation pathways and their homing state. Comparison of gene expression profiles from patients CD3⁻CD4⁺ T-cells during chronic L-HES versus CD3⁺CD4⁺ T-cells from healthy controls, activated or not by CD2/CD28 co-stimulation demonstrated that altered gene expression in the abnormal T-cell clone does not simply reflect an activated memory T-cell phenotype. Additionally, these data confirm that other previously reported functional characteristics of the CD3⁻ CD4⁺ T-cells, such as Th2 cytokine production and altered surface receptor expression, occur upon engagement of membrane co-stimulatory receptors.

The inventors further assessed the importance of increased IL-25 receptor (IL-17RB) expression on the CD3⁻CD4⁺ T-cells given their expected significant *in vivo* exposure to eosinophil-derived IL-25 in the L-HES patients.

These data demonstrate that the CD3⁻CD4⁺ T-cells response to IL-25 is characterized by Th2 cytokine production and increased proliferation *in vitro.* Given the pre-malignant nature of the CD3⁻CD4⁺ T-cells during chronic disease, these findings indicate that controlling eosinophil levels should be a therapeutic endpoint for these patients, even though their frequently isolated cutaneous manifestations may not appear to warrant systemic therapy.

The inventors conclude that the blood-derived CD3⁻CD4⁺ T-cells are in a transient state of ingress and egress with tissue microenvironments where they receive the signals for aberrant cytokine production and expansion.

The inventors further observe a switch in TGFβ superfamily signaling from TGFβ/Activin-directed to BMP-directed gene expression. TGFβ has been extensively characterized for its immune suppressive functions and is known to play critical roles in controlling thymocyte development and limiting effector/memory T-cell responses. Activin A is produced by activated Th2 cells and plays a role in Th2 mediated responses of B cells and macrophages. BMPs were initially identified for their growth factor effects on bone formation but have since been shown to regulate neurogenesis and hematopoiesis during embryonic development, and although little is known about BMP-mediated control of mature T-cell responses, BMPs have been shown to play a role in T-cell differentiation in the thymus.

The inventors further conclude that the survival and expansion of the CD3⁻CD4⁺ T-cell clone in L-HES is due in part to a switch from negative TGFβ regulation to positive BMP signaling.

The sequential analysis of P1's clinical evolution revealed that almost one-third of the genes whose expression changed in association with the development of T-lymphoma were already abnormally expressed in L-HES patients during chronic disease. Changes in these genes cannot simply be explained by the emergence of 6q13q22.1-deleted subclone in concert with P1's T-lymphoma, but rather appear to reflect progressively deregulated oncogenes, transcription factors and signaling genes.

Together with the genes that were newly altered in P1's T-lymphoma cells, this relatively small number of genes identifies critical players in chronic and malignant L-HES. A good example of this is the expression of three genes, RBBP8, MAP3K8 and PTPRN2, that were increased in chronic disease patients, further augmented in the T-lymphoma cells and paralleled decreases in microRNAs predicted to target these genes.

The inventors further performed miRNA transfection experiments to better establish these functional consequences.

For example, the MAP3K8 oncogene is a member of the serine/threonine protein kinase family that was identified by its transforming activity. Activated MAP3K8 induces the ERK1/2, JNK, NF-κB and p38MAPK pathways and a study has shown that it is constitutively activated in HTLV-I-transformed human CD4+ T-cell lines. The MAP3K8 gene therefore illustrates a gene deregulation (increased expression) detected in the patient cohort during chronic disease, which was further augmented in L-HES associated T-lymphoma and identified as a potential target of microRNAs shown to be downmodulated in the patient's cells.

The present invention therefore provides a global assessment of gene expression changes characteristic of the CD3⁻CD4⁺ T-cells during chronic and malignant L-HES as a means of identifying the deregulated pathways that underlie their abnormal persistence and expansion in vivo. These data reveal important gene expression changes in receptors whose altered expression may contribute to the CD3⁻CD4⁺ T-cells modified responses to environmental stimuli as well as deviations in homeostatic growth control pathways whose perturbations may favor outgrowth of the abnormal T-cell clone. Preliminary functional experiments confirmed that the aberrant pathways identified in the CD3⁻ CD4⁺ T-cells warrant further in depth exploration and a number of specifically deregulated genes point to potential new drug targets and diagnostic markers.
These studies about the identification of specific genes and molecular pathways altered in L-HES help further our understanding of the disease process and point to potential new drug targets.

### REFERENCES

Ravoet M et al. Haematologica. 2005;90:753-765.
Roufosse F et al. Blood. 1999;94:994-1002.
Zhang L et al. J MolBiol. 2002;317:225-235.
Kennedy RE et al. Bioinformatics. 2006;22:1272-1274.
Willard-Gallo KE et al. ExpHematol. 2005;33:1147-1159.
de Lavareille A et al. EurJ Immunol. 2001;31:1037-1046.
Freishtat RJ et al. HumImmunol. 2005;66:1223-1234.
van Doorn R et al. Cancer Res. 2004;64:5578-5586.

### Description of a preferred embodiment of the invention

The inventors then used the gene set and the diagnostic kit of the invention to determine whether patients have the lymphocytic variant of hypereosinophilic syndrome (L-HES).

Blood samples from patients were purified and the gene expression level was determined.

Alternatively, CD3⁻CD4⁺ T cells of patients were purified using antibodies recognizing CD3 and CD4.

The inventors firstly used microarrays bearing the complete gene set of the invention.

The inventors then tested the diagnostic power of a set of genes (179 genes) identified by the microarrays as significantly different in the patients and felt by the inventors to be pathologically relevant. They further tested subsets of these genes to arrive at a diagnostic test based on fewer genes, such as 25, 10 and even 5 genes.

The inventors performed the same using proteins of the protein set of the invention.

The inventors quantified the gene expression and/or protein levels in the samples.

The inventors further combined the gene set with the measure of micro RNA content and observed an increased diagnostic efficiency for the lymphocytic variant of hypereosinophilic syndrome (HES).

Finally, the inventors tested anti RANKL (TNSF11) antibody (including Denosumab) for the treatment of L-HES patients determined by using the set and/or the diagnostic and/or the method according to the invention.

The inventors also tested a CRTH2 antagonist for the treatment of L-HES patients determined by using the set and/or the diagnostic and/or the method according to the invention

## Claims

1. A set comprising or consisting of RBBP8, MAP3K8 and PTPRN2 genes and/or corresponding encoded proteins and/or antibodies (or specific hypervariable portions thereof both) being directed against the said proteins, and possibly 1, 2, 3, 4, 5, 6, 7, 8, 9, 10, 20, 30, 40 or all the other gene(s) and/or corresponding encoded protein(s) and/or antibodies (or specific hypervariable portions thereof both) being directed against the said proteins encoded by the genes of Tables 4 to 9.

2. The set of claim 1 further comprising GPR68 and RUNX2 genes and/or corresponding encoded proteins, and/or antibodies (or specific hypervariable portions thereof both) being directed against the said proteins.

3. The set of claims 1 or 2 further comprising 2, 3, 4, 5, 6, 7, 8, 9 or all the genes and/or corresponding encoded proteins and/or antibodies (or specific hypervariable portion thereof both) being directed against the said proteins, selected from the group consisting of ACVR1C, ADRB2, AMICA1, BTLA, CCR3, CCR6, CCR7, CCR8, CD200R1, CD247, CD27, CD3G, CD5, CD55, CD7, CDH1, CHRM3, CLEC2B, CLECL1, CR1, CR2, CRTAM, CTLA4, CXCR4, CXCR6, CYSLTR1, EMR1, EPHB6, FAS, GPR137B, GPR44, GPR68, IGF1R, IGSF4, IGSF9B, IL17RB, IL18R1, IL4R, IL9R/LOC729486, ITGA4, ITGA6, KIT, KLRB1, KLRK1, NINJ2, NT5E, P2RY14, SLAMF1, SLAMF7, SPON1, TGFBR2, TGFBR3, TNFRSF10D, TNFRSF11A, TRAV20, TRBV21-1/19/7-2/5-4/3-1/TRBC1, TRDV2 and VIPR1.

4. The set according to any of the claims 1 to 3 further comprising 2, 3, 4, 5, 6, 7, 8, 9 or all the genes and/or corresponding encoded proteins and/or antibodies (or specific hypervariable portion thereof both) being directed against the said proteins, selected from the group consisting of AIF1, CCL5, CST7, F8, GZMK, IL23A, LGALS3, NOG, TNFSF10, TNFSF11, TNFSF13B and TNFSF14.

5. The set according to any of the claims 1 to 4 further comprising 2, 3, 4, 5, 6, 7, 8, 9 or all the genes and/or corresponding encoded proteins and/or antibodies (or specific hypervariable portion thereof both) being directed against the said proteins, selected from the group consisting of BACH2, BATF, FOXP1, GATA3, KLF9, LM02, LM04, LM07, MSC, MYB, MYBL1, NFKBIZ, RBBP8, RUNX2, SOX4, TCEA3, TCEAL4, TRERF1, TSHZ2 and WWTR1.

6. The set according to any of the preceding claims, further comprising a set of micro RNA(s) consisting of 1, 2, 3, 4, 5, 6, 7, 8, 9, 10, 11, 12, 13, 14, 15, 16, 17, 18, 19, 20, 21, 22 or all the 23 micro RNA of Table 10, being preferably MiR-181a and/or MiR-181b micro-RNA(s) and of at least one micro-RNA selected from the group consisting of miR-31, miRNA125a, miR-126, miR-130a, miRNA135a/b and miR-335.

7. The set of claim 1 comprising or consisting of 3, 4, 5, 6, 7, 8, 9, 10 or all the proteins selected from the list comprising BTLA, CCL4, CCL5, CCR3, CCR6, CCR8, CD27, CD47, CD55, CD59, CD71, CD82, CD95, CD99, CD200R1, CLEC2B, CLEC2D, CR1, CR2, CRTAM, CRTH2, CTLA4, CXCR4, CXCR6, CYSLTR1, DCAL1, ICAM3, IGF1R, IL-4R, IL6ST, IL7R, IL9R, IL-17RB, IL-18R, IL27RA, integrin α6, integrin α4 (ITGA4), integrin β1 (ITGB1), LFA3 (CD58) - , SLAMF1(CD150), SLAMF5 (CD84) and SLAMF7 (CD319) proteins and/or antibodies (or specific hypervariable portion thereof both) being directed against the said proteins.

8. The protein set of claim 7 consisting of or comprising one or two protein selected from the group consisting of CCR3, CCR8, CD71, CD82, CD95, CD99, CD200R1, CRTH2, DCAL1, ICAM3, IL-4R, IL9R, IL-17RB, integrin β1 (ITGB1), LFA3, SLAMF5, being preferably IL-17RB and CRTH2 and
one or two protein selected from the group consisting of BTLA, CCL4, CCL5, CCR6, CD27, CD47, CD55, CD59, CLEC2B, CLEC2D, CR1, CR2, CRTAM, CTLA4, CXCR4, CXCR6, CYSLTR1, IGF1R, IL6ST, IL7R, IL-18R, IL27RA, integrin α6, integrin α4 (ITGA4), SLAMF1 and SLAMF7, being preferably CD27 and/or antibodies (or specific hypervariable portions thereof both) being directed against the said proteins.

9. A diagnostic kit or device comprising the gene and possibly the micro RNAs set according to any of the previous claims 1 to 6 and other means for real time PCR analysis, such as means for qRT-PCR.

10. A diagnostic kit or device comprising the protein set according to any of the preceding claims 1 to 5 and/or 7-8 and means for protein analysis, such as means for FACS analysis.

11. The diagnostic kit of claims 9 or 10 further comprising
- a bio-assay module configured for detecting a gene or micro RNA expression or protein synthesis from a blood sample based upon the gene or protein or micro RNA set according to any of the claims 1 to 8 and
- a processor module configured to calculate expression of these genes or micro RNA or protein synthesis and to generate a risk assessment for a subject from which the said blood sample has been obtained.

12. A method for evaluating prognosis (prognostic) of an immune disorder, preferably the lymphocytic variant of hypereosinophilic syndrome (HES), in a mammal subject, preferably in a human patient, possibly human patients suffering from immune disorders, which comprises the steps of
- measuring gene, protein or micro RNA expression of a biological sample obtained from the mammal subject by putting into contact nucleotide and/or amino acids sequences of a blood sample obtained from this subject with the set according to any of the preceding claims 1 to 8 or with the kit according to any of the claims 9 to 11 and
- possibly generating a risk assessment for the subject of being affected by the said immune disorder being preferably HES and/or of the progression of the said immune disorder, being preferably lymphocytic variant (HES) syndrome, to T-lymphoma and from which the blood sample has been obtained.

13. The method according to claim 12 wherein the CD3⁻CD4⁺ cells are enriched using the protein set of claim 8 or the diagnostic kit of claim 10.

14. An anti RANKL (TNSF11) antibody (such as Denosumab) for use in the treatment of an immune disorder, being possibly L-HES, preferably as determined by using the set according to any of the claims 1-8 and/or the diagnostic according to any of the claims 9-11 and/or the method according to claims 12 or 13.
